# EUROPEAN PATENT APPLICATION

(11) **EP 1 285 919 A1**
(43) Date of publication of application: **26.02.2003**
(21) Application number: 01118727.5
(22) Date of filing: 06.08.2001
(51) Int. Cl.: C07D 409/12, A61K 31/335, A61P 35/00

(54) **C10 Carbonate substituted taxanes as antitumor agents**

(71) Applicant: Florida State University Research Foundation, Inc., Tallahassee, Florida 32306-2763 (US)
(72) Inventor: Holton, Robert A., Tallahassee, Florida 32312 (US)
(74) Representative: Maiwald, Walter, Dr. Dipl.-Chem.

(57) **Abstract**

Taxanes having a carbonate substituent at C(10), a hydroxy substituent at C(7), and a range of C(2), C(9), C(14), and side chain substituents.

## Description

### BACKGROUND OF THE INVENTION

The present invention is directed to novel taxanes which have exceptional utility as antitumor agents.

The taxane family of terpenes, of which baccatin III and taxol are members, has been the subject of considerable interest in both the biological and chemical arts. Taxol itself is employed as a cancer chemotherapeutic agent and possesses a broad range of tumor-inhibiting activity. Taxol has a 2'R, 3'S configuration and the following structural formula: 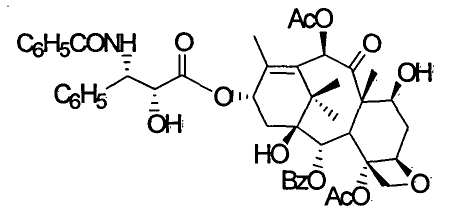 wherein Ac is acetyl.

Colin et al. reported in U.S. Patent 4,814,470 that certain taxol analogs have an activity significantly greater than that of taxol. One of these analogs, commonly referred to as docetaxel, has the following structural formula:

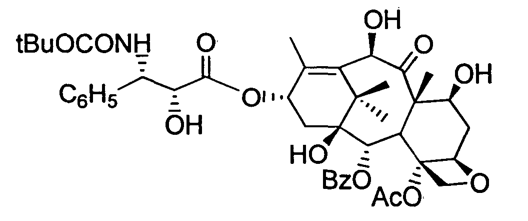

Although taxol and docetaxel are useful chemotherapeutic agents, there are limitations on their effectiveness, including limited efficacy against certain types of cancers and toxicity to subjects when administered at various doses. Accordingly, a need remains for additional chemotherapeutic agents with improved efficacy and less toxicity.

### SUMMARY OF THE INVENTION

Among the objects of the present invention, therefore, is the provision of taxanes which compare favorably to taxol and docetaxel with respect to efficacy as anti-tumor agents and with respect to toxicity. In general, these taxanes possess a carbonate substituent at C(10), a hydroxy substituent at C(7), and a range of C(2), C(9), C(14), and C(13) side chain substituents.

Briefly, therefore, the present invention is directed to the taxane composition, per se, to pharmaceutical compositions comprising the taxane and a pharmaceutically acceptable carrier, and to methods of administration.

Other objects and features of this invention will be in part apparent and in part pointed out hereinafter.

### DETAILED DESCRIPTION OF THE PREFERRED EMBODIMENTS

In one embodiment of the present invention, the taxanes of the present invention correspond to structure (1): 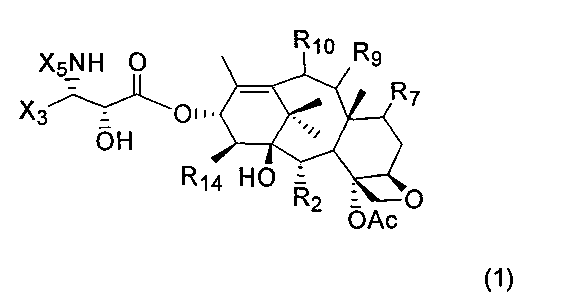 wherein
R₂ is acyloxy;
R₇ is hydroxy;
R₉ is keto, hydroxy, or acyloxy;
R₁₀ is carbonate;
R₁₄ is hydrido or hydroxy;
X₃ is substituted or unsubstituted alkyl, alkenyl, alkynyl, phenyl or heterocyclo, wherein alkyl comprises at least two carbon atoms;
X₅ is -COX₁₀, -COOX₁₀, or -CONHX₁₀;
X₁₀ is hydrocarbyl, substituted hydrocarbyl, or heterocyclo;
Ac is acetyl; and
R₇, R₉, and R₁₀ independently have the alpha or beta stereochemical configuration.

In one embodiment, R₂ is an ester (R₂ₐC(O)O-), a carbamate (R₂ₐR_{2b}NC(O)O-), a carbonate (R₂ₐOC(O)O-), or a thiocarbamate (R₂ₐSC(O)O-) wherein R₂ₐ and R_{2b} are independently hydrogen, hydrocarbyl, substituted hydrocarbyl or heterocyclo. In a preferred embodiment, R₂ is an ester (R₂ₐC(O)O-), wherein R₂ₐ is aryl or heteroaromatic. In another preferred embodiment, R₂ is an ester (R₂ₐC(O)O-), wherein R₂ₐ is substituted or unsubstituted phenyl, furyl, thienyl, or pyridyl. In one particularly preferred embodiment, R₂ is benzoyloxy.

While R₉ is keto in one embodiment of the present invention, in other embodiments R₉ may have the alpha or beta stereochemical configuration, preferably the beta stereochemical configuration, and may be, for example, α- or β-hydroxy or α- or β-acyloxy. For example, when R₉ is acyloxy, it may be an ester (R₉ₐC(O)O-), a carbamate (R₉ₐR_{9b}NC(O)O-), a carbonate (R₉ₐOC(O)O-), or a thiocarbamate (R₉ₐSC(O)O-) wherein R₉ₐ and R_{9b} are independently hydrogen, hydrocarbyl, substituted hydrocarbyl or heterocyclo. If R₉ is an ester (R₉ₐC(O)O-), R₉ₐ is substituted or unsubstituted alkyl, substituted or unsubstituted alkenyl, substituted or unsubstituted aryl or substituted or unsubstituted heteroaromatic. Still more preferably, R₉ is an ester (R₉ₐC(O)O-), wherein R₉ₐ is substituted or unsubstituted phenyl, substituted or unsubstituted furyl, substituted or unsubstituted thienyl, or substituted or unsubstituted pyridyl. In one embodiment R₉ is (R₉ₐC(O)O-) wherein R₉ₐ is methyl, ethyl, propyl (straight, branched or cyclic), butyl (straight, branched or cyclic), pentyl, (straight, branched or cyclic), or hexyl (straight, branched or cyclic). In another embodiment R₉ is (R₉ₐC(O)O-) wherein R₉ₐ is substituted methyl, substituted ethyl, substituted propyl (straight, branched or cyclic), substituted butyl (straight, branched or cyclic), substituted pentyl, (straight, branched or cyclic), or substituted hexyl (straight, branched or cyclic) wherein the substituent(s) is/are selected from the group consisting of heterocyclo, alkoxy, alkenoxy, alkynoxy, aryloxy, hydroxy, protected hydroxy, keto, acyloxy, nitro, amino, amido, thiol, ketal, acetal, ester and ether moieties, but not phosphorous containing moieties.

In one embodiment, R₁₀ is R₁₀ₐOCOO- wherein R₁₀ₐ is (i) substituted or unsubstituted C₁ to C₈ alkyl (straight, branched or cyclic), such as methyl, ethyl, propyl, butyl, pentyl, or hexyl; (ii) substituted or unsubstituted C₂ to C₈ alkenyl (straight, branched or cyclic), such as ethenyl, propenyl, butenyl, pentenyl or hexenyl; (iii) substituted or unsubstituted C₂ to C₈ alkynyl (straight or branched) such as ethynyl, propynyl, butynyl, pentynyl, or hexynyl; (iv) substituted or unsubstituted phenyl; or (v) substituted or unsubstituted heterocyclo such as furyl, thienyl, or pyridyl. The substituents may be hydrocarbyl or any of the heteroatom containing substituents identified elsewhere herein for substituted hydrocarbyl. In a preferred embodiment, R₁₀ₐ is methyl, ethyl, straight, branched or cyclic propyl, straight, branched or cyclic butyl, straight, branched or cyclic hexyl, straight or branched propenyl, isobutenyl, furyl or thienyl. In another embodiment, R₁₀ₐ is substituted ethyl, substituted propyl (straight, branched or cyclic), substituted propenyl (straight or branched), substituted isobutenyl, substituted furyl or substituted thienyl wherein the substituent(s) is/are selected from the group consisting of heterocyclo, alkoxy, alkenoxy, alkynoxy, aryloxy, hydroxy, protected hydroxy, keto, acyloxy, nitro, amino, amido, thiol, ketal, acetal, ester and ether moieties, but not phosphorous containing moieties.

Exemplary X₃ substituents include substituted or unsubstituted C₂ to C₈ alkyl, substituted or unsubstituted C₂ to C₈ alkenyl, substituted or unsubstituted C₂ to C₈ alkynyl, substituted or unsubstituted heteroaromatics containing 5 or 6 ring atoms, and substituted or unsubstituted phenyl. Exemplary preferred X₃ substituents include substituted or unsubstituted ethyl, propyl, butyl, cyclopropyl, cyclobutyl, cyclohexyl, isobutenyl, furyl, thienyl, and pyridyl.

Exemplary X₅ substituents include -COX₁₀, -COOX₁₀ or -CONHX₁₀ wherein X₁₀ is substituted or unsubstituted alkyl, alkenyl, phenyl or heteroaromatic. Exemplary preferred X₅ substituents include -COX₁₀, -COOX₁₀ or -CONHX₁₀ wherein X₁₀ is (i) substituted or unsubstituted C₁ to C₈ alkyl such as substituted or unsubstituted methyl, ethyl, propyl (straight, branched or cyclic), butyl (straight, branched or cyclic), pentyl (straight, branched or cyclic), or hexyl (straight, branched or cyclic); (ii) substituted or unsubstituted C₂ to C₈ alkenyl such as substituted or unsubstituted ethenyl, propenyl (straight, branched or cyclic), butenyl (straight, branched or cyclic), pentenyl (straight, branched or cyclic) or hexenyl (straight, branched or cyclic); (iii) substituted or unsubstituted C₂ to C₈ alkynyl such as substituted or unsubstituted ethynyl, propynyl (straight or branched), butynyl (straight or branched), pentynyl (straight or branched), or hexynyl (straight or branched); (iv) substituted or unsubstituted phenyl, or (v) substituted or unsubstituted heteroaromatic such as furyl, thienyl, or pyridyl, wherein the substituent(s) is/are selected from the group consisting of heterocyclo, alkoxy, alkenoxy, alkynoxy, aryloxy, hydroxy, protected hydroxy, keto, acyloxy, nitro, amino, amido, thiol, ketal, acetal, ester and ether moieties, but not phosphorous containing moieties.

In one embodiment, the taxanes of the present invention correspond to structure (2): 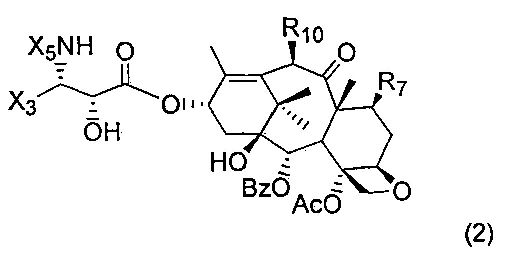 wherein
R₇ is hydroxy;
R₁₀ is carbonate;
X₃ is substituted or unsubstituted alkyl, alkenyl, alkynyl, or heterocyclo, wherein alkyl comprises at least two carbon atoms;
X₅ is -COX₁₀, -COOX₁₀, or -CONHX₁₀; and
X₁₀ is hydrocarbyl, substituted hydrocarbyl, or heterocyclo.
For example, in this preferred embodiment in which the taxane corresponds to structure (2), R₁₀ may be R₁₀ₐOCOO- wherein R₁₀ₐ is substituted or unsubstituted methyl, ethyl, propyl, butyl, pentyl or hexyl, more preferably substituted or unsubstituted methyl, ethyl or propyl, still more preferably substituted or unsubstituted methyl, ethyl, and still more preferably unsubstituted methyl or ethyl. While R₇ₐ is selected from among these, in one embodiment X₃ is selected from substituted or unsubstituted alkyl, alkenyl, phenyl or heterocyclo, more preferably substituted or unsubstituted alkenyl, phenyl or heterocyclo, still more preferably substituted or unsubstituted phenyl or heterocyclo, and still more preferably heterocyclo such as furyl, thienyl or pyridyl. While R₁₀ₐ and X₃ are selected from among these, in one embodiment X₅ is selected from -COX₁₀ wherein X₁₀ is phenyl, alkyl or heterocyclo, more preferably phenyl. Alternatively, while R₁₀ₐ and X₃ are selected from among these, in one embodiment X₅ is selected from -COX₁₀ wherein X₁₀ is phenyl, alkyl or heterocyclo, more preferably phenyl, or X₅ is -COOX₁₀ wherein X₁₀ is alkyl, preferably t-butyl. Among the more preferred embodiments, therefore, are taxanes corresponding to structure 2 in which (i) X₅ is -COOX₁₀ wherein X₁₀ is tert-butyl or X₅ is -COX₁₀ wherein X₁₀ is phenyl, (ii) X₃ is substituted or unsubstituted cycloalkyl, alkenyl, phenyl or heterocyclo, more preferably substituted or unsubstituted isobutenyl, phenyl, furyl, thienyl, or pyridyl, still more preferably unsubstituted isobutenyl, furyl, thienyl or pyridyl, and (iii) R₁₀ₐ is unsubstituted methyl, ethyl or propyl, more preferably methyl or ethyl.

Among the preferred embodiments in which the taxane corresponds to structure 1 or 2 wherein R₁₀ is R₁₀ₐOCOO-, R₁₀ₐ may be substituted or unsubstituted ethyl or propyl, and more preferably unsubstituted ethyl or propyl. While R₁₀ₐ is selected from among these, in one embodiment X₃ is selected from substituted or unsubstituted cycloalkyl, phenyl or heterocyclo, more preferably substituted or unsubstituted cycloalkyl, phenyl, furyl, thienyl or pyridyl. While R₁₀ₐ and X₃ are selected from among these, in one embodiment X₅ is selected from COOX₁₀ wherein X₁₀ is tert-butyl, tert-amyl, isobutyl, isopropyl or substituted or unsubstituted cycloalkyl. R₂, R₉ and R₁₄ are as defined in structures 1 and 2, and are preferably benzoyloxy, keto and hydrido, respectively. Among the more preferred embodiments, therefore, are taxanes corresponding to structure 2 in which (i) X₅ is -COOX₁₀ wherein X₁₀ is tert-butyl, tert-amyl, isobutyl, isopropyl, or unsubstituted cycloalkyl, and more preferably tert-butyl, (ii) X₃ is substituted or unsubstituted cycloalkyl, phenyl, furyl, thienyl, or pyridyl, and more preferably unsubstituted cycloalkyl, phenyl, furyl, thienyl or pyridyl, and (iii) R₁₀ₐ is unsubstituted ethyl or propyl. In each of the alternatives of this embodiment when the taxane has structure 1, R₇ and R₁₀ may each have the beta stereochemical configuration, R₇ and R₁₀ may each have the alpha stereochemical configuration, R₇ may have the alpha stereochemical configuration while R₁₀ has the beta stereochemical configuration or R₇ may have the beta stereochemical configuration while R₁₀ has the alpha stereochemical configuration.

Among the preferred embodiments are taxanes corresponding to structure 1 or 2 wherein R₁₀ is R₁₀ₐOCOO- wherein R₁₀ₐ is methyl. In this embodiment, X₃ is preferably cycloalkyl, isobutenyl, or heterocyclo, more preferably heterocyclo, still more preferably furyl, thienyl or pyridyl; and X₅ is preferably benzoyl, alkoxycarbonyl, or heterocyclocarbonyl, more preferably benzoyl, t-butoxycarbonyl or t-amyloxycarbonyl, still more preferably t-butoxycarbonyl. In one alternative of this embodiment, X₃ is heterocyclo; X₅ is benzoyl, alkoxycarbonyl, or heterocyclocarbonyl, more preferably benzoyl, t-butoxycarbonyl or t-amyloxycarbonyl, still more preferably t-butoxycarbonyl; R₂ is benzoyl, R₉ is keto and R₁₄ is hydrido. In another alternative of this embodiment, X₃ is heterocyclo; X₅ is benzoyl, alkoxycarbonyl, or heterocyclocarbonyl, more preferably benzoyl, t-butoxycarbonyl or t-amyloxycarbonyl, still more preferably t-butoxycarbonyl; R₂ is benzoyl, R₉ is keto and R₁₄ is hydrido. In another alternative of this embodiment, X₃ is heterocyclo; X₅ is benzoyl, alkoxycarbonyl, or heterocyclocarbonyl, more preferably benzoyl, t-butoxycarbonyl or t-amyloxycarbonyl, still more preferably t-butoxycarbonyl; R₂ is benzoyl, R₉ is keto and R₁₄ is hydroxy. In another alternative of this embodiment, X₃ is heterocyclo; X₅ is benzoyl, alkoxycarbonyl, or heterocyclocarbonyl, more preferably benzoyl, t-butoxycarbonyl or t-amyloxycarbonyl, still more preferably t-butoxycarbonyl; R₂ is benzoyl, R₉ is hydroxy and R₁₄ is hydroxy. In another alternative of this embodiment, X₃ is heterocyclo; X₅ is benzoyl, alkoxycarbonyl, or heterocyclocarbonyl, more preferably benzoyl, t-butoxycarbonyl or t-amyloxycarbonyl, still more preferably t-butoxycarbonyl; R₂ is benzoyl, R₉ is hydroxy and R₁₄ is hydrido. In another alternative of this embodiment, X₃ is heterocyclo; X₅ is benzoyl, alkoxycarbonyl, or heterocyclocarbonyl, more preferably benzoyl, t-butoxycarbonyl or t-amyloxycarbonyl, still more preferably t-butoxycarbonyl; R₂ is benzoyl, R₉ is acyloxy and R₁₄ is hydroxy. In another alternative of this embodiment, X₃ is heterocyclo; X₅ is benzoyl, alkoxycarbonyl, or heterocyclocarbonyl, more preferably benzoyl, t-butoxycarbonyl or t-amyloxycarbonyl, still more preferably t-butoxycarbonyl; R₂ is benzoyl, R₉ is acyloxy and R₁₄ is hydrido. In each of the alternatives of this embodiment when the taxane has structure 1, R₇ and R₁₀ may each have the beta stereochemical configuration, R₇ and R₁₀ may each have the alpha stereochemical configuration, R₇ may have the alpha stereochemical configuration while R₁₀ has the beta stereochemical configuration or R₇ may have the beta stereochemical configuration while R₁₀ has the alpha stereochemical configuration.

Also among the preferred embodiments are taxanes corresponding to structure 1 or 2 wherein R₁₀ is R₁₀ₐOCOO- wherein R₁₀ₐ is ethyl. In this embodiment, X₃ is preferably cycloalkyl, isobutenyl, phenyl, substituted phenyl such as p-nitrophenyl, or heterocyclo, more preferably heterocyclo, still more preferably furyl, thienyl or pyridyl; and X₅ is preferably benzoyl, alkoxycarbonyl, or heterocyclocarbonyl, more preferably benzoyl, t-butoxycarbonyl or t-amyloxycarbonyl. In one alternative of this embodiment, X₃ is heterocycto; X₅ is benzoyl, alkoxycarbonyl, or heterocyclocarbonyl, more preferably benzoyl, t-butoxycarbonyl or t-amyloxycarbonyl, still more preferably t-butoxycarbonyl; R₂ is benzoyl, R₉ is keto and R₁₄ is hydrido. In another alternative of this embodiment, X₃ is heterocyclo; X₅ is benzoyl, alkoxycarbonyl, or heterocyclocarbonyl, more preferably benzoyl, t-butoxycarbonyl or t-amyloxycarbonyl, still more preferably t-butoxycarbonyl; R₂ is benzoyl, R₉ is keto and R₁₄ is hydrido. In another alternative of this embodiment, X₃ is heterocyclo; X₅ is benzoyl, alkoxycarbonyl, or heterocyclocarbonyl, more preferably benzoyl, t-butoxycarbonyl or t-amyloxycarbonyl, still more preferably t-butoxycarbonyl; R₂ is benzoyl, R₉ is keto and R₁₄ is hydroxy. In another alternative of this embodiment, X₃ is heterocyclo; X₅ is benzoyl, alkoxycarbonyl, or heterocyclocarbonyl, more preferably benzoyl, t-butoxycarbonyl or t-amyloxycarbonyl, still more preferably t-butoxycarbonyl; R₂ is benzoyl, R₉ is hydroxy and R₁₄ is hydroxy. In another alternative of this embodiment, X₃ is heterocyclo; X₅ is benzoyl, alkoxycarbonyl, or heterocyclocarbonyl, more preferably benzoyl, t-butoxycarbonyl or t-amyloxycarbonyl, still more preferably t-butoxycarbonyl; R₂ is benzoyl, R₉ is hydroxy and R₁₄ is hydrido. In another alternative of this embodiment, X₃ is heterocyclo; X₅ is benzoyl, alkoxycarbonyl, or heterocyclocarbonyl, more preferably benzoyl, t-butoxycarbonyl or t-amyloxycarbonyl, still more preferably t-butoxycarbonyl; R₂ is benzoyl, R₉ is acyloxy and R₁₄ is hydroxy. In another alternative of this embodiment, X₃ is heterocyclo; X₅ is benzoyl, alkoxycarbonyl, or heterocyclocarbonyl, more preferably benzoyl, t-butoxycarbonyl or t-amyloxycarbonyl, still more preferably t-butoxycarbonyl; R₂ is benzoyl, R₉ is acyloxy and R₁₄ is hydrido. In each of the alternatives of this embodiment when the taxane has structure 1, R₇ and R₁₀ may each have the beta stereochemical configuration, R₇ and R₁₀ may each have the alpha stereochemical configuration, R₇ may have the alpha stereochemical configuration while R₁₀ has the beta stereochemical configuration or R₇ may have the beta stereochemical configuration while R₁₀ has the alpha stereochemical configuration.

Also among the preferred embodiments are taxanes corresponding to structure 1 or 2 wherein R₁₀ is R₁₀ₐOCOO- wherein R₁₀ₐ is propyl. In this embodiment, X₃ is preferably cycloalkyl, isobutenyl, phenyl, substituted phenyl such as p-nitrophenyl, or heterocyclo, more preferably heterocyclo, still more preferably furyl, thienyl or pyridyl; and X₅ is preferably benzoyl, alkoxycarbonyl, or heterocyclocarbonyl, more preferably benzoyl, t-butoxycarbonyl or t-amyloxycarbonyl. In one alternative of this embodiment, X₃ is heterocyclo; X₅ is benzoyl, alkoxycarbonyl, or heterocyclocarbonyl, more preferably benzoyl, t-butoxycarbonyl or t-amyloxycarbonyl, still more preferably t-butoxycarbonyl; R₂ is benzoyl, R₉ is keto and R₁₄ is hydrido. In another alternative of this embodiment, X₃ is heterocyclo; X₅ is benzoyl, alkoxycarbonyl, or heterocyclocarbonyl, more preferably benzoyl, t-butoxycarbonyl or t-amyloxycarbonyl, still more preferably t-butoxycarbonyl; R₂ is benzoyl, R₉ is keto and R₁₄ is hydrido. In another alternative of this embodiment, X₃ is heterocyclo; X₅ is benzoyl, alkoxycarbonyl, or heterocyclocarbonyl, more preferably benzoyl, t-butoxycarbonyl or t-amyloxycarbonyl, still more preferably t-butoxycarbonyl; R₂ is benzoyl, R₉ is keto and R₁₄ is hydroxy. In another alternative of this embodiment, X₃ is heterocyclo; X₅ is benzoyl, alkoxycarbonyl, or heterocyclocarbonyl, more preferably benzoyl, t-butoxycarbonyl or t-amyloxycarbonyl, still more preferably t-butoxycarbonyl; R₂ is benzoyl, R₉ is hydroxy and R₁₄ is hydroxy. In another alternative of this embodiment, X₃ is heterocyclo; X₅ is benzoyl, alkoxycarbonyl, or heterocyclocarbonyl, more preferably benzoyl, t-butoxycarbonyl or t-amyloxycarbonyl, still more preferably t-butoxycarbonyl; R₂ is benzoyl, R₉ is hydroxy and R₁₄ is hydrido. In another alternative of this embodiment, X₃ is heterocyclo; X₅ is benzoyl, alkoxycarbonyl, or heterocyclocarbonyl, more preferably benzoyl, t-butoxycarbonyl or t-amyloxycarbonyl, still more preferably t-butoxycarbonyl; R₂ is benzoyl, R₉ is acyloxy and R₁₄ is hydroxy. In another alternative of this embodiment, X₃ is heterocyclo; X₅ is benzoyl, alkoxycarbonyl, or heterocyclocarbonyl, more preferably benzoyl, t-butoxycarbonyl or t-amyloxycarbonyl, still more preferably t-butoxycarbonyl; R₂ is benzoyl, R₉ is acyloxy and R₁₄ is hydrido. In each of the alternatives of this embodiment when the taxane has structure 1, R₇ and R₁₀ may each have the beta stereochemical configuration, R₇ and R₁₀ may each have the alpha stereochemical configuration, R₇ may have the alpha stereochemical configuration while R₁₀ has the beta stereochemical configuration or R₇ may have the beta stereochemical configuration while R₁₀ has the alpha stereochemical configuration.

Taxanes having the general formula 1 may be obtained by treatment of a β-lactam with an alkoxide having the taxane tetracyclic nucleus and a C-13 metallic oxide substituent to form compounds having a β-amido ester substituent at C(13), as described more fully in Holton U.S. Patent 5,466,834, followed by removal of the hydroxy protecting groups. The β-lactam has the following structural formula (3): 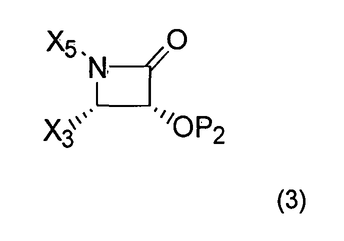 wherein P₂ is a hydroxy protecting group and X₃ and X₅ are as previously defined and the alkoxide has the structural formula (4): 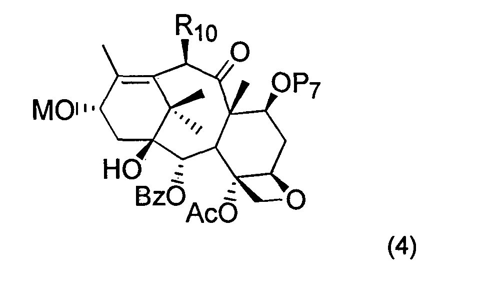 wherein M is a metal or ammonium, P₇ is a hydroxy protecting group and R₁₀ is as previously defined.

The alkoxide may be prepared from 10-deacetylbaccatin III by selective formation of a carbonate of the C-10 hydroxyl group and then protection of the C-7 hydroxyl group (as described more fully in Holton et al., PCT Patent Application WO 99/09021, followed by treatment with a metallic amide. Acylating agents which may be used for the selective acylation of the C(10) hydroxyl group of a taxane include dimethyldicarbonate, diethyldicarbonate, di-t-butyldicarbonate, dibenzyldicarbonate and the like. While the acylation of the C(10) hydroxy group of the taxane will proceed at an adequate rate for many acylating agents, it has been discovered that the reaction rate may be increased by including a Lewis acid in the reaction mixture. Preferred Lewis acids include zinc chloride, stannic chloride, cerium trichloride, cuprous chloride, lanthanum trichloride, dysprosium trichloride, and ytterbium trichloride. Zinc chloride or cerium trichloride is particularly preferred when the acylating agent is a dicarbonate.

Derivatives of 10-deacetylbaccatin III having alternative substituents at C(2), C(9) and C(14) and processes for their preparation are known in the art. Taxane derivatives having acyloxy substituents other than benzoyloxy at C(2) may be prepared, for example, as described in Holton et al., U.S. Patent No. 5,728,725 or Kingston et al., U.S. Patent No. 6,002,023. Taxanes having acyloxy or hydroxy substituents at C(9) in place of keto may be prepared, for example as described in Holton et al., U.S. Patent No. 6,011,056 or Gunawardana et al., U.S. Patent No. 5,352,806. Taxanes having a beta hydroxy substituent at C(14) may be prepared from naturally occurring 14-hydroxy-10-deacetylbaccatin III.

Processes for the preparation and resolution of the β-lactam starting material are generally well known. For example, the β-lactam may be prepared as described in Holton, U.S. Patent No. 5,430,160 and the resulting enatiomeric mixtures of β-lactams may be resolved by a stereoselective hydrolysis using a lipase or enzyme as described, for example, in Patel, U.S. Patent No. 5,879,929 Patel U.S. Patent No. 5,567,614 or a liver homogenate as described, for example, in PCT Patent Application No. 00/41204. In a preferred embodiment in which the β-lactam is furyl substituted at the C(4) position, the β-lactam can be prepared as illustrated in the following reaction scheme: 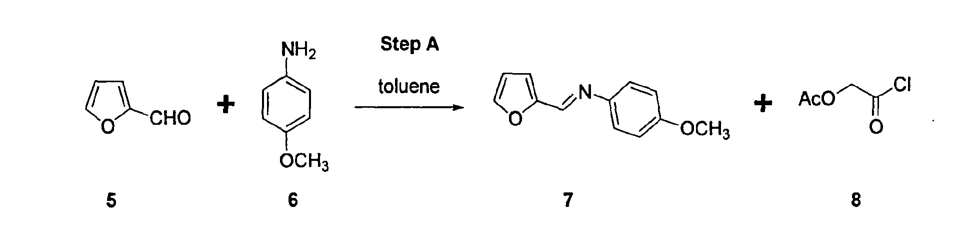 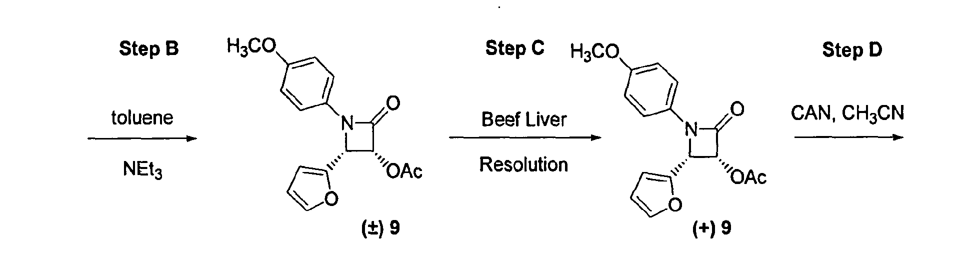 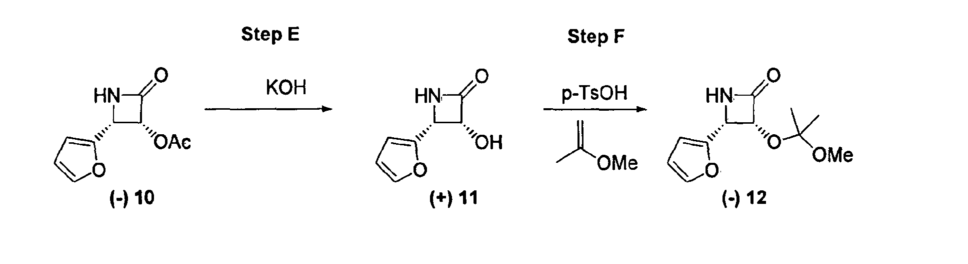 Wherein Ac is acetyl, NEt₃ is triethylamine, CAN is ceric ammonium nitrate, and p-TsOH is p-toluenesulfonic acid. The beef liver resolution may be carried out, for example, by combining the enatiomeric β-lactam mixture with a beef liver suspension (prepared, for example, by adding 20 g of frozen beef liver to a blender and then adding a pH 8 buffer to make a total volume of 1 L).

Compounds of formula 1 of the instant invention are useful for inhibiting tumor growth in mammals including humans and are preferably administered in the form of a pharmaceutical composition comprising an effective antitumor amount of a compound of the instant invention in combination with at least one pharmaceutically or pharmacologically acceptable carrier. The carrier, also known in the art as an excipient, vehicle, auxiliary, adjuvant, or diluent, is any substance which is pharmaceutically inert, confers a suitable consistency or form to the composition, and does not diminish the therapeutic efficacy of the antitumor compounds. The carrier is "pharmaceutically or pharmacologically acceptable" if it does not produce an adverse, allergic or other untoward reaction when administered to a mammal or human, as appropriate.

The pharmaceutical compositions containing the antitumor compounds of the present invention may be formulated in any conventional manner. Proper formulation is dependent upon the route of administration chosen. The compositions of the invention can be formulated for any route of administration so long as the target tissue is available via that route. Suitable routes of administration include, but are not limited to, oral, parenteral (e.g., intravenous, intraarterial, subcutaneous, rectal, subcutaneous, intramuscular, intraorbital, intracapsular, intraspinal, intraperitoneal, or intrasternal), topical (nasal, transdermal, intraocular), intravesical, intrathecal, enteral, pulmonary, intralymphatic, intracavital, vaginal, transurethral, intradermal, aural, intramammary, buccal, orthotopic, intratracheal, intralesional, percutaneous, endoscopical, transmucosal, sublingual and intestinal administration.

Pharmaceutically acceptable carriers for use in the compositions of the present invention are well known to those of ordinary skill in the art and are selected based upon a number of factors: the particular antitumor compound used, and its concentration, stability and intended bioavailability; the disease, disorder or condition being treated with the composition; the subject, its age, size and general condition; and the route of administration. Suitable carriers are readily determined by one of ordinary skill in the art (see, for example, J. G. Nairn, in: Remington's Pharmaceutical Science (A. Gennaro, ed.), Mack Publishing Co., Easton, Pa., (1985), pp. 1492-1517, the contents of which are incorporated herein by reference).

The compositions are preferably formulated as tablets, dispersible powders, pills, capsules, gelcaps, caplets, gels, liposomes, granules, solutions, suspensions, emulsions, syrups, elixirs, troches, dragees, lozenges, or any other dosage form which can be administered orally. Techniques and compositions for making oral dosage forms useful in the present invention are described in the following references: 7 Modern Pharmaceutics, Chapters 9 and 10 (Banker & Rhodes, Editors, 1979); Lieberman et al., Pharmaceutical Dosage Forms: Tablets (1981); and Ansel, Introduction to Pharmaceutical Dosage Forms 2nd Edition (1976).

The compositions of the invention for oral administration comprise an effective antitumor amount of a compound of the invention in a pharmaceutically acceptable carrier. Suitable carriers for solid dosage forms include sugars, starches, and other conventional substances including lactose, talc, sucrose, gelatin, carboxymethylcellulose, agar, mannitol, sorbitol, calcium phosphate, calcium carbonate, sodium carbonate, kaolin, alginic acid, acacia, corn starch, potato starch, sodium saccharin, magnesium carbonate, tragacanth, microcrystalline cellulose, colloidal silicon dioxide, croscarmellose sodium, talc, magnesium stearate, and stearic acid. Further, such solid dosage forms may be uncoated or may be coated by known techniques; e.g., to delay disintegration and absorption.

The antitumor compounds of the present invention are also preferably formulated for parenteral administration, e.g., formulated for injection via intravenous, intraarterial, subcutaneous, rectal, subcutaneous, intramuscular, intraorbital, intracapsular, intraspinal, intraperitoneal, or intrasternal routes. The compositions of the invention for parenteral administration comprise an effective antitumor amount of the antitumor compound in a pharmaceutically acceptable carrier. Dosage forms suitable for parenteral administration include solutions, suspensions, dispersions, emulsions or any other dosage form which can be administered parenterally. Techniques and compositions for making parenteral dosage forms are known in the art.

Suitable carriers used in formulating liquid dosage forms for oral or parenteral administration include nonaqueous, pharmaceutically-acceptable polar solvents such as oils, alcohols, amides, esters, ethers, ketones, hydrocarbons and mixtures thereof, as well as water, saline solutions, dextrose solutions (e.g., DW5), electrolyte solutions, or any other aqueous, pharmaceutically acceptable liquid.

Suitable nonaqueous, pharmaceutically-acceptable polar solvents include, but are not limited to, alcohols (e.g., α-glycerol formal, β-glycerol formal, 1, 3-butyleneglycol, aliphatic or aromatic alcohols having 2-30 carbon atoms such as methanol, ethanol, propanol, isopropanol, butanol, t-butanol, hexanol, octanol, amylene hydrate, benzyl alcohol, glycerin (glycerol), glycol, hexylene glycol, tetrahydrofurfuryl alcohol, lauryl alcohol, cetyl alcohol, or stearyl alcohol, fatty acid esters of fatty alcohols such as polyalkylene glycols (e.g., polypropylene glycol, polyethylene glycol), sorbitan, sucrose and cholesterol); amides (e.g., dimethylacetamide (DMA), benzyl benzoate DMA, dimethylformamide, N-(β-hydroxyethyl)-lactamide, N, N-dimethylacetamide_amides, 2-pyrrolidinone, 1-methyl-2-pyrrolidinone, or polyvinylpyrrolidone); esters (e.g., 1-methyl-2-pyrrolidinone, 2-pyrrolidinone, acetate esters such as monoacetin, diacetin, and triacetin, aliphatic or aromatic esters such as ethyl caprylate or octanoate, alkyl oleate, benzyl benzoate, benzyl acetate, dimethylsulfoxide (DMSO), esters of glycerin such as mono, di, or triglyceryl citrates or tartrates, ethyl benzoate, ethyl acetate, ethyl carbonate, ethyl lactate, ethyl oleate, fatty acid esters of sorbitan, fatty acid derived PEG esters, glyceryl monostearate, glyceride esters such as mono, di, or tri-glycerides, fatty acid esters such as isopropyl myristrate, fatty acid derived PEG esters such as PEG-hydroxyoleate and PEG-hydroxystearate, N-methyl pyrrolidinone, pluronic 60, polyoxyethylene sorbitol oleic polyesters such as poly(ethoxylated)₃₀₋₆₀ sorbitol poly(oleate)_{2-4,} poly(oxyethylene)₁₅₋₂₀ monooleate, poly(oxyethylene)₁₅₋₂₀ mono 12-hydroxystearate, and poly(oxyethylene)₁₅₋₂₀ mono ricinoleate, polyoxyethylene sorbitan esters such as polyoxyethylene-sorbitan monooleate, polyoxyethylene-sorbitan monopalmitate, polyoxyethylene-sorbitan monolaurate, polyoxyethylene-sorbitan monostearate, and Polysorbate® 20, 40, 60 or 80 from ICI Americas, Wilmington, DE, polyvinylpyrrolidone, alkyleneoxy modified fatty acid esters such as polyoxyl 40 hydrogenated castor oil and polyoxyethylated castor oils (e.g., Cremophor® EL solution or Cremophor® RH 40 solution), saccharide fatty acid esters (i.e., the condensation product of a monosaccharide (e.g., pentoses such as ribose, ribulose, arabinose, xylose, lyxose and xylulose, hexoses such as glucose, fructose, galactose, mannose and sorbose, trioses, tetroses, heptoses, and octoses), disaccharide (e.g., sucrose, maltose, lactose and trehalose) or oligosaccharide or mixture thereof with a C₄-C₂₂ fatty acid(s)(e.g., saturated fatty acids such as caprylic acid, capric acid, lauric acid, myristic acid, palmitic acid and stearic acid, and unsaturated fatty acids such as palmitoleic acid, oleic acid, elaidic acid, erucic acid and linoleic acid)), or steroidal esters); alkyl, aryl, or cyclic ethers having 2-30 carbon atoms (e.g., diethyl ether, tetrahydrofuran, dimethyl isosorbide, diethylene glycol monoethyl ether); glycofurol (tetrahydrofurfuryl alcohol polyethylene glycol ether); ketones having 3-30 carbon atoms (e.g., acetone, methyl ethyl ketone, methyl isobutyl ketone); aliphatic, cycloaliphatic or aromatic hydrocarbons having 4-30 carbon atoms (e.g., benzene, cyclohexane, dichloromethane, dioxolanes, hexane, n-decane, n-dodecane, n-hexane, sulfolane, tetramethylenesulfon, tetramethylenesulfoxide, toluene, dimethylsulfoxide (DMSO), or tetramethylenesulfoxide); oils of mineral, vegetable, animal, essential or synthetic origin (e.g., mineral oils such as aliphatic or wax-based hydrocarbons, aromatic hydrocarbons, mixed aliphatic and aromatic based hydrocarbons, and refined paraffin oil, vegetable oils such as linseed, tung, safflower, soybean, castor, cottonseed, groundnut, rapeseed, coconut, palm, olive, corn, corn germ, sesame, persic and peanut oil and glycerides such as mono-, di- or triglycerides, animal oils such as fish, marine, sperm, cod-liver, haliver, squalene, squalane, and shark liver oil, oleic oils, and polyoxyethylated castor oil); alkyl or aryl halides having 1-30 carbon atoms and optionally more than one halogen substituent; methylene chloride; monoethanolamine; petroleum benzin; trolamine; omega-3 polyunsaturated fatty acids (e.g., alpha-linolenic acid, eicosapentaenoic acid, docosapentaenoic acid, or docosahexaenoic acid); polyglycol ester of 12-hydroxystearic acid and polyethylene glycol (Solutol® HS-15, from BASF, Ludwigshafen, Germany); polyoxyethylene glycerol; sodium laurate; sodium oleate; or sorbitan monooleate.

Other pharmaceutically acceptable solvents for use in the invention are well known to those of ordinary skill in the art, and are identified in The Chemotherapy Source Book (Williams & Wilkens Publishing), The Handbook of Pharmaceutical Excipients, (American Pharmaceutical Association, Washington, D.C., and The Pharmaceutical Society of Great Britain, London, England, 1968), Modern Pharmaceutics, (G. Banker et al., eds., 3d ed.)(Marcel Dekker, Inc., New York, New York, 1995), The Pharmacological Basis of Therapeutics, (Goodman & Gilman, McGraw Hill Publishing), Pharmaceutical Dosage Forms, (H. Lieberman et al., eds., )(Marcel Dekker, Inc., New York, New York, 1980), Remington's Pharmaceutical Sciences (A. Gennaro, ed., 19th ed.)(Mack Publishing, Easton, PA, 1995), The United States Pharmacopeia 24, The National Formulary 19, (National Publishing, Philadelphia, PA, 2000), A.J. Spiegel et al., and Use of Nonaqueous Solvents in Parenteral Products, JOURNAL OF PHARMACEUTICAL SCIENCES, Vol. 52, No. 10, pp. 917-927 (1963).

Preferred solvents include those known to stabilize the antitumor compounds, such as oils rich in triglycerides, for example, safflower oil, soybean oil or mixtures thereof, and alkyleneoxy modified fatty acid esters such as polyoxyl 40 hydrogenated castor oil and polyoxyethylated castor oils (e.g., Cremophor® EL solution or Cremophor® RH 40 solution). Commercially available triglycerides include Intralipid® emulsified soybean oil (Kabi-Pharmacia Inc., Stockholm, Sweden), Nutralipid ® emulsion (McGaw, Irvine, California), Liposyn® II 20% emulsion (a 20% fat emulsion solution containing 100 mg safflower oil, 100 mg soybean oil, 12 mg egg phosphatides, and 25 mg glycerin per ml of solution; Abbott Laboratories, Chicago, Illinois), Liposyn® III 2% emulsion (a 2% fat emulsion solution containing 100 mg safflower oil, 100 mg soybean oil, 12 mg egg phosphatides, and 25 mg glycerin per ml of solution; Abbott Laboratories, Chicago, Illinois), natural or synthetic glycerol derivatives containing the docosahexaenoyl group at levels between 25% and 100% by weight based on the total fatty acid content (Dhasco® (from Martek Biosciences Corp., Columbia, MD), DHA Maguro® (from Daito Enterprises, Los Angeles, CA), Soyacal®, and Travemulsion®. Ethanol is a preferred solvent for use in dissolving the antitumor compound to form solutions, emulsions, and the like.

Additional minor components can be included in the compositions of the invention for a variety of purposes well known in the pharmaceutical industry. These components will for the most part impart properties which enhance retention of the antitumor compound at the site of administration, protect the stability of the composition, control the pH, facilitate processing of the antitumor compound into pharmaceutical formulations, and the like. Preferably, each of these components is individually present in less than about 15 weight % of the total composition, more preferably less than about 5 weight %, and most preferably less than about 0.5 weight % of the total composition. Some components, such as fillers or diluents, can constitute up to 90 wt.% of the total composition, as is well known in the formulation art. Such additives include cryoprotective agents for preventing reprecipitation of the taxane, surface active, wetting or emulsifying agents (e.g., lecithin, polysorbate-80, Tween® 80, pluronic 60, polyoxyethylene stearate), preservatives (e.g., ethyl-p-hydroxybenzoate), microbial preservatives (e.g., benzyl alcohol, phenol, m-cresol, chlorobutanol, sorbic acid, thimerosal and paraben), agents for adjusting pH or buffering agents (e.g., acids, bases, sodium acetate, sorbitan monolaurate), agents for adjusting osmolarity (e.g., glycerin), thickeners (e.g., aluminum monostearate, stearic acid, cetyl alcohol, stearyl alcohol, guar gum, methyl cellulose, hydroxypropylcellulose, tristearin, cetyl wax esters, polyethylene glycol), colorants, dyes, flow aids, non-volatile silicones (e.g., cyclomethicone), clays (e.g., bentonites), adhesives, bulking agents, flavorings, sweeteners, adsorbents, fillers (e.g., sugars such as lactose, sucrose, mannitol, or sorbitol, cellulose, or calcium phosphate), diluents (e.g., water, saline, electrolyte solutions), binders (e.g., starches such as maize starch, wheat starch, rice starch, or potato starch, gelatin, gum tragacanth, methyl cellulose, hydroxypropyl methylcellulose, sodium carboxymethyl cellulose, polyvinylpyrrolidone, sugars, polymers, acacia), disintegrating agents (e.g., starches such as maize starch, wheat starch, rice starch, potato starch, or carboxymethyl starch, cross-linked polyvinyl pyrrolidone, agar, alginic acid or a salt thereof such as sodium alginate, croscarmellose sodium or crospovidone), lubricants (e.g., silica, talc, stearic acid or salts thereof such as magnesium stearate, or polyethylene glycol), coating agents (e.g., concentrated sugar solutions including gum arabic, talc, polyvinyl pyrrolidone, carbopol gel, polyethylene glycol, or titanium dioxide), and antioxidants (e.g., sodium metabisulfite, sodium bisulfite, sodium sulfite, dextrose, phenols, and thiophenols).

In a preferred embodiment, a pharmaceutical composition of the invention comprises at least one nonaqueous, pharmaceutically acceptable solvent and an antitumor compound having a solubility in ethanol of at least about 100, 200, 300, 400, 500, 600, 700 or 800 mg/ml. While not being bound to a particular theory, it is believed that the ethanol solubility of the antitumor compound may be directly related to its efficacy. The antitumor compound can also be capable of being crystallized from a solution. In other words, a crystalline antitumor compound can be dissolved in a solvent to form a solution and then recrystallized upon evaporation of the solvent without the formation of any amorphous antitumor compound. It is also preferred that the antitumor compound have an ID50 value (i.e, the drug concentration producing 50% inhibition of colony formation) of at least 4, 5, 6, 7, 8, 9, or 10 times less that of paclitaxel when measured according to the protocol set forth in the working examples.

Dosage form administration by these routes may be continuous or intermittent, depending, for example, upon the patient's physiological condition, whether the purpose of the administration is therapeutic or prophylactic, and other factors known to and assessable by a skilled practitioner.

Dosage and regimens for the administration of the pharmaceutical compositions of the invention can be readily determined by those with ordinary skill in treating cancer. It is understood that the dosage of the antitumor compounds will be dependent upon the age, sex, health, and weight of the recipient, kind of concurrent treatment, if any, frequency of treatment, and the nature of the effect desired. For any mode of administration, the actual amount of antitumor compound delivered, as well as the dosing schedule necessary to achieve the advantageous effects described herein, will also depend, in part, on such factors as the bioavailability of the antitumor compound, the disorder being treated, the desired therapeutic dose, and other factors that will be apparent to those of skill in the art. The dose administered to an animal, particularly a human, in the context of the present invention should be sufficient to effect the desired therapeutic response in the animal over a reasonable period of time. Preferably, an effective amount of the antitumor compound, whether administered orally or by another route, is any amount which would result in a desired therapeutic response when administered by that route. Preferably, the compositions for oral administration are prepared in such a way that a single dose in one or more oral preparations contains at least 20 mg of the antitumor compound per m² of patient body surface area, or at least 50, 100, 150, 200, 300, 400, or 500 mg of the antitumor compound per m² of patient body surface area, wherein the average body surface area for a human is 1.8 m². Preferably, a single dose of a composition for oral administration contains from about 20 to about 600 mg of the antitumor compound per m² of patient body surface area, more preferably from about 25 to about 400 mg/m²' even more preferably, from about 40 to about 300 mg/m², and even more preferably from about 50 to about 200 mg/m². Preferably, the compositions for parenteral administration are prepared in such a way that a single dose contains at least 20 mg of the antitumor compound per m² of patient body surface area, or at least 40, 50, 100, 150, 200, 300, 400, or 500 mg of the antitumor compound per m² of patient body surface area. Preferably, a single dose in one or more parenteral preparations contains from about 20 to about 500 mg of the antitumor compound per m² of patient body surface area, more preferably from about 40 to about 400 mg/m²' and even more preferably, from about 60 to about 350 mg/m². However, the dosage may vary depending on the dosing schedule which can be adjusted as necessary to achieve the desired therapeutic effect. It should be noted that the ranges of effective doses provided herein are not intended to limit the invention and represent preferred dose ranges. The most preferred dosage will be tailored to the individual subject, as is understood and determinable by one of ordinary skill in the art without undue experimentation.

The concentration of the antitumor compound in a liquid pharmaceutical composition is preferably between about 0.01 mg and about 10 mg per ml of the composition, more preferably between about 0.1 mg and about 7 mg per ml, even more preferably between about 0.5 mg and about 5 mg per ml, and most preferably between about 1.5 mg and about 4 mg per ml. Relatively low concentrations are generally preferred because the antitumor compound is most soluble in the solution at low concentrations. The concentration of the antitumor compound in a solid pharmaceutical composition for oral administration is preferably between about 5 weight % and about 50 weight %, based on the total weight of the composition, more preferably between about 8 weight % and about 40 weight %, and most preferably between about 10 weight % and about 30 weight %.

In one embodiment, solutions for oral administration are prepared by dissolving an antitumor compound in any pharmaceutically acceptable solvent capable of dissolving the compound (e.g., ethanol or methylene chloride) to form a solution. An appropriate volume of a carrier which is a solution, such as Cremophor® EL solution, is added to the solution while stirring to form a pharmaceutically acceptable solution for oral administration to a patient. If desired, such solutions can be formulated to contain a minimal amount of, or to be free of, ethanol, which is known in the art to cause adverse physiological effects when administered at certain concentrations in oral formulations.

In another embodiment, powders or tablets for oral administration are prepared by dissolving an antitumor compound in any pharmaceutically acceptable solvent capable of dissolving the compound (e.g.,ethanol or methylene chloride) to form a solution. The solvent can optionally be capable of evaporating when the solution is dried under vacuum. An additional carrier can be added to the solution prior to drying, such as Cremophor® EL solution. The resulting solution is dried under vacuum to form a glass. The glass is then mixed with a binder to form a powder. The powder can be mixed with fillers or other conventional tabletting agents and processed to form a tablet for oral administration to a patient. The powder can also be added to any liquid carrier as described above to form a solution, emulsion, suspension or the like for oral administration.

Emulsions for parenteral administration can be prepared by dissolving an antitumor compound in any pharmaceutically acceptable solvent capable of dissolving the compound (e.g., ethanol or methylene chloride) to form a solution. An appropriate volume of a carrier which is an emulsion, such as Liposyn® II or Liposyn® III emulsion, is added to the solution while stirring to form a pharmaceutically acceptable emulsion for parenteral administration to a patient. If desired, such emulsions can be formulated to contain a minimal amount of, or to be free of, ethanol or Cremophor® solution, which are known in the art to cause adverse physiological effects when administered at certain concentrations in parenteral formulations.

Solutions for parenteral administration can be prepared by dissolving an antitumor compound in any pharmaceutically acceptable solvent capable of dissolving the compound (e.g., ethanol or methylene chloride) to form a solution. An appropriate volume of a carrier which is a solution, such as Cremophor® solution, is added to the solution while stirring to form a pharmaceutically acceptable solution for parenteral administration to a patient. If desired, such solutions can be formulated to contain a minimal amount of, or to be free of, ethanol or Cremophor® solution, which are known in the art to cause adverse physiological effects when administered at certain concentrations in parenteral formulations.

If desired, the emulsions or solutions described above for oral or parenteral administration can be packaged in IV bags, vials or other conventional containers in concentrated form and diluted with any pharmaceutically acceptable liquid, such as saline, to form an acceptable taxane concentration prior to use as is known in the art.

### Definitions

The terms "hydrocarbon" and "hydrocarbyl" as used herein describe organic compounds or radicals consisting exclusively of the elements carbon and hydrogen. These moieties include alkyl, alkenyl, alkynyl, and aryl moieties. These moieties also include alkyl, alkenyl, alkynyl, and aryl moieties substituted with other aliphatic or cyclic hydrocarbon groups, such as alkaryl, alkenaryl and alkynaryl. Unless otherwise indicated, these moieties preferably comprise 1 to 20 carbon atoms.

The "substituted hydrocarbyl" moieties described herein are hydrocarbyl moieties which are substituted with at least one atom other than carbon, including moieties in which a carbon chain atom is substituted with a hetero atom such as nitrogen, oxygen, silicon, phosphorous, boron, sulfur, or a halogen atom. These substituents include halogen, heterocyclo, alkoxy, alkenoxy, alkynoxy, aryloxy, hydroxy, protected hydroxy, keto, acyl, acyloxy, nitro, amino, amido, nitro, cyano, thiol, ketals, acetals, esters and ethers.

Unless otherwise indicated, the alkyl groups described herein are preferably lower alkyl containing from one to eight carbon atoms in the principal chain and up to 20 carbon atoms. They may be straight or branched chain or cyclic and include methyl, ethyl, propyl, isopropyl, butyl, hexyl and the like.

Unless otherwise indicated, the alkenyl groups described herein are preferably lower alkenyl containing from two to eight carbon atoms in the principal chain and up to 20 carbon atoms. They may be straight or branched chain or cyclic and include ethenyl, propenyl, isopropenyl, butenyl, isobutenyl, hexenyl, and the like.

Unless otherwise indicated, the alkynyl groups described herein are preferably lower alkynyl containing from two to eight carbon atoms in the principal chain and up to 20 carbon atoms. They may be straight or branched chain and include ethynyl, propynyl, butynyl, isobutynyl, hexynyl, and the like.

The terms "aryl" or "ar" as used herein alone or as part of another group denote optionally substituted homocyclic aromatic groups, preferably monocyclic or bicyclic groups containing from 6 to 12 carbons in the ring portion, such as phenyl, biphenyl, naphthyl, substituted phenyl, substituted biphenyl or substituted naphthyl. Phenyl and substituted phenyl are the more preferred aryl.

The terms "halogen" or "halo" as used herein alone or as part of another group refer to chlorine, bromine, fluorine, and iodine.

The terms "heterocyclo" or "heterocyclic" as used herein alone or as part of another group denote optionally substituted, fully saturated or unsaturated, monocyclic or bicyclic, aromatic or nonaromatic groups having at least one heteroatom in at least one ring, and preferably 5 or 6 atoms in each ring. The heterocyclo group preferably has 1 or 2 oxygen atoms, 1 or 2 sulfur atoms, and/or 1 to 4 nitrogen atoms in the ring, and may be bonded to the remainder of the molecule through a carbon or heteroatom. Exemplary heterocyclo include heteroaromatics such as furyl, thienyl, pyridyl, oxazolyl, pyrrolyl, indolyl, quinolinyl, or isoquinolinyl and the like. Exemplary substituents include one or more of the following groups: hydrocarbyl, substituted hydrocarbyl, keto, hydroxy, protected hydroxy, acyl, acyloxy, alkoxy, alkenoxy, alkynoxy, aryloxy, halogen, amido, amino, nitro, cyano, thiol, ketals, acetals, esters and ethers.

The term "heteroaromatic" as used herein alone or as part of another group denote optionally substituted aromatic groups having at least one heteroatom in at least one ring, and preferably 5 or 6 atoms in each ring. The heteroaromatic group preferably has 1 or 2 oxygen atoms, 1 or 2 sulfur atoms, and/or 1 to 4 nitrogen atoms in the ring, and may be bonded to the remainder of the molecule through a carbon or heteroatom. Exemplary heteroaromatics include furyl, thienyl, pyridyl, oxazolyl, pyrrolyl, indolyl, quinolinyl, or isoquinolinyl and the like. Exemplary substituents include one or more of the following groups: hydrocarbyl, substituted hydrocarbyl, keto, hydroxy, protected hydroxy, acyl, acyloxy, alkoxy, alkenoxy, alkynoxy, aryloxy, halogen, amido, amino, nitro, cyano, thiol, ketals, acetals, esters and ethers.

The term "acyl," as used herein alone or as part of another group, denotes the moiety formed by removal of the hydroxyl group from the group --COOH of an organic carboxylic acid, e.g., RC(O)-, wherein R is R¹, R¹O-, R¹R²N-, or R¹S-, R¹ is hydrocarbyl, heterosubstituted hydrocarbyl, or heterocyclo and R² is hydrogen, hydrocarbyl or substituted hydrocarbyl.

The term "acyloxy," as used herein alone or as part of another group, denotes an acyl group as described above bonded through an oxygen linkage (--O--), e.g., RC(O)O- wherein R is as defined in connection with the term "acyl."

Unless otherwise indicated, the alkoxycarbonyloxy moieties described herein comprise lower hydrocarbon or substituted hydrocarbon or substituted hydrocarbon moieties.

Unless otherwise indicated, the carbamoyloxy moieties described herein are derivatives of carbamic acid in which one or both of the amine hydrogens is optionally replaced by a hydrocarbyl, substituted hydrocarbyl or heterocyclo moiety.

The terms "hydroxyl protecting group" and "hydroxy protecting group" as used herein denote a group capable of protecting a free hydroxyl group ("protected hydroxyl") which, subsequent to the reaction for which protection is employed, may be removed without disturbing the remainder of the molecule. A variety of protecting groups for the hydroxyl group and the synthesis thereof may be found in "Protective Groups in Organic Synthesis" by T. W. Greene, John Wiley and Sons, 1981, or Fieser & Fieser. Exemplary hydroxyl protecting groups include methoxymethyl, 1-ethoxyethyl, benzyloxymethyl, (.beta.-trimethylsilylethoxy)methyl, tetrahydropyranyl, 2,2,2-trichloroethoxycarbonyl, t-butyl(diphenyl)silyl, trialkylsilyl, trichloromethoxycarbonyl and 2,2,2-trichloroethoxymethyl.

As used herein, "Ac" means acetyl; "Bz" means benzoyl; "Et" means ethyl; "Me" means methyl; "Ph" means phenyl; "iPr" means isopropyl; "tBu" and "t-Bu" means tert-butyl; "R" means lower alkyl unless otherwise defined; "py" means pyridine or pyridyl; "TES" means triethylsilyl; "TMS" means trimethylsilyl; "LAH" means lithium aluminum hydride; "10-DAB" means 10-desacetylbaccatin III"; "amine protecting group" includes, but is not limited to, carbamates, for example, 2,2,2-trichloroethylcarbamate or tertbutylcarbamate; "protected hydroxy" means -OP wherein P is a hydroxy protecting group; "tBuOCO" and "BOC" mean tert-butoxycarbonyl; "tAmOCO" means tert-amyloxycarbonyl; "PhCO" means phenylcarbonyl; "2-FuCO" means 2-furylcarbonyl; "2-ThCO" means 2-thienylcarbonyl; "2-PyCO" means 2-pyridylcarbonyl; "3-PyCO" means 3-pyridylcarbonyl; "4-PyCO" means 4-pyridylcarbonyl; "C₄H₇CO" means butenylcarbonyl; "tC₃H₅CO" means trans-propenylcarbonyl; "EtOCO" means ethoxycarbonyl; "ibueCO" means isobutenylcarbonyl; "iBuCO" means isobutylcarbonyl; "iBuOCO" means isobutoxycarbonyl; "iPrOCO" means isopropyloxycarbonyl; "nPrOCO" means n-propyloxycarbonyl; "nPrCO" means n-propylcarbonyl; "ibue" means isobutenyl; "THF" means tetrahydrofuran; "DMAP" means 4-dimethylamino pyridine; "LHMDS" means Lithium HexamethylDiSilazanide.

The following examples illustrate the invention.

### Example 1

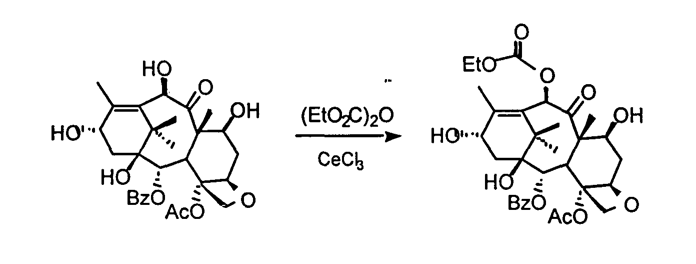

**10-Ethoxycarbonyl-10-deacetyl baccatin III.** To a mixture of 0.941 g (1.73 mmol) of 10-deacetyl baccatin III and 0.043g (0.17 mmol) of CeCl₃ in 40 mL of THF at 25 °C was added 0.64 mL (4.32 mmol) of diethyl pyrocarbonate. After 3 h the reaction mixture was diluted with 200 mL of EtOAc, then washed three times with 50 mL of saturated aqueous NaHCO₃ solution and brine. The organic extract was dried over Na₂SO₄ and concentrated *in vacuo.* The crude solid was purified by flash column chromatography on silica gel using 40% EtOAc/hexane as eluent to give 0.960 g (90%) of 10-ethoxycarbonyl-10-deacetyl baccatin III as a solid. 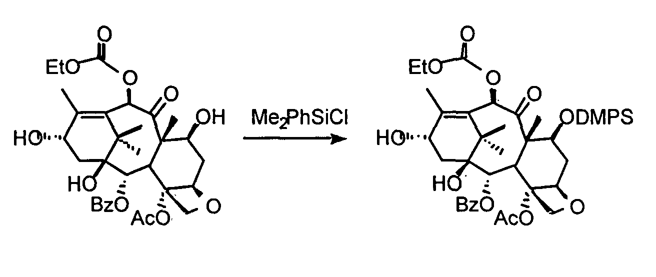 **7-Dimethylphenylsilyl-10-ethoxycarbonyl-10-deacetyl baccatin III.** To a solution of 1.02 g (1.65 mmol) of 10-ethoxycarbonyl-10-deacetyl baccatin III in 30 mL of THF at -10 °C under a nitrogen atmosphere was added dropwise 0.668 mL (4.00 mmol) of chlorodimethylphenylsilane and 2.48 mL (30.64 mmol) of pyridine. After 90 min the mixture was diluted with 200 mL of a 1:1 mixture of ethyl acetate and hexane. The mixture was washed with 30 mL of saturated aqueous sodium bicarbonate solution and the organic layer separated. The aqueous layer was extracted with 50 mL of a 1:1 mixture of ethyl acetate and hexane, and the combined organic extracts were washed with brine, dried over Na₂SO₄, and concentrated *in vacuo.* The crude solid was purified by flash column chromatography on silica gel using 30% EtOAc/hexane as eluent to give 1.16 g (94%) of 7-dimethylphenylsilyl-10-ethoxycarbonyl-10-deacetyl baccatin III as a solid. ₁HNMR (400 MHz, CDCl₃): δ 8.09 (dm, J= 7.64 Hz, 2 H, benzoate, o), 7.59 (tt, J= 7.54, 1.43 Hz, 1 H, benzoate, p), 7.57 (m, 2 H, phenyl, o), 7.46 (t, J= 7.54 Hz, 2 H, benzoate, m), 7.37-7.33 (m, 3 H, phenyl, m,p), 6.21 (s, 1 H, H10), 5.63 (d, J= 7.05 Hz, 1 H, H2), 4.87-4.80 (m, 2 H, H5 and H13), 4.44 (dd, J= 6.84, 10.37 Hz, 1 H, H7), 4.27 (d, J= 8.27 Hz, 1 H, H20α), 4.16 (qm, J= 7.00 Hz, 2 H, CH₃-CH₂-), 4.13 (d, J= 8.27 Hz, 1 H, H20β), 3.83 (d, J= 7.05 Hz, 1 H, H3), 2.34 (ddd, J= 6.84, 9.63, 14.66 Hz, 1 H, H6α), 2.26 (d, J= 7.65 Hz, 2 H, H14α,β), 2.25 (s, 3 H, Ac4), 2.03 (s, 3 H, Me18), 1.98 (d, J= 5.29, 1 H, C13OH 1.77 (ddd, J= 2.12, 10.37, 14.66 Hz, 1 H, H6β), 1.73 (s, 1 H, Me19), 1.59 (s, 1 H, C1OH), 1.32 (t, J= 7.00 Hz, 3 H, CH₃-CH₂-), 1.19 (s, 3 H, Me17), 1.07 (s, 3 H, Me16), 0.45 (s, 3 H, PhMe₂Si-), 0.35 (s, 3 H, PhMe₂Si-). 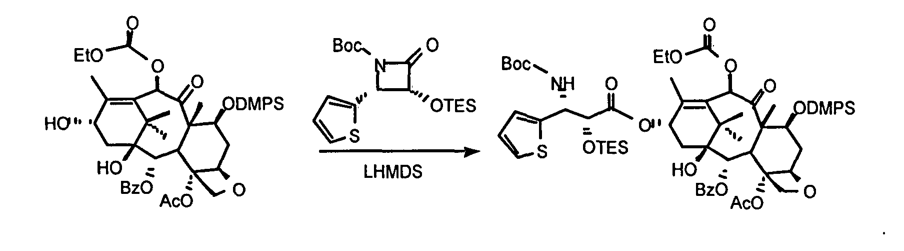 **7-Dimethylphenylsilyl-2'-O-triethylsilyl-3'-desphenyl-3'-(2-thienyl)-10-ethoxycarbonyl-10-deacetyl taxotere.** To a solution of 0.409 g (0.544 mmol) of 7-dimethylphenylsilyl-10-ethoxycarbonyl-10-deacetyl baccatin III in 5.5 mL of THF at -45 °C under a nitrogen atmosphere was added 0.681 mL (0.681 mmol) of a 1M solution of LHMDS in THF. After 1 h, a solution of 0.317 g (0.818 mmol) of *cis*-N-benzoyl-3-triethylsilyloxy-4-(2-thienyl) azetidin-2-one in 3 mL of THF was added slowly. The mixture was warmed to 0 °C and after 3 h 10 mL of saturated aqueous sodium bicarbonate solution was added and the mixture was extracted three times with 50 mL of ethyl acetate. The combined organic extracts were washed with brine, dried over Na₂SO₄, and concentrated *in vacuo.* The crude product was purified by flash column chromatography on silica gel using 40% EtOAc/hexane as eluent to give 0.574 g (93%) of 7-dimethylphenylsilyl-2'-O-triethylsilyl-3'-desphenyl-3'-(2-thienyl)-10-ethoxycarbonyl-10-deacetyl taxotere as a solid. 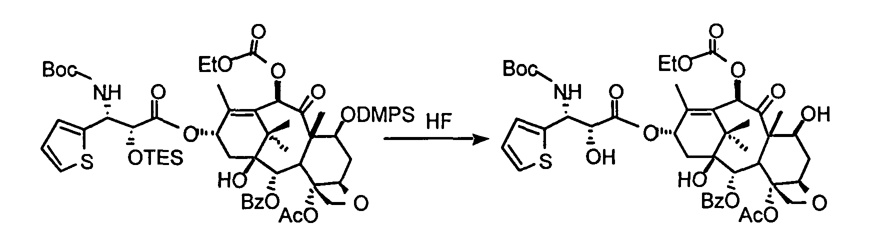 **3'-Desphenyl-3'-(2-thienyl)-10-ethoxycarbonyl-10-deacetyl taxotere.** To a solution of 0.527 g (0.464 mmol) of 7-dimethylphenylsilyl-2'-O-triethylsilyl-3'-desphenyl-3'-(2-thienyl)-10-ethoxycarbonyl-10-deacetyl taxotere in 2 mL of CH₃CN and 2 mL of pyridine at 0 °C was added 0.5 mL of a solution of 30% HF in H₂O. After 3 h 20 mL of a saturated aqueous sodium bicarbonate solution was added and the mixture was extracted three times with 50 mL of ethyl acetate. The combined organic extracts were washed with brine, dried over Na₂SO₄, and concentrated *in vacuo.* The crude product was purified by flash column chromatography on silica gel using 70% EtOAc/hexane as eluent to give 0.411 g (100%) of 3'-desphenyl-3'-(2-thienyl)-10-ethoxycarbonyl-10-deacetyl taxotere as a solid. m.p. 160-161 °C; [α]_{D}²⁵ = -59.1 (c 1.0 in CH₂Cl₂); Anal. Calcd. for C₄₄H₅₅NO₁₆S: C, 59.65; H, 6.26; Found: C, 59.39; H, 6.34.

### 3'-Desphenyl-3'-(2-thienyl)-10-ethoxycarbonyl-10-deacetyl taxotere ¹H NMR data (500 MHz, CDCl₃)

| **Proton** | **δ (ppm)** | **Pattern** | **J (Hz)** |
|---|---|---|---|
| 1OH | 1.68 | s | |
| 2 | 5.68 | d | H3(7.0) |
| 3 | 3.80 | d | H3(7.0) |
| 4Ac | 2.38 | s | |
| 5 | 4.95 | dd | H6β(2.0), H6β(9.8) |
| 6α | 2.56 | ddd | H7(6.6), H5(9.8), H6β(14.65) |
| 6β | 1.89 | ddd | H5(2.0), H7(10.9), H6α(14.65) |
| 7 | 4.40 | ddd | C7OH(4.2), H6α(6.6), H6β(10.9) |
| 7OH | 2.50 | d | H7(4.2) |
| 10 | 6.12 | s | |
| 13 | 6.25 | t | H14α(9.1), H14β(9.1) |
| 14α | 2.35 | dd | H13(9.1), H14β(14.2) |
| 14β | 2.34 | dd | H13(9.1), H14α(14.2) |
| 16Me | 1.17 | s | |
| 17Me | 1.26 | s | |
| 18Me | 1.90 | s | |
| 19Me | 1.70 | s | |
| 20α | 4.31 | d | H20β(8.6) |
| 20β | 4.19 | d | H20α(8.6) |
| 2' | 4.64 | dd | C2'OH(5.5), H3'(2.0) |
| 2'OH | 3.38 | d | H3'(5.5) |
| 3' | 5.51 | br d | NH(9.5) |
| NH | 5.28 | d | H3'(9.5) |
| 3'(2-thienyl), H3" | 7.29 | dd | 3'(2-thienyl), H5"(1.1), 3'(2-thienyl), H3"(5.1) |
| 3'(2-thienyl), H4" | 7.02 | dd | 3'(2-thienyl), H5"(3.6), 3'(2-thienyl), H3"(5.1) |
| 3'(2-thienyl), H5" | 7.09 | d | 3'(2-thienyl), H4"(3.6) |
| Boc | 1.34 | s | |
| benzoate, m | 7.51 | t | benzoate, o(7.8), benzoate, p(7.8) |
| benzoate, o | 8.12 | D | Benzoate, m(7.8) |
| benzoate, p | 7.61 | T | Benzoate, m(7.8) |
| CH3-CH2-OCO | 1.37 | T | CH3-CH2-OCO(7.1) |
| CH3-CH2-OCO | 4.28 | M | |

### Example 2

The procedures described in Example 1 were repeated, but other suitably protected β-lactams were substituted for the β-lactam of Example 1 to prepare the series of compounds having structural formula (13) and the combinations of substituents identified in the following table.

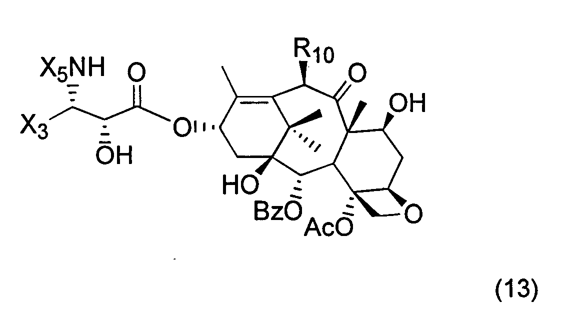

| **Compound** | **X**_{**5**} | **X**_{**3**} | **R**_{**10**} |
|---|---|---|---|
| 1755 | tBuOCO- | 2-thienyl | EtOCOO- |
| 1767 | tBuOCO- | Isopropyl | EtOCOO- |
| 1781 | tBuOCO- | Isobutenyl | EtOCOO- |
| 1799 | tBuOCO- | 2-pyridyl | EtOCOO- |
| 1808 | tBuOCO- | 3-pyridyl | EtOCOO- |
| 1811 | tBuOCO- | 4-pyridyl | EtOCOO- |
| 1822 | tBuOCO- | 2-furyl | EtOCOO- |
| 1838 | tBuOCO- | 3-furyl | EtOCOO- |
| 1841 | tBuOCO- | 3-thienyl | EtOCOO- |
| 1855 | tBuOCO- | Cyclobutyl | EtOCOO- |
| 1999 | tBuOCO- | Isobutenyl | MeOCOO- |
| 2002 | tBuOCO- | 2-pyridyl | MeOCOO- |
| 2011 | tBuOCO- | 3-pyridyl | MeOCOO- |
| 2020 | tBuOCO- | 4-pyridyl | MeOCOO- |
| 2032 | tBuOCO- | 3-furyl | MeOCOO- |
| 2044 | tBuOCO- | 2-thienyl | MeOCOO- |
| 2050 | tBuOCO- | 3-thienyl | MeOCOO- |
| 2062 | tBuOCO- | Isopropyl | MeOCOO- |
| 2077 | tBuOCO- | Cyclobutyl | MeOCOO- |
| 2666 | tBuOCO- | 2-furyl | MeOCOO- |
| 2972 | PhCO- | 2-thienyl | EtOCOO- |
| 2988 | EtOCO- | 2-thienyl | EtOCOO- |
| 2999 | iPrOCO- | 2-thienyl | EtOCOO- |
| 3003 | iBuOCO- | 2-thienyl | EtOCOO- |
| 3011 | 2-FuCO- | 2-thienyl | EtOCOO- |
| 3020 | 2-ThCO- | 2-thienyl | EtOCOO- |
| 3033 | C₄H₇CO- | 2-thienyl | EtOCOO- |
| 3155 | nPrCO- | 2-thienyl | EtOCOO- |
| 3181 | iBuOCO- | 2-furyl | EtOCOO- |
| 3243 | tC₃H₅CO- | 2-thienyl | EtOCOO- |
| 3300 | 3-PyCO- | 2-thienyl | EtOCOO- |
| 3393 | 4-PyCO- | 2-thienyl | EtOCOO- |
| 3433 | 2-PyCO- | 2-thienyl | EtOCOO- |
| 3911 | 2-FuCO- | 2-furyl | EtOCOO- |
| 3929 | nPrCO- | 2-furyl | EtOCOO- |
| 3963 | iPrOCO- | 2-furyl | EtOCOO- |
| 4000 | tC₃H₅CO- | 2-furyl | EtOCOO- |
| 4020 | EtOCO- | 2-furyl | EtOCOO- |
| 4074 | C₄H₇CO- | 2-furyl | EtOCOO- |
| 4088 | 2-ThCO- | 2-furyl | EtOCOO- |
| 4090 | PhCO- | 2-furyl | EtOCOO- |
| 4374 | ibueCO- | 2-thienyl | EtOCOO- |
| 4636 | iBuOCO- | 3-furyl | EtOCOO- |
| 6466 | iPrCO- | 2-furyl | EtOCOO- |
| 4959 | tC₃H₅CO- | 3-furyl | EtOCOO- |
| 4924 | iBuOCO- | 3-thienyl | EtOCOO- |
| 4844 | iBuOCO- | Cpro | EtOCOO- |
| 5171 | tBuOCO- | Cpro | EtOCOO- |
| 5155 | iBuOCO- | Isobutenyl | EtOCOO- |
| 1788 | tBuOCO- | Isobutenyl | EtOCOO- |
| 1767 | tBuOCO- | Isopropyl | EtOCOO- |
| 1771 | tBuOCO- | Phenyl | EtOCOO- |
| 1866 | tBuOCO- | p-nitrophenyl | EtOCOO- |
| 2060 | tBuOCO- | Isopropyl | MeOCOO- |
| 2092 | tBuOCO- | Phenyl | MeOCOO- |
| 2088 | tBuOCO- | p-nitrophenyl | MeOCOO- |

### Example 3

Following the processes described in Example 1 and elsewhere herein, the following specific taxanes having structural formula 14 may be prepared, wherein R₁₀ is as previously defined including wherein R₁₀ is R₁₀ₐOCOO- and R₁₀ₐ is (i) substituted or unsubstituted C₁ to C₈ alkyl such as methyl, ethyl, or straight, branched or cyclic propyl, butyl, pentyl, or hexyl; (ii) substituted or unsubstituted C₃ to C₈ alkenyl such as propenyl or straight, branched or cyclic butenyl, pentenyl or hexenyl; (iii) substituted or unsubstituted C₃ to C₈ alkynyl such as propynyl or straight or branched butynyl, pentynyl, or hexynyl; (iv) substituted or unsubstituted phenyl, or (v) substituted or unsubstituted heteroaromatic such as pyridyl. The substituents may be those identified elsewhere herein for substituted hydrocarbyl. For example, R₁₀ may be R₁₀ₐOCOO- wherein R₁₀ₐ is methyl, ethyl, or straight, branched or cyclic propyl.

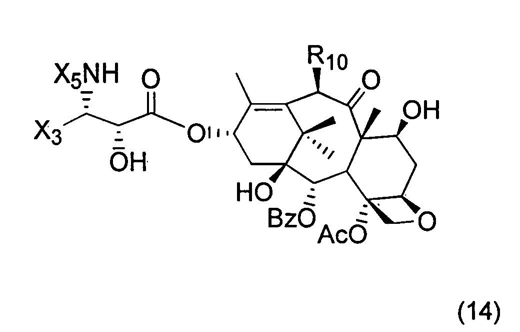

| **X**_{**5**} | **X**_{**3**} | **R**_{**10**} |
|---|---|---|
| tBuOCO | 2-furyl | RₐOCOO- |
| tBuOCO | 3-furyl | RₐOCOO- |
| tBuOCO | 2-thienyl | RₐOCOO- |
| tBuOCO | 3-thienyl | RₐOCOO- |
| tBuOCO | 2-pyridyl | RₐOCOO- |
| tBuOCO | 3-pyridyl | RₐOCOO- |
| tBuOCO | 4-pyridyl | RₐOCOO- |
| tBuOCO | Isobutenyl | RₐOCOO- |
| tBuOCO | Isopropyl | RₐOCOO- |
| tBuOCO | cyclopropyl | RₐOCOO- |
| tBuOCO | cyclobutyl | RₐOCOO- |
| tBuOCO | cyclopentyl | RₐOCOO- |
| tBuOCO | phenyl | RₐOCOO- |
| Benzoyl | 2-furyl | RₐOCOO- |
| Benzoyl | 3-furyl | RₐOCOO- |
| Benzoyl | 2-thienyl | RₐOCOO- |
| Benzoyl | 3-thienyl | RₐOCOO- |
| Benzoyl | 2-pyridyl | RₐOCOO- |
| Benzoyl | 3-pyridyl | RₐOCOO- |
| Benzoyl | 4-pyridyl | RₐOCOO- |
| Benzoyl | isobutenyl | RₐOCOO- |
| Benzoyl | isopropyl | RₐOCOO- |
| Benzoyl | cyclopropyl | RₐOCOO- |
| Benzoyl | cyclobutyl | RₐOCOO- |
| Benzoyl | cyclopentyl | RₐOCOO- |
| Benzoyl | phenyl | RₐOCOO- |
| 2-FuCO- | 2-furyl | RₐOCOO- |
| 2-FuCO- | 3-furyl | RₐOCOO- |
| 2-FuCO- | 2-thienyl | RₐOCOO- |
| 2-FuCO- | 3-thienyl | RₐOCOO- |
| 2-FuCO- | 2-pyridyl | RₐOCOO- |
| 2-FuCO- | 3-pyridyl | RₐOCOO- |
| 2-FuCO- | 4-pyridyl | RₐOCOO- |
| 2-FuCO- | isobutenyl | RₐOCOO- |
| 2-FuCO- | isopropyl | RₐOCOO- |
| 2-FuCO- | cyclopropyl | RₐOCOO- |
| 2-FuCO- | cyclobutyl | RₐOCOO- |
| 2-FuCO- | cyclopentyl | RₐOCOO- |
| 2-FuCO- | phenyl | RₐOCOO- |
| 2-ThCO- | 2-furyl | RₐOCOO- |
| 2-ThCO- | 3-furyl | RₐOCOO- |
| 2-ThCO- | 2-thienyl | RₐOCOO- |
| 2-ThCO- | 3-thienyl | RₐOCOO- |
| 2-ThCO- | 2-pyridyl | RₐOCOO- |
| 2-ThCO- | 3-pyridyl | RₐOCOO- |
| 2-ThCO- | 4-pyridyl | RₐOCOO- |
| 2-ThCO- | isobutenyl | RₐOCOO- |
| 2-ThCO- | isopropyl | RₐOCOO- |
| 2-ThCO- | cyclopropyl | RₐOCOO- |
| 2-ThCO- | cyclobutyl | RₐOCOO- |
| 2-ThCO- | cyclopentyl | RₐOCOO- |
| 2-ThCO- | phenyl | RₐOCOO- |
| 2-PyCO- | 2-furyl | RₐOCOO- |
| 2-PyCO- | 3-furyl | RₐOCOO- |
| 2-PyCO- | 2-thienyl | RₐOCOO- |
| 2-PyCO- | 3-thienyl | RₐOCOO- |
| 2-PyCO- | 2-pyridyl | RₐOCOO- |
| 2-PyCO- | 3-pyridyl | RₐOCOO- |
| 2-PyCO- | 4-pyridyl | RₐOCOO- |
| 2-PyCO- | isobutenyl | RₐOCOO- |
| 2-PyCO- | isopropyl | RₐOCOO- |
| 2-PyCO- | cyclopropyl | RₐOCOO- |
| 2-PyCO- | cyclobutyl | RₐOCOO- |
| 2-PyCO- | cyclopentyl | RₐOCOO- |
| 2-PyCO- | phenyl | RₐOCOO- |
| 3PyCO- | 2-furyl | RₐOCOO- |
| 3-PyCO- | 3-furyl | RₐOCOO- |
| 3-PyCO- | 2-thienyl | RₐOCOO- |
| 3-PyCO- | 3-thienyl | RₐOCOO- |
| 3-PyCO- | 2-pyridyl | RₐOCOO- |
| 3-PyCO- | 3-pyridyl | RₐOCOO- |
| 3-PyCO- | 4-pyridyl | RₐOCOO- |
| 3-PyCO- | isobutenyl | RₐOCOO- |
| 3-PyCO- | isopropyl | RₐOCOO- |
| 3-PyCO- | cyclopropyl | RₐOCOO- |
| 3-PyCO- | cyclobutyl | RₐOCOO- |
| 3-PyCO- | cyclopentyl | RₐOCOO- |
| 3-PyCO- | phenyl | RₐOCOO- |
| 4-PyCO- | 2-furyl | RₐOCOO- |
| 4-PyCO- | 3-furyl | RₐOCOO- |
| 4-PyCO- | 2-thienyl | RₐOCOO- |
| 4-PyCO- | 3-thienyl | RₐOCOO- |
| 4-PyCO- | 2-pyridyl | RₐOCOO- |
| 4-PyCO- | 3-pyridyl | RₐOCOO- |
| 4-PyCO- | 4-pyridyl | RₐOCOO- |
| 4-PyCO- | isobutenyl | RₐOCOO- |
| 4-PyCO- | isopropyl | RₐOCOO- |
| 4-PyCO- | cyclopropyl | RₐOCOO- |
| 4-PyCO- | cyclobutyl | RₐOCOO- |
| 4-PyCO- | cyclopentyl | RₐOCOO- |
| 4-PyCO- | phenyl | RₐOCOO- |
| C₄H₇CO- | 2-furyl | RₐOCOO- |
| C₄H₇CO- | 3-furyl | RₐOCOO- |
| C₄H₇CO- | 2-thienyl | RₐOCOO- |
| C₄H₇CO- | 3-thienyl | RₐOCOO- |
| C₄H₇CO- | 2-pyridyl | RₐOCOO- |
| C₄H₇CO- | 3-pyridyl | RₐOCOO- |
| C₄H₇CO- | 4-pyridyl | RₐOCOO- |
| C₄H₇CO- | isobutenyl | RₐOCOO- |
| C₄H₇CO- | isopropyl | RₐOCOO- |
| C₄H₇CO- | cyclopropyl | RₐOCOO- |
| C₄H₇CO- | Cyclobutyl | RₐOCOO- |
| C₄H₇CO- | Cyclopentyl | RₐOCOO- |
| C₄H₇CO- | Phenyl | RₐOCOO- |
| EtOCO- | 2-furyl | RₐOCOO- |
| EtOCO- | 3-furyl | RₐOCOO- |
| EtOCO- | 2-thienyl | RₐOCOO- |
| EtOCO- | 3-thienyl | RₐOCOO- |
| EtOCO- | 2-pyridyl | RₐOCOO- |
| EtOCO- | 3-pyridyl | RₐOCOO- |
| EtOCO- | 4-pyridyl | RₐOCOO- |
| EtOCO- | Isobutenyl | RₐOCOO- |
| EtOCO- | Isopropyl | RₐOCOO- |
| EtOCO- | Cyclopropyl | RₐOCOO- |
| EtOCO- | Cyclobutyl | RₐOCOO- |
| EtOCO- | Cyclopentyl | RₐOCOO- |
| EtOCO- | Phenyl | RₐOCOO- |
| ibueCO- | 2-furyl | RₐOCOO- |
| ibueCO- | 3-furyl | RₐOCOO- |
| ibueCO- | 2-thienyl | RₐOCOO- |
| ibueCO- | 3-thienyl | RₐOCOO- |
| ibueCO- | 2-pyridyl | RₐOCOO- |
| ibueCO- | 3-pyridyl | RₐOCOO- |
| ibueCO- | 4-pyridyl | RₐOCOO- |
| ibueCO- | Isobutenyl | RₐOCOO- |
| ibueCO- | Isopropyl | RₐOCOO- |
| ibueCO- | Cyclopropyl | RₐOCOO- |
| ibueCO- | Cyclobutyl | RₐOCOO- |
| ibueCO- | Cyclopentyl | RₐOCOO- |
| ibueCO- | Phenyl | RₐOCOO- |
| iBuCO- | 2-furyl | RₐOCOO- |
| iBuCO- | 3-furyl | RₐOCOO- |
| iBuCO- | 2-thienyl | RₐOCOO- |
| iBuCO- | 3-thienyl | RₐOCOO- |
| iBuCO- | 2-pyridyl | RₐOCOO- |
| iBuCO- | 3-pyridyl | RₐOCOO- |
| iBuCO- | 4-pyridyl | RₐOCOO- |
| iBuCO- | Isobutenyl | RₐOCOO- |
| iBuCO- | Isopropyl | RₐOCOO- |
| iBuCO- | Cyclopropyl | RₐOCOO- |
| iBuCO- | Cyclobutyl | RₐOCOO- |
| iBuCO- | Cyclopentyl | RₐOCOO- |
| iBuCO- | Phenyl | RₐOCOO- |
| iBuOCO- | 2-furyl | RₐOCOO- |
| iBuOCO- | 3-furyl | RₐOCOO- |
| iBuOCO- | 2-thienyl | RₐOCOO- |
| iBuOCO- | 3-thienyl | RₐOCOO- |
| iBuOCO- | 2-pyridyl | RₐOCOO- |
| iBuOCO- | 3-pyridyl | RₐOCOO- |
| iBuOCO- | 4-pyridyl | RₐOCOO- |
| iBuOCO- | Isobutenyl | RₐOCOO- |
| iBuOCO- | Isopropyl | RₐOCOO- |
| iBuOCO- | Cyclopropyl | RₐOCOO- |
| iBuOCO- | Cyclobutyl | RₐOCOO- |
| iBuOCO- | Cyclopentyl | RₐOCOO- |
| iBuOCO- | Phenyl | RₐOCOO- |
| iPrOCO- | 2-furyl | RₐOCOO- |
| iPrOCO- | 3-furyl | RₐOCOO- |
| iPrOCO- | 2-thienyl | RₐOCOO- |
| iPrOCO- | 3-thienyl | RₐOCOO- |
| iPrOCO- | 2-pyridyl | RₐOCOO- |
| iPrOCO- | 3-pyridyl | RₐOCOO- |
| iPrOCO- | 4-pyridyl | RₐOCOO- |
| iPrOCO- | Isobutenyl | RₐOCOO- |
| iPrOCO- | Isopropyl | RₐOCOO- |
| iPrOCO- | Cyclopropy | RₐOCOO- |
| iPrOCO- | Cyclobutyl | RₐOCOO- |
| iPrOCO- | Cyclopentyl | RₐOCOO- |
| iPrOCO- | Phenyl | RₐOCOO- |
| nPrOCO- | 2-furyl | RₐOCOO- |
| nPrOCO- | 3-furyl | RₐOCOO- |
| nPrOCO- | 2-thienyl | RₐOCOO- |
| nPrOCO- | 3-thienyl | RₐOCOO- |
| nPrOCO- | 2-pyridyl | RₐOCOO- |
| nPrOCO- | 3-pyridyl | RₐOCOO- |
| nPrOCO- | 4-pyridyl | RₐOCOO- |
| nPrQCO- | Isobutenyl | RₐOCOO- |
| nPrOCO- | Isopropyl | RₐOCOO- |
| nPrOCO- | Cyclopropyl | RₐOCOO- |
| nPrOCO- | Cyclobutyl | RₐOCOO- |
| nPrOCO- | Cyclopentyl | RₐOCOO- |
| nPrOCO- | Phenyl | RₐOCOO- |
| nPrCO- | 2-furyl | RₐOCOO- |
| nPrCO- | 3-furyl | RₐOCOO- |
| nPrCO- | 2-thienyl | RₐOCOO- |
| nPrCO- | 3-thienyl | RₐOCOO- |
| nPrCO- | 2-pyridyl | RₐOCOO- |
| nPrCO- | 3-pyridyl | RₐOCOO- |
| nPrCO- | 4-pyridyl | RₐOCOO- |
| nPrCO- | Isobutenyl | RₐOCOO- |
| nPrCO- | Isopropyl | RₐOCOO- |
| nPrCO- | Cyclopropyl | RₐOCOO- |
| nPrCO- | Cyclobutyl | RₐOCOO- |
| nPrCO- | Cyclopentyl | RₐOCOO- |
| nPrCO- | Phenyl | RₐOCOO- |
| tBuOCO | Cyclopentyl | EtOCOO- |
| Benzoyl | 3-furyl | EtOCOO- |
| Benzoyl | 3-thienyl | EtOCOO- |
| Benzoyl | 2-pyridyl | EtOCOO- |
| Benzoyl | 3-pyridyl | EtOCOO- |
| Benzoyl | 4-pyridyl | EtOCOO- |
| Benzoyl | Isobutenyl | EtOCOO- |
| Benzoyl | Isopropyl | EtOCOO- |
| Benzoyl | Cyclopropyl | EtOCOO- |
| Benzoyl | Cyclobutyl | EtOCOO- |
| Benzoyl | Cyclopentyl | EtOCOO- |
| Benzoyl | Phenyl | EtOCOO- |
| 2-FuCO- | 3-furyl | EtOCOO- |
| 2-FuCO- | 3-thienyl | EtOCOO- |
| 2-FuCO- | 2-pyridyl | EtOCOO- |
| 2-FuCO- | 3-pyridyl | EtOCOO- |
| 2-FuCO- | 4-pyridyl | EtOCOO- |
| 2-FuCO- | Isobutenyl | EtOCOO- |
| 2-FuCO- | Isopropyl | EtOCOO- |
| 2-FuCO- | Cyclopropyl | EtOCOO- |
| 2-FuCO- | Cyclobutyl | EtOCOO- |
| 2-FuCO- | Cyclopentyl | EtOCOO- |
| 2-FuCO- | Phenyl | EtOCOO- |
| 2-ThCO- | 3-furyl | EtOCOO- |
| 2-ThCO- | 3-thienyl | EtOCOO- |
| 2-ThCO- | 2-pyridyl | EtOCOO- |
| 2-ThCO- | 3-pyridyl | EtOCOO- |
| 2-ThCO- | 4-pyridyl | EtOCOO- |
| 2-ThCO- | Isobutenyl | EtOCOO- |
| 2-ThCO- | Isopropyl | EtOCOQ- |
| 2-ThCO- | Cyclopropyl | EtOCOO- |
| 2-ThCO- | Cyclobutyl | EtOCOO- |
| 2-ThCO- | Cyclopentyl | EtOCOO- |
| 2-ThCO- | Phenyl | EtOCOO- |
| 2-PyCO- | 2-furyl | EtOCOO- |
| 2-PyCO- | 3-furyl | EtOCOO- |
| 2-PyCO- | 3-thienyl | EtOCOO- |
| 2-PyCO- | 2-pyridyl | EtOCOO- |
| 2-PyCO- | 3-pyridyl | EtOCOO- |
| 2-PyCO- | 4-pyridyl | EtOCOO- |
| 2-PyCO- | Isobutenyl | EtOCOO- |
| 2-PyCO- | Isopropyl | EtOCOO- |
| 2-PyCO- | Cyclopropyl | EtOCOO- |
| 2-PyCO- | Cyclobutyl | EtOCOO- |
| 2-PyCO- | Cyclopentyl | EtOCOO- |
| 2-PyCO- | Phenyl | EtOCOO- |
| 3PyCO- | 2-furyl | EtOCOO- |
| 3-PyCO- | 3-furyl | EtOCOO- |
| 3-PyCO- | 3-thienyl | EtOCOO- |
| 3-PyCO- | 2-pyridyl | EtOCOO- |
| 3-PyCO- | 3-pyridyl | EtOCOO- |
| 3-PyCO- | 4-pyridyl | EtOCOO- |
| 3-PyCO- | Isobutenyl | EtOCOO- |
| 3-PyCO- | Isopropyl | EtOCOO- |
| 3-PyCO- | Cyclopropyl | EtOCOO- |
| 3-PyCO- | Cyclobutyl | EtOCOO- |
| 3-PyCO- | Cyclopentyl | EtOCOO- |
| 3-PyCO- | Phenyl | EtOCOO- |
| 4-PyCO- | 2-furyl | EtOCOO- |
| 4-PyCO- | 3-furyl | EtOCOO- |
| 4-PyCO- | 3-thienyl | EtOCOO- |
| 4-PyCO- | 2-pyridyl | EtOCOO- |
| 4-PyCO- | 3-pyridyl | EtOCOO- |
| 4-PyCO- | 4-pyridyl | EtOCOO- |
| 4-PyCO- | Isobutenyl | EtOCOO- |
| 4-PyCO- | Isopropyl | EtOCOO- |
| 4-PyCO- | Cyclopropyl | EtOCOO- |
| 4-PyCO- | Cyclobutyl | EtOCOO- |
| 4-PyCO- | Cyclopentyl | EtOCOO- |
| 4-PyCO- | Phenyl | EtOCOO- |
| C₄H₇CO- | 3-furyl | EtOCOO- |
| C₄H₇CO- | 3-thienyl | EtOCOO- |
| C₄H₇CO- | 2-pyridyl | EtOCOO- |
| C₄H₇CO- | 3-pyridyl | EtOCOO- |
| C₄H₇CO- | 4-pyridyl | EtOCOO- |
| C₄H₇CO- | Isobutenyl | EtOCOO- |
| C₄H₇CO- | Isopropyl | EtOCOO- |
| C₄H₇CO- | Cyclopropyl | EtOCOO- |
| C₄H₇CO- | Cyclobutyl | EtOCOO- |
| C₄H₇CO- | Cyclopentyl | EtOCOO- |
| C₄H₇CO- | Phenyl | EtOCOO- |
| EtOCO- | 3-furyl | EtOCOO- |
| EtOCO- | 3-thienyl | EtOCOO- |
| EtOCO- | 2-pyridyl | EtOCOO- |
| EtOCO- | 3-pyridyl | EtOCOO- |
| EtOCO- | 4-pyridyl | EtOCOO- |
| EtOCO- | Isobutenyl | EtOCOO- |
| EtOCO- | Isopropyl | EtOCOO- |
| EtOCO- | Cyclopropyl | EtOCOO- |
| EtOCO- | Cyclobutyl | EtOCOO- |
| EtOCO- | Cyclopentyl | EtOCOO- |
| EtOCO- | Phenyl | EtOCOO- |
| ibueCO- | 2-furyl | EtOCOO- |
| ibueCO- | 3-furyl | EtOCOO- |
| ibueCO- | 2-thienyl | EtOCOO- |
| ibueCO- | 3-thienyl | EtOCOO- |
| ibueCO- | 2-pyridyl | EtOCOO- |
| ibueCO- | 3-pyridyl | EtOCOO- |
| ibueCO- | 4-pyridyl | EtOCOO- |
| ibueCO- | Isobutenyl | EtOCOO- |
| ibueCO- | Isopropyl | EtOCOO- |
| ibueCO- | Cyclopropyl | EtOCOO- |
| ibueCO- | Cyclobutyl | EtOCOO- |
| ibueCO- | Cyclopentyl | EtOCOO- |
| ibueCO- | Phenyl | EtOCOO- |
| iBuCO- | 2-furyl | EtOCOO- |
| iBuCO- | 3-furyl | EtOCOO- |
| iBuCO- | 2-thienyl | EtOCOO- |
| iBuCO- | 3-thienyl | EtOCOO- |
| iBuCO- | 2-pyridyl | EtOCOO- |
| iBuCO- | 3-pyridyl | EtOCOO- |
| iBuCO- | 4-pyridyl | EtOCOO- |
| iBuCO- | Isobutenyl | EtOCOO- |
| iBuCO- | Isopropyl | EtOCOO- |
| iBuCO- | Cyclopropyl | EtOCOO- |
| iBuCO- | Cyclobutyl | EtOCOO- |
| iBuCO- | Cyclopentyl | EtOCOO- |
| iBuCO- | Phenyl | EtOCOO- |
| iBuOCO- | 2-pyridyl | EtOCOO- |
| iBuOCO- | 3-pyridyl | EtOCOO- |
| iBuOCO- | 4-pyridyl | EtOCOO- |
| iBuOCO- | Isopropyl | EtOCOO- |
| iBuOCO- | Cyclobutyl | EtOCOO- |
| iBuOCO- | Cyclopentyl | EtOCOO- |
| iBuOCO- | Phenyl | EtOCOO- |
| iPrOCO- | 3-furyl | EtOCOO- |
| iPrOCO- | 3-thienyl | EtOCOO- |
| iPrOCO- | 2-pyridyl | EtOCOO- |
| iPrOCO- | 3-pyridyl | EtOCOO- |
| iPrOCO- | 4-pyridyl | EtOCOO- |
| iPrOCO- | Isobutenyl | EtOCOO- |
| iPrOCO- | Isopropyl | EtOCOO- |
| iPrOCO- | Cyclopropyl | EtOCOO- |
| iPrOCO- | Cyclobutyl | EtOCOO- |
| iPrOCO- | Cyclopentyl | EtOCOO- |
| iPrOCO- | Phenyl | EtOCOO- |
| nPrOCO- | 2-furyl | EtOCOO- |
| nPrOCO- | 3-furyl | EtOCOO- |
| nPrOCO- | 2-thienyl | EtOCOO- |
| nPrOCO- | 3-thienyl | EtOCOO- |
| nPrOCO- | 2-pyridyl | EtOCOO- |
| nPrOCO- | 3-pyridyl | EtOCOO- |
| nPrOCO- | 4-pyridyl | EtOCOO- |
| nPrOCO- | Isobutenyl | EtOCOO- |
| nPrOCO- | Isopropyl | EtOCOO- |
| nPrOCO- | Cyclopropyl | EtOCOO- |
| nPrOCO- | Cyclobutyl | EtOCOO- |
| nPrOCO- | Cyclopentyl | EtQCOO- |
| nPrOCO- | Phenyl | EtOCOO- |
| nPrCO- | 3-furyl | EtOCOO- |
| nPrCO- | 3-thienyl | EtOCOO- |
| nPrCO- | 2-pyridyl | EtOCOO- |
| nPrCO- | 3-pyridyl | EtOCOO- |
| nPrCO- | 4-pyridyl | EtOCOO- |
| nPrCO- | Isobutenyl | EtOCOO- |
| nPrCO- | Isopropyl | EtOCOO- |
| nPrCO- | Cyclopropyl | EtOCOO- |
| nPrCO- | Cyclobutyl | EtOCOO- |
| nPrCO- | Cyclopentyl | EtOCOO- |
| nPrCO- | Phenyl | EtOCOO- |
| tBuOCO | Cyclopropyl | MeOCOO- |
| tBuOCO | Cyclopentyl | MeOCOO- |
| Benzoyl | 2-furyl | MeOCOO- |
| Benzoyl | 3-furyl | MeOCOO- |
| Benzoyl | 2-thienyl | MeOCOO- |
| Benzoyl | 3-thienyl | MeOCOO- |
| Benzoyl | 2-pyridyl | MeOCOO- |
| Benzoyl | 3-pyridyl | MeOCOO- |
| Benzoyl | 4-pyridyl | MeOCOO- |
| Benzoyl | Isobutenyl | MeOCOO- |
| Benzoyl | Isopropyl | MeOCOO- |
| Benzoyl | Cyclopropyl | MeOCOO- |
| Benzoyl | Cyclobutyl | MeOCOO- |
| Benzoyl | Cyclopentyl | MeOCOO- |
| Benzoyl | Phenyl | MeOCOO- |
| 2-FuCO- | 2-furyl | MeOCOO- |
| 2-FuCO- | 3-furyl | MeOCOO- |
| 2-FuCO- | 2-thienyl | MeOCOO- |
| 2-FuCO- | 3-thienyl | MeOCOO- |
| 2-FuCO- | 2-pyridyl | MeOCOO- |
| 2-FuCO- | 3-pyridyl | MeOCOO- |
| 2-FuCO- | 4-pyridyl | MeOCOO- |
| 2-FuCO- | Isobutenyl | MeOCOO- |
| 2-FuCO- | Isopropyl | MeOCOO- |
| 2-FuCO- | Cyclopropyl | MeOCOO- |
| 2-FuCO- | Cyclobutyl | MeOCOO- |
| 2-FuCO- | Cyclopentyl | MeOCOO- |
| 2-FuCO- | Phenyl | MeOCOO- |
| 2-ThCO- | 2-furyl | MeOCOO- |
| 2-ThCO- | 3-furyl | MeOCOO- |
| 2-ThCO- | 2-thienyl | MeOCOO- |
| 2-ThCO- | 3-thienyl | MeOCOO- |
| 2-ThCO- | 2-pyridyl | MeOCOO- |
| 2-ThCO- | 3-pyridyl | MeOCOO- |
| 2-ThCO- | 4-pyridyl | MeOCOO- |
| 2-ThCO- | Isobutenyl | MeOCOO- |
| 2-ThCO- | Isopropyl | MeOCOO- |
| 2-ThCO- | Cyclopropyl | MeOCOO- |
| 2-ThCO- | Cyclobutyl | MeOCOO- |
| 2-ThCO- | Cyclopentyl | MeOCOO- |
| 2-ThCO- | Phenyl | MeOCOO- |
| 2-PyCO- | 2-furyl | MeOCOO- |
| 2-PyCO- | 3-furyl | MeOCOO- |
| 2-PyCO- | 2-thienyl | MeOCOO- |
| 2-PyCO- | 3-thienyl | MeOCOO- |
| 2-PyCO- | 2-pyridyl | MeOCOO- |
| 2-PyCO- | 3-pyridyl | MeOCOO- |
| 2-PyCO- | 4-pyridyl | MeOCOO- |
| 2-PyCO- | Isobutenyl | MeOCOO- |
| 2-PyCO- | Isopropyl | MeOCOO- |
| 2-PyCO- | Cyclopropy | MeOCOO- |
| 2-PyCO- | Cyclobutyl | MeOCOO- |
| 2-PyCO- | Cyclopentyl | MeOCOO- |
| 2-PyCO- | Phenyl | MeOCOO- |
| 3PyCO- | 2-furyl | MeOCOO- |
| 3-PyCO- | 3-furyl | MeOCOO- |
| 3-PyCO- | 2-thienyl | MeOCOO- |
| 3-PyCO- | 3-thienyl | MeOCOO- |
| 3-PyCO- | 2-pyridyl | MeOCOO- |
| 3-PyCO- | 3-pyridyl | MeOCOO- |
| 3-PyCO- | 4-pyridyl | MeOCOO- |
| 3-PyCO- | Isobutenyl | MeOCOO- |
| 3-PyCO- | Isopropyl | MeOCOO- |
| 3-PyCO- | Cyclopropyl | MeOCOO- |
| 3-PyCO- | Cyclobutyl | MeOCOO- |
| 3-PyCO- | Cyclopentyl | MeOCOO- |
| 3-PyCO- | Phenyl | MeOCOO- |
| 4-PyCO- | 2-furyl | MeOCOO- |
| 4-PyCO- | 3-furyl | MeOCOO- |
| 4-PyCO- | 2-thienyl | MeOCOO- |
| 4-PyCO- | 3-thienyl | MeOCOO- |
| 4-PyCO- | 2-pyridyl | MeOCOO- |
| 4-PyCO- | 3-pyridyl | MeOCOO- |
| 4-PyCO- | 4-pyridyl | MeOCOO- |
| 4-PyCO- | Isobutenyl | MeOCOO- |
| 4-PyCO- | Isopropyl | MeOCOO- |
| 4-PyCO- | Cyclopropyl | MeOCOO- |
| 4-PyCO- | Cyclobutyl | MeOCOO- |
| 4-PyCO- | Cyclopentyl | MeOCOO- |
| 4-PyCO- | Phenyl | MeOCOO- |
| C₄H₇CO- | 2-furyl | MeOCOO- |
| C₄H₇CO- | 3-furyl | MeOCOO- |
| C₄H₇CO- | 2-thienyl | MeOCOO- |
| C₄H₇CO- | 3-thienyl | MeOCOO- |
| C₄H₇CO- | 2-pyridyl | MeOCOO- |
| C₄H₇CO- | 3-pyridyl | MeOCOO- |
| C₄H₇CO- | 4-pyridyl | MeOCOO- |
| C₄H₇CO- | Isobutenyl | MeOCOO- |
| C₄H₇CO- | Isopropyl | MeOCOO- |
| C₄H₇CO- | Cyclopropyl | MeOCOO- |
| C₄H₇CO- | Cyclobutyl | MeOCOO- |
| C₄H₇CO- | Cyclopentyl | MeOCOO- |
| C₄H₇CO- | Phenyl | MeOCOO- |
| EtOCO- | 2-furyl | MeOCOO- |
| EtOCO- | 3-furyl | MeOCOO- |
| EtOCO- | 2-thienyl | MeOCOO- |
| EtOCO- | 3-thienyl | MeOCOO- |
| EtOCO- | 2-pyridyl | MeOCOO- |
| EtOCO- | 3-pyridyl | MeOCOO- |
| EtOCO- | 4-pyridyl | MeOCOO- |
| EtOCO- | Isobutenyl | MeOCOO- |
| EtOCO- | Isopropyl | MeOCOO- |
| EtOCO- | Cyclopropyl | MeOCOO- |
| EtOCO- | Cyclobutyl | MeOCOO- |
| EtOCO- | Cyclopentyl | MeOCOO- |
| EtOCO- | Phenyl | MeOCOO- |
| ibueCO- | 2-furyl | MeOCOO- |
| ibueCO- | 3-furyl | MeOCOO- |
| ibueCO- | 2-thienyl | MeOCOO- |
| ibueCO- | 3-thienyl | MeOCOO- |
| ibueCO- | 2-pyridyl | MeOCOO- |
| ibueCO- | 3-pyridyl | MeOCOO- |
| ibueCO- | 4-pyridyl | MeOCOO- |
| ibueCO- | Isobutenyl | MeOCOO- |
| ibueCO- | Isopropyl | MeOCOO- |
| ibueCO- | Cyclopropyl | MeOCOO- |
| ibueCO- | Cyclobutyl | MeOCOO- |
| ibueCO- | Cyclopentyl | MeOCOO- |
| ibueCO- | Phenyl | MeOCOO- |
| iBuCO- | 2-furyl | MeOCOO- |
| iBuCO- | 3-furyl | MeOCOO- |
| iBuCO- | 2-thienyl | MeOCOO- |
| iBuCO- | 3-thienyl | MeOCOO- |
| iBuCO- | 2-pyridyl | MeOCOO- |
| iBuCO- | 3-pyridyl | MeOCOO- |
| iBuCO- | 4-pyridyl | MeOCOO- |
| iBuCO- | Isobutenyl | MeOCOO- |
| iBuCO- | Isopropyl | MeOCOO- |
| iBuCO- | Cyclopropyl | MeOCOO- |
| iBuCO- | Cyclobutyl | MeOCOO- |
| iBuCO- | Cyclopentyl | MeOCOO- |
| iBuCO- | Phenyl | MeOCOO- |
| iBuOCO- | 2-furyl | MeOCOO- |
| iBuOCO- | 3-furyl | MeOCOO- |
| iBuOCO- | 2-thienyl | MeOCOO- |
| iBuOCO- | 3-thienyl | MeOCOO- |
| iBuOCO- | 2-pyridyl | MeOCOO- |
| iBuOCO- | 3-pyridyl | MeOCOO- |
| iBuOCO- | 4-pyridyl | MeOCOO- |
| iBuOCO- | Isobutenyl | MeOCOO- |
| iBuOCO- | Isopropyl | MeOCOO- |
| iBuOCO- | Cyclopropyl | MeOCOO- |
| iBuOCO- | Cyclobutyl | MeOCOO- |
| iBuOCO- | Cyclopentyl | MeOCOO- |
| iBuOCO- | Phenyl | MeOCOO- |
| iPrOCO- | 2-furyl | MeOCOO- |
| iPrOCO- | 3-furyl | MeOCOO- |
| iPrOCO- | 2-thienyl | MeOCOO- |
| iPrOCO- | 3-thienyl | MeOCOO- |
| iPrOCO- | 2-pyridyl | MeOCOO- |
| iPrOCO- | 3-pyridyl | MeOCOO- |
| iPrOCO- | 4-pyridyl | MeOCOO- |
| iPrOCO- | Isobutenyl | MeOCOO- |
| iPrOCO- | Isopropyl | MeOCOO- |
| iPrOCO- | Cyclopropyl | MeOCOO- |
| iPrOCO- | Cyclobutyl | MeOCOO- |
| iPrOCO- | Cyclopentyl | MeOCOO- |
| iPrOCO- | Phenyl | MeOCOO- |
| nPrOCO- | 2-furyl | MeOCOO- |
| nPrOCO- | 3-furyl | MeOCOO- |
| nPrOCO- | 2-thienyl | MeOCOO- |
| nPrOCO- | 3-thienyl | MeOCOO- |
| nPrOCO- | 2-pyridyl | MeOCOO- |
| nPrOCO- | 3-pyridyl | MeOCOO- |
| nPrOCO- | 4-pyridyl | MeOCOO- |
| nPrOCO- | Isobutenyl | MeOCOO- |
| nPrOCO- | Isopropyl | MeOCOO- |
| nPrOCO- | Cyclopropyl | MeOCOO- |
| nPrOCO- | Cyclobutyl | MeOCOO- |
| nPrOCO- | Cyclopentyl | MeOCOO- |
| nPrOCO- | Phenyl | MeOCOO- |
| nPrCO- | 2-furyl | MeOCOO- |
| nPrCO- | 3-furyl | MeOCOO- |
| nPrCO- | 2-thienyl | MeOCOO- |
| nPrCO- | 3-thienyl | MeOCOO- |
| nPrCO- | 2-pyridyl | MeOCOO- |
| nPrCO- | 3-pyridyl | MeOCOO- |
| nPrCO- | 4-pyridyl | MeOCOO- |
| nPrCO- | Isobutenyl | MeOCOO- |
| nPrCO- | Isopropyl | MeOCOO- |
| nPrCO- | Cyclopropyl | MeOCOO- |
| nPrCO- | Cyclobutyl | MeOCOO- |
| nPrCO- | Cyclopentyl | MeOCOO- |
| nPrCO- | Phenyl | MeOCOO- |

### Example 4

Following the processes described in Example 1 and elsewhere herein, the following specific taxanes having structural formula 15 may be prepared, wherein in each of the series (that is, each of series "A" through "K") R₇ is hydroxy and R₁₀ is as previously defined, including wherein R₁₀ is R₁₀ₐOCOO- and R₁₀ₐ is (i) substituted or unsubstituted, preferably unsubstituted, C₂ to C₈ alkyl (straight, branched or cyclic), such as ethyl, propyl, butyl, pentyl, or hexyl; (ii) substituted or unsubstituted, preferably unsubstituted, C₂ to C₈ alkenyl (straight, branched or cyclic), such as ethenyl, propenyl, butenyl, pentenyl or hexenyl; (iii) substituted or unsubstituted, preferably unsubstituted, C₂ to C₈ alkynyl (straight or branched) such as ethynyl, propynyl, butynyl, pentynyl, or hexynyl; (iv) substituted or unsubstituted, preferably unsubstituted, phenyl; or (v) substituted or unsubstituted, preferably unsubstituted, heteroaromatic such as furyl, thienyl, or pyridyl.

In the "A" series of compounds, X₁₀ is as otherwise as defined herein. Preferably, heterocyclo is substituted or unsubstituted furyl, thienyl, or pyridyl, X₁₀ is substituted or unsubstituted furyl, thienyl, pyridyl, phenyl, or lower alkyl (e.g., tert-butyl), and R₇ and R₁₀ each have the beta stereochemical configuration.

In the "B" series of compounds, X₁₀ and R₂ₐ are as otherwise as defined herein. Preferably, heterocyclo is preferably substituted or unsubstituted furyl, thienyl, or pyridyl, X₁₀ is preferably substituted or unsubstituted furyl, thienyl, pyridyl, phenyl, or lower alkyl (e.g., tert-butyl), R₂ₐ is preferably substituted or unsubstituted furyl, thienyl, pyridyl, phenyl, or lower alkyl, and R₇ and R₁₀ each have the beta stereochemical configuration.

In the "C" series of compounds, X₁₀ and R₉ₐ are as otherwise as defined herein. Preferably, heterocyclo is preferably substituted or unsubstituted furyl, thienyl, or pyridyl, X₁₀ is preferably substituted or unsubstituted furyl, thienyl, pyridyl, phenyl, or lower alkyl (e.g., tert-butyl), R₉ₐ is preferably substituted or unsubstituted furyl, thienyl, pyridyl, phenyl, or lower alkyl, and R₇, R₉ and R₁₀ each have the beta stereochemical configuration.

In the "D" and "E" series of compounds, X₁₀ is as otherwise as defined herein. Preferably, heterocyclo is preferably substituted or unsubstituted furyl, thienyl, or pyridyl, X₁₀ is preferably substituted or unsubstituted furyl, thienyl, pyridyl, phenyl, or lower alkyl (e.g., tert-butyl), and R₇, R₉ (series D only) and R₁₀ each have the beta stereochemical configuration.

In the "F" series of compounds, X₁₀, R₂ₐ and R₉ₐ are as otherwise as defined herein. Preferably, heterocyclo is preferably substituted or unsubstituted furyl, thienyl, or pyridyl, X₁₀ is preferably substituted or unsubstituted furyl, thienyl, pyridyl, phenyl, or lower alkyl (e.g., tert-butyl), R₂ₐ is preferably substituted or unsubstituted furyl, thienyl, pyridyl, phenyl, or lower alkyl, and R₇, R₉ and R₁₀ each have the beta stereochemical configuration.

In the "G" series of compounds, X₁₀ and R₂ₐ are as otherwise as defined herein. Preferably, heterocyclo is preferably substituted or unsubstituted furyl, thienyl, or pyridyl, X₁₀ is preferably substituted or unsubstituted furyl, thienyl, pyridyl, phenyl, or lower alkyl (e.g., tert-butyl), R₂ₐ is preferably substituted or unsubstituted furyl, thienyl, pyridyl, phenyl, or lower alkyl, and R₇, R₉ and R₁₀ each have the beta stereochemical configuration.

In the "H" series of compounds, X₁₀ is as otherwise as defined herein. Preferably, heterocyclo is preferably substituted or unsubstituted furyl, thienyl, or pyridyl, X₁₀ is preferably substituted or unsubstituted furyl, thienyl, pyridyl, phenyl, or lower alkyl (e.g., tert-butyl), R₂ₐ is preferably substituted or unsubstituted furyl, thienyl, pyridyl, phenyl, or lower alkyl, and R₇ and R₁₀ each have the beta stereochemical configuration.

In the "I" series of compounds, X₁₀ and R₂ₐ are as otherwise as defined herein. Preferably, heterocyclo is preferably substituted or unsubstituted furyl, thienyl, or pyridyl, X₁₀ is preferably substituted or unsubstituted furyl, thienyl, pyridyl, phenyl, or lower alkyl (e.g., tert-butyl), R₂ₐ is preferably substituted or unsubstituted furyl, thienyl, pyridyl, phenyl, or lower alkyl, and R₇ and R₁₀ each have the beta stereochemical configuration.

In the "J" series of compounds, X₁₀ and R₂ₐ are as otherwise as defined herein. Preferably, heterocyclo is preferably substituted or unsubstituted furyl, thienyl, or pyridyl, X₁₀ is preferably substituted or unsubstituted furyl, thienyl, pyridyl, phenyl, or lower alkyl (e.g., tert-butyl), R₂ₐ is preferably substituted or unsubstituted furyl, thienyl, pyridyl, phenyl, or lower alkyl, and R₇, R₉ and R₁₀ each have the beta stereochemical configuration.

In the "K" series of compounds, X₁₀, R₂ₐ and R₉ₐ are as otherwise as defined herein. Preferably, heterocyclo is preferably substituted or unsubstituted furyl, thienyl, or pyridyl, X₁₀ is preferably substituted or unsubstituted furyl, thienyl, pyridyl, phenyl, or lower alkyl (e.g., tert-butyl), R₂ₐ is preferably substituted or unsubstituted furyl, thienyl, pyridyl, phenyl, or lower alkyl, and R₇, R₉ and R₁₀ each have the beta stereochemical configuration.

Any substituents of each of X₃, X₅, R₂, R₉ and R₁₀ may be hydrocarbyl or any of the heteroatom containing substituents selected from the group consisting of heterocyclo, alkoxy, alkenoxy, alkynoxy, aryloxy, hydroxy, protected hydroxy, keto, acyloxy, nitro, amino, amido, thiol, ketal, acetal, ester and ether moieties, but not phosphorous containing moieties.

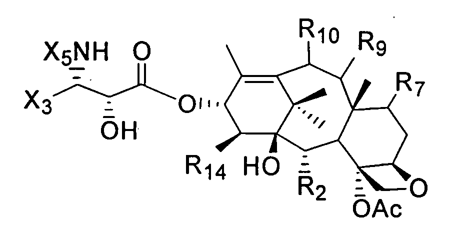

| **Series** | **X**_{**5**} | **X**_{**3**} | **R**_{**10**} | **R**_{**2**} | **R**_{**9**} | **R**_{**14**} |
|---|---|---|---|---|---|---|
| A1 | -COOX₁₀ | Heterocyclo | R₁₀ₐOCOO- | C₆H₅COO- | O | H |
| A2 | -COX₁₀ | Heterocyclo | R₁₀ₐOCOO- | C₆H₅COO- | O | H |
| A3 | -CONHX₁₀ | Heterocyclo | R₁₀ₐOCOO- | C₆H₅COO- | O | H |
| A4 | -COOX₁₀ | Optionally substituted C₂ to C₈ alkyl | R₁₀ₐOCOO- | C₆H₅COO- | O | H |
| A5 | -COX₁₀ | Optionally substituted C₂ to C₈ alkyl | R₁₀ₐOCOO- | C₆H₅COO- | O | H |
| A6 | -CONHX₁₀ | Optionally substituted C₂ to C₈ alkyl | R₁₀ₐOCOO- | C₆H₅COO- | O | H |
| A7 | -COOX₁₀ | Optionally substituted C₂ to C₈ alkenyl | R₁₀ₐOCOO- | C₆H₅COO- | O | H |
| A8 | -COX₁₀ | Optionally substituted C₂ to C₈ alkenyl | R₁₀ₐOCOO- | C₆H₅COO- | O | H |
| A9 | -CONHX₁₀ | Optionally substituted C₂ to C₈ alkenyl | R₁₀ₐOCOO- | C₆H₅COO- | O | H |
| A10 | -COOX₁₀ | Optionally substituted C₂ to C₈ alkynyl | R₁₀ₐOCOO- | C₆H₅COO- | O | H |
| A11 | -COX₁₀ | Optionally substituted C₂ to C₈ alkynyl | R₁₀ₐOCOO- | C₆H₅COO- | O | H |
| A12 | -CONHX₁₀ | Optionally substituted C₂ to C₈ alkynyl | R₁₀ₐOCOO- | C₆H₅COO- | O | H |
| B1 | -COOX₁₀ | Heterocyclo | R₁₀ₐOCOO- | R₂ₐCOO- | O | H |
| B2 | -COX₁₀ | Heterocyclo | R₁₀ₐOCOO- | R₂ₐCOO- | O | H |
| B3 | -CONHX₁₀ | Heterocyclo | R₁₀ₐOCOO- | R₂ₐCOO- | O | H |
| B4 | -COOX₁₀ | Optionally substituted C₂ to C₈ alkyl | R₁₀ₐOCOO- | R₂ₐCOO- | O | H |
| B5 | -COX₁₀ | Optionally substituted C₂ to C₈ alkyl | R₁₀ₐOCOO- | R₂ₐCOO- | O | H |
| B6 | -CONHX₁₀ | Optionally substituted C₂ to C₈ alkyl | R₁₀ₐOCOO- | R₂ₐCOO- | O | H |
| B7 | -COOX₁₀ | Optionally substituted C₂ to C₈ alkenyl | R₁₀ₐOCOO- | R₂ₐCOO- | O | H |
| B8 | -COX₁₀ | Optionally substituted C₂ to C₈ alkenyl | R₁₀ₐOCOO- | R₂ₐCOO- | O | H |
| B9 | -CONHX₁₀ | Optionally substituted C₂ to C₈ alkenyl | R₁₀ₐOCOO- | R₂ₐCOO- | O | H |
| B10 | -COOX₁₀ | Optionally substituted C₂ to C₈ alkynyl | R₁₀ₐOCOO- | R₂ₐCOO- | O | H |
| B11 | -COX₁₀ | Optionally substituted C₂ to C₈ alkynyl | R₁₀ₐOCOO- | R₂ₐCOO- | O | H |
| B12 | -CONHX₁₀ | Optionally substituted C₂ to C₈ alkynyl | R₁₀ₐOCOO- | R₂ₐCOO- | O | H |
| C1 | -COOX₁₀ | Heterocyclo | R₁₀ₐOCOO- | C₆H₅COO- | R₉ₐCOO- | H |
| C2 | -COX₁₀ | Heterocyclo | R₁₀ₐOCOO- | C₆H₅COO- | R₉ₐCOO- | H |
| C3 | -CONHX₁₀ | Heterocyclo | R₁₀ₐOCOO- | C₆H₅COO- | R₉ₐCOO- | H |
| C4 | -COOX₁₀ | Optionally substituted C₂ to C₈ alkyl | R₁₀ₐOCOO- | C₆H₅COO- | R₉ₐCOO- | H |
| C5 | -COX₁₀ | Optionally substituted C₂ to C₈ alkyl | R₁₀ₐOCOO- | C₆H₅COO- | R₉ₐCOO- | H |
| C6 | -CONHX₁₀ | Optionally substituted C₂ to C₈ alkyl | R₁₀ₐOCOO- | C₆H₅COO- | R₉ₐCOO- | H |
| C7 | -COOX₁₀ | Optionally substituted C₂ to C₈ alkenyl | R₁₀ₐOCOO- | C₆H₅COO- | R₉ₐCOO- | H |
| C8 | -COX₁₀ | Optionally substituted C₂ to C₈ alkenyl | R₁₀ₐOCOO- | C₆H₅COO- | R₉ₐCOO- | H |
| C9 | -CONHX₁₀ | Optionally substituted C₂ to C₈ alkenyl | R₁₀ₐOCOO- | C₆H₅COO- | R₉ₐCOO- | H |
| C10 | -COOX₁₀ | Optionally substituted C₂ to C₈ alkynyl | R₁₀ₐOCOO- | C₆H₅COO- | R₉ₐCOO- | H |
| C11 | -COX₁₀ | Optionally substituted C₂ to C₈ alkynyl | R₁₀ₐOCOO- | C₆H₅COO- | R₉ₐCOO- | H |
| C12 | -CONHX₁₀ | Optionally substituted C₂ to C₈ alkynyl | R₁₀ₐOCOO- | C₆H₅COO- | R₉ₐCOO- | H |
| D1 | -COOX₁₀ | Heterocyclo | R₁₀ₐOCOO- | C₆H₅COO- | OH | H |
| D2 | -COX₁₀ | Heterocyclo | R₁₀ₐOCOO- | C₆H₅COO- | OH | H |
| D3 | -CONHX₁₀ | Heterocyclo | R₁₀ₐOCOO- | C₆H₅COO- | OH | H |
| D4 | -COOX₁₀ | Optionally substituted C₂ to C₈ alkyl | R₁₀ₐOCOO- | C₆H₅COO- | OH | H |
| D5 | -COX₁₀ | Optionally substituted C₂ to C₈ alkyl | R₁₀ₐOCOO- | C₆H₅COO- | OH | H |
| D6 | -CONHX₁₀ | Optionally substituted C₂ to C₈ alkyl | R₁₀ₐOCOO- | C₆H₅COO- | OH | H |
| D7 | -COOX₁₀ | Optionally substituted C₂ to C₈ alkenyl | R₁₀ₐOCOO- | C₆H₅COO- | OH | H |
| D8 | -COX₁₀ | Optionally substituted C₂ to C₈ alkenyl | R₁₀ₐOCOO- | C₆H₅COO- | OH | H |
| D9 | -CONHX₁₀ | Optionally substituted C₂ to C₈ alkenyl | R₁₀ₐOCOO- | C₆H₅COO- | OH | H |
| D10 | -COOX₁₀ | Optionally substituted C₂ to C₈ alkynyl | R₁₀ₐOCOO- | C₆H₅COO- | OH | H |
| D11 | -COX₁₀ | Optionally substituted C₂ to C₈ alkynyl | R₁₀ₐOCOO- | C₆H₅COO- | OH | H |
| D12 | -CONHX₁₀ | Optionally substituted C₂ to C₈ alkynyl | R₁₀ₐOCOO- | C₆H₅COO- | OH | H |
| E1 | -COOX₁₀ | Heterocyclo | R₁₀ₐOCOO- | C₆H₅COO- | O | OH |
| E2 | -COX₁₀ | Heterocyclo | R₁₀ₐOCOO- | C₆H₅COO- | O | OH |
| E3 | -CONHX₁₀ | Heterocyclo | R₁₀ₐOCOO- | C₆H₅COO- | O | OH |
| E4 | -COOX₁₀ | Optionally substituted C₂ to C₈ alkyl | R₁₀ₐOCOO- | C₆H₅COO- | O | OH |
| E5 | -COX₁₀ | Optionally substituted C₂ to C₈ alkyl | R₁₀ₐOCOO- | C₆H₅COO- | O | OH |
| E6 | -CONHX₁₀ | Optionally substituted C₂ to C₈ alkyl | R₁₀ₐOCOO- | C₆H₅COO- | O | OH |
| E7 | -COOX₁₀ | Optionally substituted C₂ to C₈ alkenyl | R₁₀ₐOCOO- | C₆H₅COO- | O | OH |
| E8 | -COX₁₀ | Optionally substituted C₂ to C₈ alkenyl | R₁₀ₐOCOO- | C₆H₅COO- | O | OH |
| E9 | -CONHX₁₀ | Optionally substituted C₂ to C₈ alkenyl | R₁₀ₐOCOO- | C₆H₅COO- | O | OH |
| E10 | -COOX₁₀ | Optionally substituted C₂ to C₈ alkynyl | R₁₀ₐOCOO- | C₆H₅COO- | O | OH |
| E11 | -COX₁₀ | Optionally substituted C₂ to C₈ alkynyl | R₁₀ₐOCOO- | C₆H₅COO- | O | OH |
| E12 | -CONHX₁₀ | Optionally substituted C₂ to C₈ alkynyl | R₁₀ₐOCOO- | C₆H₅COO- | O | OH |
| F1 | -COOX₁₀ | Heterocyclo | R₁₀ₐOCOO- | R₂ₐCOO- | R₉ₐCOO- | H |
| F2 | -COX₁₀ | Heterocyclo | R₁₀ₐOCOO- | R₂ₐCOO- | R₉ₐCOO- | H |
| F3 | -CONHX₁₀ | Heterocyclo | R₁₀ₐOCOO- | R₂ₐCOO- | R₉ₐCOO- | H |
| F4 | -COOX₁₀ | Optionally substituted C₂ to C₈ alkyl | R₁₀ₐOCOO- | R₂ₐCOO- | R₉ₐCOO- | H |
| F5 | -COX₁₀ | Optionally substituted C₂ to C₈ alkyl | R₁₀ₐOCOO- | R₂ₐCOO- | R₉ₐCOO- | H |
| F6 | -CONHX₁₀ | Optionally substituted C₂ to C₈ alkyl | R₁₀ₐOCOO- | R₂ₐCOO- | R₉ₐCOO- | H |
| F7 | -COOX₁₀ | Optionally substituted C₂ to C₈ alkenyl | R₁₀ₐOCOO- | R₂ₐCOO- | R₉ₐCOO- | H |
| F8 | -COX₁₀ | Optionally substituted C₂ to C₈ alkenyl | R₁₀ₐOCOO- | R₂ₐCOO- | R₉ₐCOO- | H |
| F9 | -CONHX₁₀ | Optionally substituted C₂ to C₈ alkenyl | R₁₀ₐOCOO- | R₂ₐCOO- | R₉ₐCOO- | H |
| F10 | -COOX₁₀ | Optionally substituted C₂ to C₈ alkynyl | R₁₀ₐOCOO- | R₂ₐCOO- | R₉ₐCOO- | H |
| F11 | -COX₁₀ | Optionally substituted C₂ to C₈ alkynyl | R₁₀ₐOCOO- | R₂ₐCOO- | R₉ₐCOO- | H |
| F12 | -CONHX₁₀ | Optionally substituted C₂ to C₈ alkynyl | R₁₀ₐOCOO- | R₂ₐCOO- | R₉ₐCOO- | H |
| G1 | -COOX₁₀ | Heterocyclo | R₁₀ₐOCOO- | R₂ₐCOO- | OH | H |
| G2 | -COX₁₀ | Heterocyclo | R₁₀ₐOCOO- | R₂ₐCOO- | OH | H |
| G3 | -CONHX₁₀ | Heterocyclo | R₁₀ₐOCOO- | R₂ₐCOO- | OH | H |
| G4 | -COOX₁₀ | Optionally substituted C₂ to C₈ alkyl | R₁₀ₐOCOO- | R₂ₐCOO- | OH | H |
| G5 | -COX₁₀ | Optionally substituted C₂ to C₈ alkyl | R₁₀ₐOCOO- | R₂ₐCOO- | OH | H |
| G6 | -CONHX₁₀ | Optionally substituted C₂ to C₈ alkyl | R₁₀ₐOCOO- | R₂ₐCOO- | OH | H |
| G7 | -COOX₁₀ | Optionally substituted C₂ to C₈ alkenyl | R₁₀ₐOCOO- | R₂ₐCOO- | OH | H |
| G8 | -COX₁₀ | Optionally substituted C₂ to C₈ alkenyl | R₁₀ₐOCOO- | R₂ₐCOO- | OH | H |
| G9 | -CONHX₁₀ | Optionally substituted C₂ to C₈ alkenyl | R₁₀ₐOCOO- | R₂ₐCOO- | OH | H |
| G10 | -COOX₁₀ | Optionally substituted C₂ to C₈ alkynyl | R₁₀ₐOCOO- | R₂ₐCOO- | OH | H |
| G11 | -COX₁₀ | Optionally substituted C₂ to C₈ alkynyl | R₁₀ₐOCOO- | R₂ₐCOO- | OH | H |
| G12 | -CONHX₁₀ | Optionally substituted C₂ to C₈ alkynyl | R₁₀ₐOCOO- | R₂ₐCOO- | OH | H |
| H1 | -COOX₁₀ | Heterocyclo | R₁₀ₐOCOO- | C₆H₅COO- | OH | OH |
| H2 | -COX₁₀ | Heterocyclo | R₁₀ₐOCOO- | C₆H₅COO- | OH | OH |
| H3 | -CONHX₁₀ | Heterocyclo | R₁₀ₐOCOO- | C₆H₅COO- | OH | OH |
| H4 | -COOX₁₀ | Optionally substituted C₂ to C₈ alkyl | R₁₀ₐOCOO- | C₆H₅COO- | OH | OH |
| H5 | -COX₁₀ | Optionally substituted C₂ to C₈ alkyl | R₁₀ₐOCOO- | C₆H₅COO- | OH | OH |
| H6 | -CONHX₁₀ | Optionally substituted C₂ to C₈ alkyl | R₁₀ₐOCOO- | C₆H₅COO- | OH | OH |
| H7 | -COOX₁₀ | Optionally substituted C₂ to C₈ alkenyl | R₁₀ₐOCOO- | C₆H₅COO- | OH | OH |
| H8 | -COX₁₀ | Optionally substituted C₂ to C₈ alkenyl | R₁₀ₐOCOO- | C₆H₅COO- | OH | OH |
| H9 | -CONHX₁₀ | Optionally substituted C₂ to C₈ alkenyl | R₁₀ₐOCOO- | C₆H₅COO- | OH | OH |
| H10 | -COOX₁₀ | Optionally substituted C₂ to C₈ alkynyl | R₁₀ₐOCOO- | C₆H₅COO- | OH | OH |
| H11 | -COX₁₀ | Optionally substituted C₂ to C₈ alkynyl | R₁₀ₐOCOO- | C₆H₅COO- | OH | OH |
| H12 | -CONHX₁₀ | Optionally substituted C₂ to C₈ alkynyl | R₁₀ₐOCOO- | C₆H₅COO- | OH | OH |
| I1 | -COOX₁₀ | Heterocyclo | R₁₀ₐOCOO- | R₂ₐCOO- | O | OH |
| I2 | -COX₁₀ | Heterocyclo | R₁₀ₐOCOO- | R₂ₐCOO- | O | OH |
| I3 | -CONHX₁₀ | Heterocyclo | R₁₀ₐOCOO- | R₂ₐCOO- | O | OH |
| I4 | -COOX₁₀ | Optionally substituted C₂ to C₈ alkyl | R₁₀ₐOCOO- | R₂ₐCOO- | O | OH |
| I5 | -COX₁₀ | Optionally substituted C₂ to C₈ alkyl | R₁₀ₐOCOO- | R₂ₐCOO- | O | OH |
| I6 | -CONHX₁₀ | Optionally substituted C₂ to C₈ alkyl | R₁₀ₐOCOO- | R₂ₐCOO- | O | OH |
| I7 | -COOX₁₀ | Optionally substituted C₂ to C₈ alkenyl | R₁₀ₐOCOO- | R₂ₐCOO- | O | OH |
| I8 | -COX₁₀ | Optionally substituted C₂ to C₈ alkenyl | R₁₀ₐOCOO- | R₂ₐCOO- | O | OH |
| I9 | -CONHX₁₀ | Optionally substituted C₂ to C₈ alkenyl | R₁₀ₐOCOO- | R₂ₐCOO- | O | OH |
| I10 | -COOX₁₀ | Optionally substituted C₂ to C₈ alkynyl | R₁₀ₐOCOO- | R₂ₐCOO- | O | OH |
| I11 | -COX₁₀ | Optionally substituted C₂ to C₈ alkynyl | R₁₀ₐOCOO- | R₂ₐCOO- | O | OH |
| 112 | -CONHX₁₀ | Optionally substituted C₂ to C₈ alkynyl | R₁₀ₐOCOO- | R₂ₐCOO- | O | OH |
| J1 | -COOX₁₀ | Heterocyclo | R₁₀ₐOCOO- | R₂ₐCOO- | OH | OH |
| J2 | -COX₁₀ | Heterocyclo | R₁₀ₐOCOO- | R₂ₐCOO- | OH | OH |
| J3 | -CONHX₁₀ | Heterocyclo | R₁₀ₐOCOO- | R₂ₐCOO- | OH | OH |
| J4 | -COOX₁₀ | Optionally substituted C₂ to C₈ alkyl | R₁₀ₐOCOO- | R₂ₐCOO- | OH | OH |
| J5 | -COX₁₀ | Optionally substituted C₂ to C₈ alkyl | R₁₀ₐOCOO- | R₂ₐCOO- | OH | OH |
| J6 | -CONHX₁₀ | Optionally substituted C₂ to C₈ alkyl | R₁₀ₐOCOO- | R₂ₐCOO- | OH | OH |
| J7 | -COOX₁₀ | Optionally substituted C₂ to C₈ alkenyl | R₁₀ₐOCOO- | R₂ₐCOO- | OH | OH |
| J8 | -COX₁₀ | Optionally substituted C₂ to C₈ alkenyl | R₁₀ₐOCOO- | R₂ₐCOO- | OH | OH |
| J9 | -CONHX₁₀ | Optionally substituted C₂ to C₈ alkenyl | R₁₀ₐOCOO- | R₂ₐCOO- | OH | OH |
| J10 | -COOX₁₀ | Optionally substituted C₂ to C₈ alkynyl | R₁₀ₐOCOO- | R₂ₐCOO- | OH | OH |
| J11 | -COX₁₀ | Optionally substituted C₂ to C₈ alkynyl | R₁₀ₐOCOO- | R₂ₐCOO- | OH | OH |
| J12 | -CONHX₁₀ | Optionally substituted C₂ to C₈ alkynyl | R₁₀ₐOCOO- | R₂ₐCOO- | OH | OH |
| K1 | -COOX₁₀ | Heterocyclo | R₁₀ₐOCOO- | R₂ₐCOO- | R₉ₐCOO- | OH |
| K2 | -COX₁₀ | Heterocyclo | R₁₀ₐOCOO- | R₂ₐCOO- | R₉ₐCOO- | OH |
| K3 | -CONHX₁₀ | Heterocyclo | R₁₀ₐOCOO- | R₂ₐCOO- | R₉ₐCOO- | OH |
| K4 | -COOX₁₀ | Optionally substituted C₂ to C₈ alkyl | R₁₀ₐOCOO- | R₂ₐCOO- | R₉ₐCOO- | OH |
| K5 | -COX₁₀ | Optionally substituted C₂ to C₈ alkyl | R₁₀ₐOCOO- | R₂ₐCOO- | R₉ₐCOO- | OH |
| K6 | -CONHX₁₀ | Optionally substituted C₂ to C₈ alkyl | R₁₀ₐOCOO- | R₂ₐCOO- | R₉ₐCOO- | OH |
| K7 | -COOX₁₀ | Optionally substituted C₂ to C₈ alkenyl | R₁₀ₐOCOO- | R₂ₐCOO- | R₉ₐCOO- | OH |
| K8 | -COX₁₀ | Optionally substituted C₂ to C₈ alkenyl | R₁₀ₐOCOO- | R₂ₐCOO- | R₉ₐCOO- | OH |
| K9 | -CONHX₁₀ | Optionally substituted C₂ to C₈ alkenyl | R₁₀ₐOCOO- | R₂ₐCOO- | R₉ₐCOO- | OH |
| K10 | -COOX₁₀ | Optionally substituted C₂ to C₈ alkynyl | R₁₀ₐOCOO- | R₂ₐCOO- | R₉ₐCOO- | OH |
| K11 | -COX₁₀ | Optionally substituted C₂ to C₈ alkynyl | R₁₀ₐOCOO- | R₂ₐCOO- | R₉ₐCOO- | OH |
| K12 s | -CONHX₁₀ | Optionally ubstituted C₂ to C₈ alkynyl | R₁₀ₐOCOO- | R₂ₐCOO- | R₉ₐCOO- | OH |

### Example 5

### In Vitro cytotoxicity measured by the cell colony formation assay

Four hundred cells (HCT116) were plated in 60 mm Petri dishes containing 2.7 mL of medium (modified McCoy's 5a medium containing 10% fetal bovine serum and 100 units/mL penicillin and 100 mg/mL streptomycin). The cells were incubated in a CO2 incubator at 37 °C for 5 h for attachment to the bottom of Petri dishes. The compounds identified in Example 2 were made up fresh in medium at ten times the final concentration, and then 0.3 mL of this stock solution was added to the 2.7 mL of medium in the dish. The cells were then incubated with drugs for 72 h at 37 ° C. At the end of incubation the drug-containing media were decanted, the dishes were rinsed with 4 mL of Hank's Balance Salt Solution (HBSS), 5 mL of fresh medium was added, and the dishes were returned to the incubator for colony formation. The cell colonies were counted using a colony counter after incubation for 7 days. Cell survival was calculated and the values of ID50 (the drug concentration producing 50% inhibition of colony formation) were determined for each tested compound.

| **Compound** | **IN VITRO ID 50 (nm) HCT116** |
|---|---|
| Taxol | 2.1 |
| Docetaxel | 0.6 |
| 1755 | <1 |
| 1767 | <10 |
| 1781 | <1 |
| 1799 | <1 |
| 1808 | <10 |
| 1811 | <1 |
| 1822 | <1 |
| 1838 | <1 |
| 1841 | <1 |
| 1855 | <10 |
| 1867 | <1 |
| 1999 | <1 |
| 2002 | <1 |
| 2011 | <10 |
| 2020 | <1 |
| 2032 | <1 |
| 2044 | <1 |
| 2050 | <1 |
| 2062 | <10 |
| 2077 | <10 |
| 2086 | <1 |
| 2097 | <1 |
| 2666 | <1 |
| 2972 | <10 |
| 2988 | <1 |
| 2999 | <1 |
| 3003 | <10 |
| 3011 | <1 |
| 3020 | <1 |
| 3033 | <10 |
| 3155 | <1 |
| 3181 | <1 |
| 3243 | <1 |
| 3300 | <10 |
| 3393 | >50 |
| 3433 | 22.3 |
| 3911 | <1 |
| 3929 | <1 |
| 3963 | <1 |
| 4000 | <1 |
| 4020 | <1 |
| 4074 | <1 |
| 4088 | <10 |
| 4090 | <1 |
| 4374 | <1 |
| 4636 | <10 |
| 6466 | <10 |
| 4959 | <1 |
| 4924 | <10 |
| 4844 | <1 |
| 5171 | <1 |
| 5155 | <10 |
| 1788 | <1 |
| 1767 | <10 |
| 1771 | <10 |
| 1866 | <1 |
| 2060 | <10 |
| 2092 | <1 |
| 2088 | <1 |

### Example 6

### Preparation of Solutions for Oral Administration

Solution 1: Antitumor compound 1771 was dissolved in ethanol to form a solution containing 145 mg of the compound per ml of solution. An equal volume of Cremophor® EL solution was added to the solution while stirring to form a solution containing 72.5 mg of compound 1771 per ml of solution. This solution was diluted using 9 parts by weight of saline to form a pharmaceutically acceptable solution for administration to a patient.
Solution 2: Antitumor compound 1781 was dissolved in ethanol to form a solution containing 98 mg of the compound per ml of solution. An equal volume of Cremophor® EL was added to the solution while stirring to form a solution containing 49 mg of compound 1781 per ml of solution. This solution was diluted using 9 parts by weight of saline to form a pharmaceutically acceptable solution for administration to a patient.

### Example 7

### Preparation of a Suspension for Oral Administration

An oral composition of an antitumor compound of the invention can be prepared by suspending 25 mg of the compound as a fine powder in one ml of carrier containing 1% carboxymethylcellulose (CMC) in deionized water.

### Example 8

### Preparation of a Tablet for Oral Administration

An antitumor compound of the invention (100 mg) can be dissolved in methylene chloride (2 ml) and Cremophor® EL solution (100mg) can be added. The methylene chloride can be evaporated under vacuum to form a glass. Microcrystalline cellulose (600 mg) can be added to the glass and mixed to form a powder which can be processed to form a tablet.

### Example 9

### Preparation of Emulsions for Parenteral Administration

Emulsion 1: An antitumor compound of the invention can be dissolved in 100% ethanol to form a solution containing 40 mg of the compound per ml of the solution. The solution can be diluted with 19 parts by weight of Liposyn® II (20%) with stirring to form an emulsion containing 2 mg of the compound per ml for parenteral administration.
Emulsion 2: An antitumor compound of the invention can be dissolved in 100% ethanol to form a solution containing 40 mg of the compound per ml of the solution. The solution can be diluted with 19 parts by weight of Liposyn® III (2%) with stirring to form an emulsion containing 2 mg of the compound per ml for parenteral administration.
Emulsion 3: An antitumor compound of the invention can be dissolved in 100% ethanol to form a solution containing 40 mg of the compound per ml of the solution. The solution can be diluted with 9 parts by weight of Liposyn® III (2%) with stirring to form an emulsion containing 4 mg of the compound per ml for parenteral administration.

### Example 10

### Preparation of Solutions Containing the Compound for Parenteral Administration

Solution 1: An antitumor compound of the invention can be dissolved in 100% ethanol to form a solution containing 140 mg of the compound per ml. The solution can be diluted with an equal volume of Cremophor® EL solution with stirring and then diluted with 9 parts by weight of normal saline to form a solution containing 7 mg of the compound per ml of solution for parenteral administration.
Solution 2: An antitumor compound of the invention can be dissolved in 100% ethanol to form a solution containing 140 mg of the compound per ml of the solution. The solution can be diluted with an equal volume of Cremophor® EL solution with stirring and then diluted with 4 parts by weight of normal saline to form a solution containing 11.7 mg of the compound per ml of solution for parenteral administration.
Solution 3: An antitumor compound of the invention can be dissolved in 100% ethanol to form a solution containing 140 mg of the compound per ml of the solution. The solution can be diluted with an equal volume of Cremophor® EL solution with stirring and then diluted with 2.33 parts by weight of normal saline to form a solution containing 16.2 mg of the compound per ml of solution for parenteral administration.

### Example 11

### In Vivo Evaluation of Antitumor Compounds Against Human Lung Carcinoma Xenografts

Antitumor compound 1755 was formulated for an i.v. dosing regime prepared in the following vehicle: 10% ethanol, 10% Cremophor and 80% isotonic saline. The compounds were prepared in the manner as described for Solution 2 of Example 10. Taxol (paclitaxel, Bristol Meyers Squibb), used as a control, was obtained as the marketed pharmaceutical drug.

The two lung tumor xenografts used in the study were the SK-MES carcinoma, which is very sensitive to Taxol, and the NCI-H1299 (H1299) carcinoma, which is more resistant to Taxol compared to the SK-MES neoplasm.

Female NCr-nude mice bearing either SK-MES or H1299 neoplasms (implanted subcutaneously in the flank with 1mm³ human lung carcinoma fragments) were pair-matched into groups of six mice each on Day 1, with the mean tumor size for the groups ranging from 241-244 mg. Treatment groups consisted of: treatment with the vehicle (Group 1); treatment with Taxol (Group 2); and treatment with antitumor compound 1755 (Group 3).

Treatments with antitumor compound 1755 was administered intravenously at the maximum tolerated dose (MTD) appropriate for the compound, with half the dose being given one hour apart on the qd x 1 schedule. The MTD for antitumor compound 1755 was calculated from earlier single dose data for mice receiving i.v. administration of this compound. Taxol itself was given as a split i.v. dose of 36 mg/kg, with the two 18 mg/kg doses separated by one hour. Vehicle control animals were dosed twice intravenously with half the total volume being given separated by one hour. The study was terminated on Day 60.

The results are summarized in Table 1 and include mean days of survival (MDS), number of toxic deaths, number of survivors, number of complete or partial responses, and number with stable disease. The mean days of survival is the number of days after which a SKMES neoplasm reached a size of 1.5 g and the animal was euthanized. A complete response is obtained if the tumor is not palpable at the end of the study. A partial response is obtained if the tumor shrinks to a size smaller than its size on Day 1 of the study. "Stable disease" occurs when the treatment limits the growth of the neoplasm to a small size that does not reach 1.5 g in size by the termination of the study.

A tumor scoring system was designed to provide a more quantitative ranking of efficacy (therapeutic potential) among the antitumor agents evaluated against the different human solid tumor xenografts. Each mouse in a study was given a score of from 1 to 10 at the end of the study. The scores can be summarized as follows:

| Score | Description |
|---|---|
| <1 | Serious toxicity |
| 2-3 | Tumor reached the cut-off size but significant tumor growth |
| | delay was apparent |
| 4-6 | Tumor growth was significantly inhibited (Stable Disease) |
| 7-9 | Partial tumor shrinkage (Partial Response) |
| 10 | Complete Response (Perfect score) |

The individual score for each mouse was averaged into a mean score for each treatment group. This tumor scoring system provided a better comparison of the different antitumor compounds by quantifying the treatment results (complete response versus partial response).

**Table 1**

| Group | MDS (n) | Toxic Deaths | Survivors | Complete Response | Partial Response | Stable Disease | Mean Score |
|---|---|---|---|---|---|---|---|
| 1 | 12.2 ± 1.3 (6) | 0 | 0 | 0 | 0 | 0 | 1 ± 0 |
| 2 | 16 ± 2.0 (6) | 0 | 0 | 0 | 0 | 0 | 1.3 ± 0.3 |
| 3 | -- | 0 | 6 | 5 | 0 | 1 | 9.2 ± 0.8 |

An MDS value of 12.2 days was calculated for the vehicle-treated control group. Taxol (36 mg/kg; Group 2) produced a modest 30% survival extension (MDS = 16.0 days) compared to vehicle controls, with no recorded tumor regressions. In contrast, the antitumor compound 1755 was highly active. Compound 1755 (49 mg/kg; Group 4) produced five complete responses with significant survival extension recorded in the remaining treated animal compared to the vehicle or Taxol control groups. Antitumor compound 1755 was well tolerated.

In the H1299 experiment, mice were pair-matched into six groups of six animals each on Day 1, with the mean tumor size for the six groups ranging from 229-233 mg. The treatment protocol for the H1299 test was identical to the SK-MES test protocol. A MDS value of 24.5 days was calculated for the vehicle-treated control Group 1. Taxol (36 mg/kg; Group 2) was not active in the H1299 model, causing only a 10% survival extension (MDS = 27.0 days) in five animals compared to vehicle-treated animals (not statistically significant). One partial response was observed following Taxol treatment.

**Table 2**

| Group | MDS (n) | Toxic Deaths | Survivors | Complete Response | Partial Response | Stable Disease | Mean Score |
|---|---|---|---|---|---|---|---|
| 1 | 24.5 ± 3.3 (6) | 0 | 0 | 0 | 0 | 0 | 1.2 ± 0.2 |
| 2 | 27.0 ± 4.6 (5) | 0 | 1 | 0 | 1 | 0 | 2.7 ± 1.3 |
| 3 | - | 2 | 4 | 3 | 0 | 1 | 5.8 ± 2 |

Antitumor compound 1755 (49 mg/kg; Group 4) demonstrated significant efficacy against the Taxol-refractory H1299 neoplasm producing three complete responses in the H1299 test. Other mice treated with this compound experienced significantly prolonged survival as compared to vehicle-treated or Taxol-treated animals.

Two mice died in the H1299 test from toxicity in the group treated by antitumor compound 1755. Since the doses and schedule were the same for the three compounds as in the SK-MES experiment, the side effects experienced by the nude mice most likely were not due to systemic drug toxicity. One explanation based on the extreme reaction of the H1299 tumors to antitumor compound 1755, including necrosis and hemorrhaging of the carcinomas, is that the compound caused a destruction of the tumor architecture and stroma resulting in a release of toxic substance from the neoplasm to the host mice.

Compound 1755 was effective when administered as a single bolus against upstaged (250 mg) human lung carcinoma xenografts.

### Example 12

### In Vivo Evaluation of Antitumor Compounds Against DU 145 Human Prostrate Carcinoma Xenografts

Antitumor compounds were prepared in a vehicle of 5% ethanol, 5% Cremophor and 90% isotonic saline in the manner as described for Solution 1 of Example 10. Taxol (paclitaxel; Bristol Meyers Squibb) was obtained as the marketed pharmaceutical drug. The dosing volume was 0.3 ml per 20 g mouse.

Male NCr-nude mice were implanted subcutaneously with 1 mm³ DU145 prostate carcinoma fragments in the flank and sorted into treatment groups of five mice each. The DU145 group mean size range was 223-228 mg. Dosing was initiated on Day 1. The antitumor compounds were given i.v. at an estimated maximum tolerated dose (MTD) appropriate for each agent on a qd x 1 schedule (single administration of compound). The i.v. Taxol was given on the qd x 1 schedule as two split doses (one hour between) of 12 mg/kg per split dose (total dose = 24 mg/kg). Taxol was also administered intraperitoneally (i.p.) on a qd x 5 schedule at a daily dose of 18 mg/kg. The vehicle was administered i.v. to control mice in Group 1 on the qd x 1 schedule. The study was terminated on Day 91. Treatment groups are detailed in Table 3.

**Table 3**

| **Group** | **Compound** | **Mg/kg** | **Route** | **Schedule** |
|---|---|---|---|---|
| 1 | Vehicle | --- | i.v. | qd x 1 |
| 2 | Taxol | 24 | i.v. | qd x 1 |
| 3 | Taxol | 18 | i.p | qd x 5 |
| 4 | 1781 | 72.6 | i.v. | qd x 1 |

The results are summarized in Table 4 and include mean days of survival (MDS), number of toxic deaths, number of survivors, number of complete or partial responses, and number with stable disease. The mean days of survival is the number of days after which a neoplasm reached a size of 1.5 g and the animal was euthanized. A complete response is obtained if the tumor shrinks to a size smaller than its size on Day 1 of the study. "Stable disease" occurs when the treatment limits growth of the neoplasm to a small size that does not reach 1.5 g in size by the termination of the study.

**Table 4**

| **Group** | **MDS (n)** | **Toxic Deaths** | **Survivors** | **Complete Response** | **Partial Response** | **Stable Disease** | **Mean Score** |
|---|---|---|---|---|---|---|---|
| 1 | 34.3 ± 9.4 (3) | 1 | 1 | 1 | 0 | 0 | 2.8 ± 1.8 |
| 2 | 37.4 ± 8.9 (3) | 1 | 1 | 0 | 0 | 1 | 2 ± 1 |
| 3 | --- | 5 | 0 | 0 | 0 | 0 | 0 ± 0 |
| 4 | --- | 5 | 0 | 0 | 0 | 0 | 0 ± 0 |

DU145 tumors grew progressively in three of the five mice treated with vehicle (Group 1); an MDS value of 34.3 days was calculated for these animals. One mouse in Group 1 died of unknown causes on Day 40 and the carcinoma of another mouse in Group 1 regressed slowly from its 220 mg starting size of pair-match until the neoplasm was no longer palpable on Day 71. This latter case is most likely an example of a poor tumor take.

The prostate carcinomas in three of five animals receiving Taxol at 24 mg/kg (i.v.; qd x 1) grew steadily and reached the 1.5 g endpoint with a MDS = 37.4 days (Group 2). This 9% increase in survival compared to Group 1 controls is not statistically different (p = 0.82; unpaired t-test). One animal in Group 2 died on Day 32, possibly from drug-related side effects. The fifth mouse in Group 2 did not experience net tumor growth over the 91 day study; the carcinoma was the same size (196 mg) on Day 6 as on Day 91. Taxol administered i.p. at a dose of 18 mg/kg on a qd x 5 schedule (Group 3) was highly toxic. All five mice died of drug-related side effects including substantial (often more than 20%) weight loss.

Antitumor compound1781 had a higher than 40% mortality rate at the administered dose out of the five animals treated with each compound. Antitumor compound1781 was 100% lethal to the mice at the administered dose. This compound produced weight loss over 20% in a number of the treated mice prior to their deaths.

### Example 13

### In Vivo Evaluation of Antitumor Compounds Against A2780 Human Ovarian Carcinoma Xenografts

Antitumor compounds were prepared in a vehicle of 5% ethanol, 5% Cremophor and 90% isotonic saline in the manner as described for Solution 1 in Example 10. Taxol (paclitaxel; Bristol Meyers Squibb) and Taxotere (docetaxel; Rhône-Poulenc Rorer) were obtained as the marketed pharmaceutical drugs. The dosing volume was 0.3 ml per 20 g mouse.

Female NCr-nude mice were implanted subcutaneously with 1 mm³ A2780 melanoma fragments in the flank were sorted into treatment groups of 5 mice each, except the Taxotere treatment group which was a group of six mice. The A2780 group mean size range was 237-243 mg. Dosing was initiated on Day 1. The antitumor compounds were given i.v. at an estimated maximum tolerated dose (MTD) appropriate for each agent on a qd x 1 schedule (single administration of compound). The i.v. Taxol was given on the qd x 1 schedule as two split doses (one hour between) of 12 mg/kg per split dose (total dose = 24 mg/kg). Taxol was also administered i.p. on a qd x 5 schedule at a daily dose of 15 mg/kg. Taxotere was given i.v. on a qd x 1 schedule at a dose of 70 mg/kg. The vehicle was administered i.v. to control mice in Group 1 on the qd x 1 schedule. The study was terminated on Day 60. Treatment groups are detailed in Table 5.

**Table 5**

| **Group** | **Compound** | **Mg/kg** | **Route** | **Schedule** |
|---|---|---|---|---|
| 1 | Vehicle | --- | i.v. | qd x 1 |
| 2 | Taxol | 24 | i.v. | qd x 1 |
| 3 | Taxol | 15 | i.p. | qd x 5 |
| 4 | 1781 | 60.6 | i.v. | qd x 1 |
| 5 | Taxotere | 70 | i.v. | qd x 1 |

The results are summarized in Table 6 and include mean days of survival (MDS), number of toxic deaths, number of survivors, number of complete or partial responses, and number with stable disease. The mean days of survival is the number of days after which a neoplasm reached a size of 2.0 g and the animal was euthanized. A complete response is obtained if the tumor shrinks to a size smaller than its size on Day 1 of the study. "Stable disease" occurs when the treatment limits growth of the neoplasm to a small size that does not reach 2.0 g in size by the termination of the study.

**Table 6**

| **Group** | **MDS (n)** | **Toxic Deaths** | **Survivors** | **Complete Response** | **Partial Response** | **Stable Disease** | **Mean Score** |
|---|---|---|---|---|---|---|---|
| 1 | 11.7 ± 2.5(5) | 0 | 0 | 0 | 0 | 0 | 1.2 ± 0.2 |
| 2 | 21.9 ± 5.5 (5) | 0 | 1 | 0 | 0 | 0 | 1.8 ± 0.4 |
| 3 | 26.5 (1) | 4 | 0 | 0 | 0 | 0 | 0.6 ± 0.6 |
| 4 | 38.5 ± 8.3 (2) | 0 | 3 | 0 | 2 | 1 | 6 ± 1.3 |
| 5 | 27.4 ± 3.5 (5) | 0 | 0 | 0 | 0 | 0 | 2.5 ± 0.3 |

The ovarian tumors in all five mice in Group 1 receiving vehicle grew progressively and reached the 2.0 g cut-off with a MDS value of 11.7 days. Thus, this ovarian carcinoma xenograft is a fast growing neoplasm that kills its host in a relatively short time period.

Taxol given on a qd x 1 schedule (Group 2) was moderately efficacious in this test. The MDS value of 21.9 days calculated for Group 2 mice represents a 87% increase in survival compared to Group 1 control animals, which is not significant (at p = 0.13; unpaired t-test). One animal in Group 2 was a recorded case of stable disease on Day 61. Taxol administered i.p. at a dose of 15 mg/kg on a qd x 5 schedule (Group 3) was highly toxic; four mice succumbed to drug-related side effects.

Taxotere (70 mg/kg; i.v.; qd x 1) was more active than Taxol in this study. The MDS of 27.4 days for all five mice in Group 5 gives a calculated survival extension of 134% compared to Group 1 controls, which is statistically significant at p = 0.007 (unpaired t-test).

Antitumor compound 1781 produced at least one complete response or partial response (total response rate of at least 20%) in mice bearing A2780 ovarian carcinomas. Compound 1781 produced 2 partial responses among the five mice treated.

The groups treated with antitumor compound 1781 experienced maximal mean group weight losses in the 3% to 19% range around Day 11, and the animal weights rebounded thereafter. Thus the side effects recorded for antitumor compound 1781 were within the NCI acceptable range. This indicated that the animals in the A2780 experiment received reasonable MTD dosages.

### Example 14

### In Vivo Evaluation of Antitumor Compounds Against A375 Human Melanoma Xenografts

Antitumor compounds were prepared in a vehicle of 5% ethanol, 5% Cremophor and 90% isotonic saline in the manner as described for Solution 1 of Example 10. Taxol (paclitaxel; Bristol Meyers Squibb) and Taxotere (docetaxel; Rhône-Poulenc Rorer) were obtained as the marketed pharmaceutical drugs. The dosing volume was 0.3 ml per 20 g mouse.

Female NCr-nude mice were implanted subcutaneously with 1 mm³ A375 melanoma fragments in the flank were sorted into treatment groups of six mice each, except the Taxotere treatment group which was a group of seven mice. The A375 tumor group mean size range was 206-212 mg. Dosing was initiated on Day 1. The antitumor compounds were given i.v. at an estimated maximum tolerated dose (MTD) appropriate for each agent on a qd x 1 schedule (single administration of compound). The i.v. taxol was given on the qd x 1 schedule as two split doses (one hour between) of 12 mg/kg per split dose (total dose = 24 mg/kg). Taxol was also administered i.p. on a qd x 5 schedule at a daily dose of 18 mg/kg. Taxotere was given i.v. on a qd x 1 schedule at a dose of 70 mg/kg. The vehicle was administered i.v. to control mice in Group 1 on the qd x 1 schedule. The study was terminated on Day 60. Treatment groups are detailed in Table 7.

**Table 7**

| **Group** | **Compound** | **Mg/kg** | **Route** | **Schedule** |
|---|---|---|---|---|
| 1 | Vehicle | --- | i.v. | qd x 1 |
| 2 | Taxol | 24 | i.v. | qd x 1 |
| 3 | Taxol | 18 | i.p. | qd x 5 |
| 4 | 1781 | 72.6 | i.p. | qd x 1 |
| 5 | Taxotere | 70 | i.v. | qd x 1 |

The results are summarized in Table 8 and include mean days of survival (MDS), number of toxic deaths, number of survivors, number of complete or partial responses, and number with stable disease. The mean days of survival is the number of days after which a neoplasm reached a size of 2.0 g and the animal was euthanized. A complete response is obtained if the tumor shrinks to a size smaller than its size on Day 1 of the study. "Stable disease" occurs when the treatment limits growth of the neoplasm to a small size that does not reach 2.0 g in size by the termination of the study.

**Table 8**

| **Group** | **MDS (n)** | **Toxic Deaths** | **Survivors** | **Complete Response** | **Partial Response** | **Stable Disease** | **Mean Score** |
|---|---|---|---|---|---|---|---|
| 1 | 14.4 ± 0.9 (6) | 0 | 0 | 0 | 0 | 0 | 1 ± 0 |
| 2 | 18.1 ± 0.6 (4) | 2 | 0 | 0 | 0 | 0 | 0.8 ± 0.3 |
| 3 | --- | 6 | 0 | 0 | 0 | 0 | 0 ± 0 |
| 4 | 45.6(1) | 5 | 0 | 0 | 0 | 0 | 0.5 ± 0.5 |
| 5 | 21.5 ± 0.5(7) | 0 | 0 | 0 | 0 | 0 | 2 ± 0 |

The A375 melanoma implants grew rapidly and progressively in all six mice treated with vehicle (Group 1). These neoplasms reached the 2.0 g cut-off with a MDS value of 14.4 days.

Animals administered i.v. Taxol on a qd x 1 schedule (24 mg/kg; Group 2) experienced a modest therapeutic benefit from the chemotherapy drug. The MDS value of 18.1 days represented a 26% survival extension compared to Group 1 controls. This survival increase was statistically significant (p = 0.016; unpaired t-test). Taxol given on a qd x 5 schedule (18 mg/kg; i.p.) was highly toxic to the mice. All six animals dosed at 18 mg/kg died of toxicity.

Taxotere given i.v. at a dose of 70 mg/kg (qd x 1; Group 5) produced an MDS of 21.5 days. This 49% survival extension was significant compared to Group 1 controls at p < 0.0001 (unpaired t-test).

Antitumor compound 1781 caused five toxic deaths among the six mice in the group receiving this agent. In general, 20-30% mean group weight losses were recorded for animals treated with this agent.

### Example 15

### In Vivo Evaluation of Antitumor Compounds Against Panc-1 Human Pancreatic Carcinoma

Antitumor compounds were prepared in a vehicle of 5% ethanol, 5% Cremophor and 90% isotonic saline in the manner as described for Solution 1 of Example 10. Taxol (paclitaxel; Bristol Meyers Squibb) was obtained as the marketed pharmaceutical drug. The dosing volume was 0.3 ml per 20 g mouse.

Female NCr-nude mice were implanted subcutaneously with 1 mm³ Panc-1 pancreatic carcinoma fragments in the flank and sorted into treatment groups of six mice each. The Panc-1 tumor group mean size range was 192-213 mg. Dosing was initiated on Day 1. The antitumor compounds were given i.v. at an estimated maximum tolerated dose (MTD) appropriate for each agent on a qd x 1 schedule (single administration of compound). The i.v. taxol was given on the qd x 1 schedule as two split doses (one hour between) of 12 mg/kg per split dose (total dose = 24 mg/kg). Taxol was also administered at a dose of 15 mg/kg i.p. on a qd x 5 schedule. The vehicle was administered i.v. to control mice in Group 1 on the qd x 1 schedule. The study was terminated on Day 63. Treatment groups are detailed in Table 9.

**Table 9**

| **Group** | **Compound** | **Mg/kg** | **Route** | **Schedule** |
|---|---|---|---|---|
| 1 | Vehicle | --- | i.v. | qd x 1 |
| 2 | Taxol | 24 | i.v. | qd x 1 |
| 3 | Taxol | 15 | i.p. | qd x 5 |
| 4 | 1781 | 60 | i.v. | qd x 1 |

The results are summarized in Table 10 and include mean days of survival (MDS), number of toxic deaths, number of survivors, number of complete or partial responses, and number with stable disease. The mean days of survival is the number of days after which a neoplasm reached a size of 1.5 g and the animal was euthanized. A complete response is obtained if the tumor shrinks to a size smaller than its size on Day 1 of the study. "Stable disease" occurs when the treatment limits growth of the neoplasm to a small size that does not reach 1.5 g in size by the termination of the study.

**Table 10**

| **Group** | **MDS (n)** | **Toxic Deaths** | **Survivors** | **Complete Response** | **Partial Response** | **Stable Disease** | **Mean Score** |
|---|---|---|---|---|---|---|---|
| 1 | 23.0 ± 3.1(5) | 0 | 1 | 0 | 0 | 1 | 2 ± 0.8 |
| 2 | 34.7 ± 3.4(4) | 1 | 1 | 0 | 0 | 1 | 2.2 ± 0.8 |
| 3 | --- | 4 | 1 | 0 | 1 | 0 | 1.2 ± 1.2 |
| 4 | 59.3 ± 2.9(2) | 0 | 4 | 0 | 4 | 0 | 6.7 ± 1.2 |

The Panc-1 neoplasms of mice receiving vehicle (Group 1) reached the 1.5 g cut-off with a calculated MDS of 23.0 days. One carcinoma in the vehicle control group did not grow out, presumably because of a poor tumor take.

Taxol (Group 2) administered i.v. at a dose of 24 mg/kg (qd x 1) produced one case of stable disease, and caused a MDS of 34.7 days in four other mice (significantly different from Group 1 controls at p = 0.038; unpaired *t*-test). One mouse died from the toxicity caused by this dose of Taxol. Taxol given i.p. at a dose of 15 mg/kg (qd x 5) caused four toxic deaths among the six animals in Group 3.

Antitumor compound 1781 produced an overall response rate of 66.7%. Antitumor compound 1781 was well-tolerated, causing no toxic deaths and minimal mean group weight loss. Therefore this compound was administered at a reasonable MTD dosage.

### Example 16

### In Vivo Evaluation of Antitumor Compounds Against VM46 Human Colon Carcinoma

Antitumor compounds were prepared in a vehicle of 5% ethanol, 5% Cremophor and 90% isotonic saline in the manner as described for Solution 1 of Example 10. Taxol (paclitaxel; Bristol Meyers Squibb) and Taxotere (docetaxel; Rhône-Poulenc Rorer) were obtained as the marketed pharmaceutical drugs. The dosing volume was 0.3 ml per 20 g mouse.

Female NCr-nude mice were implanted subcutaneously with 1 mm³ VM46 colon carcinoma fragments in the flank and sorted into treatment groups of six mice each. The VM46 tumor group mean size range was 181-188 mg. Dosing was initiated on Day 1. The antitumor compounds were given i.v. at an estimated maximum tolerated dose (MTD) appropriate for each agent on a qd x 1 schedule (single administration of compound). The i.v. Taxol was given on the qd x 1 schedule as two split doses (one hour between) of 12 mg/kg per split dose (total dose = 24 mg/kg). Taxol was also administered at a dose of 15 mg/kg i.p. on a qd x 5 schedule. Taxotere was given i.v. at a dose of 70 mg/kg on a qd x 1 schedule. The vehicle was administered i.v. to control mice in Group 1 on the qd x 1 schedule. The study was terminated on Day 64. Treatment groups are detailed in Table 11.

**Table 11**

| **Group** | **Compound** | **Mg/kg** | **Route** | **Schedule** |
|---|---|---|---|---|
| 1 | Vehicle | --- | i.v. | qd x 1 |
| 2 | Taxol | 24 | i.v. | qd x 1 |
| 3 | Taxol | 15 | i.p. | qd x 5 |
| 4 | 1781 | 60 | i.v. | qd x 1 |
| 5 | Taxotere | 70 | i.v. | qd x 1 |

The results are summarized in Table 12 and include mean days of survival (MDS), number of toxic deaths, number of survivors, number of complete or partial responses, and number with stable disease. The mean days of survival is the number of days after which a neoplasm reached a size of 1.5 g and the animal was euthanized. A complete response is obtained if the tumor shrinks to a size smaller than its size on Day 1 of the study. "Stable disease" occurs when the treatment limits growth of the neoplasm to a small size that does not reach 1.5 g in size by the termination of the study.

**Table 12**

| **Group** | **MDS (n)** | **Toxic Deaths** | **Complete Response** | **Partial Response** | **Stable Disease** | **Mean Score** |
|---|---|---|---|---|---|---|
| 1 | 31.0 ± 4.4(6) | 0 | 0 | 0 | 0 | 1.2 ± 0.2 |
| 2 | 43.3 ± 3.7 (4) | 0 | 0 | 1 | 1 | 3.8 ± 1.4 |
| 3 | 36.1 ± 12.2(2) | 3 | 0 | 1 | 0 | 1.8 ± 1.3 |
| 4 | 42.2 ± 7.1(5) | 0 | 0 | 1 | 0 | 3 ± 1.2 |
| 5 | 45.3 ± 3.6(6) | 0 | 0 | 0 | 0 | 2 ± 0 |

The colon cancers in all six mice receiving vehicle (Group 1) grew progressively, and reached the 1.5 g endpoint with a calculated MDS value of 31.0 days.

Taxol (Group 2) given at 24 mg/kg (i.v.; qd x 1) caused one partial response, one case of stable disease, and a MDS = 43.3 days for the remaining four mice in the group (not significantly different from controls at p = 0.086; unpaired *t*-test). Taxol administered i.p. at a dose of 15 mg/kg (qd x 5) was excessively toxic, causing three mortalities among the six treated animals. Taxotere was quite active in this test, producing MDS values of 45.3 days (significant at p #0.05 compared to Group 1 controls; unpaired *t*-test).

Antitumor compound 1781 achieved a response rate of 16.7%. Survival extensions in the range of 11 to 20 days longer than the MDS of 31.0 days calculated for the control group was obtained with antitumor compound 1781. Antitumor compound 1781 was well-tolerated, causing no toxic deaths and maximal mean group weight losses in the 3-14% range on Day 8.

### Example 17

### In Vivo Evaluation of the Oral Administration of Antitumor Compound 1755 Against the SKMES Human Lung Carcinoma Xenograft

Antitumor compound 1755, in a vehicle of 5% ethanol, 5% Cremophor and 90% isotonic saline, was prepared in a manner as described for Solution 1 of Example 6. The compound was administered as a single bolus by the oral route in a study to investigate the efficacy of this compound against the SKMES human lung cancer xenograft.

Female Nu/Nu mice (6-8 weeks of age; implanted with 1 mm³ SKMES lung carcinoma fragments in the flank) were sorted into four treatment groups of six mice each. SKMES size ranges and group mean SKMES size ranges were 126-448 mg and 238-241 mg, respectively. Treatment groups consisted of: no treatment (Group 1), vehicle only (Group 2), Taxol (40 mg/kg; Group 3), and antitumor compound 1755 (49 mg/kg; Group 4). Treatments were administered as a single oral bolus on Day 1, and the study was terminated on Day 60.

The results are summarized in Table 13 and include mean days of survival (MDS), number of toxic deaths, number of survivors, number of complete or partial responses, and number with stable disease. The mean days of survival is the number of days after which a SKMES neoplasm reached a size of 2.0 g and the animal was euthanized. A complete response is obtained if the tumor is not palpable at the end of the study. A partial response is obtained if the tumor shrinks to a size smaller than its size on Day 1 of the study. "Stable disease" occurs when the treatment limits the growth of the neoplasm to a small size that does not reach 2.0 g in size by the termination of the study.

**Table 13**

| Group | MDS (n) | Toxic Deaths | Survivors | Complete Response | Partial Response | Stable Disease | % Total Response |
|---|---|---|---|---|---|---|---|
| 1 | 14.5 ± 1.7(5) | 0 | 1 | 0 | 0 | 1 | 0 |
| 2 | 13.7 ± 1.4(6) | 0 | 0 | | 0 | 0 | 0 |
| 3 | 13.2 ± 1.4(6) | 0 | 0 | 0 | 0 | 0 | 0 |
| 4 | --- | 0 | 6 | 4 | 2 | 0 | 100 |

Antitumor compound 1755 was highly active with a single oral administration. The total response rate was 100% with four complete responses and two partial responses. The total response rate is defined as the percentage of evaluable animals (excluding deaths due to procedure or toxicity) in a group that experience complete or partial responses at the termination of the study.

### Example 18

### In Vivo Evaluation of the Oral Administration of Antitumor Compounds Against the MX-1 Human Breast Carcinoma Xenograft

Antitumor compounds were administered orally in a study to investigate whether such formulations are efficacious against the MX-1 human breast carcinoma xenograft. The compounds were prepared in the following vehicle: 90% saline, 5% Cremophor, and 5% ethanol. Antitumor compound 1771 was formulated as described for Solution 1 in Example 6. Antitumor compound 1781 was formulated as described for Solution 2 in Example 6.

Female *nu*/*nu* mice (11-12 weeks of age; implanted subcutaneously with 1 mm³ MX-1 human breast carcinoma fragments in the flank) were sorted into one control (n=10) and two treatment (n=6) groups (group mean tumor size of 47-49 mg). Treatment groups are detailed in Table 14. All treatments were administered as a single oral bolus on Day 1, and the study was terminated on Day 63.

The results are summarized in Table 14 and include mean days of survival (MDS), number of toxic deaths, number of survivors, number of complete or partial responses, and number with stable disease. The mean days of survival are the number of days after which a neoplasm reached a size of 1.5 g and the animal was euthanized. A complete response is obtained if the tumor shrinks to a size smaller than its size on Day 1 of the study. "Stable disease" occurs when the treatment limits growth of the neoplasm to a small size that does not reach 1.5 g in size by the termination of the study.

**Table 14**

| Group | Anti-Tumor Cmpd. | Dose (mg/kg) | Mean Day of Survival (n) | Maximum Weight Loss (Day) | Toxic Deaths | Complete Response | Partial Response | Stable Disease |
|---|---|---|---|---|---|---|---|---|
| 1 | vehicle | n/a | 21.5 ± 0.7 (8) | n/a | 0 | 0 | 0 | 2 |
| 2 | 1771 | 107 | 60.8 ± 1.0 (2)) | -8.1% (5) | 0 | 4 | 0 | 0 |
| 3 | 1781 | 73 | 33.3 ± 2.9 (5) | -9.4% (5) | 0 | 0 | 0 | 1 |

The antitumor compounds evaluated are active in the MX-1 human breast carcinoma xenograft model, at well tolerated doses. Antitumor compound 1771 resulted in four complete tumor regressions of six mice treated, the other two mice in the group experiencing a significant survival increase versus controls before reaching the study endpoint. The response produced by antitumor compounds 1781 was characterized by significant survival extensions compared to vehicle control mice.

In addition to survival and tumor regression responses, the effect of treatment on blood cell populations was assessed. The major effect of these antitumor compounds on blood cell populations was a marked reduction in the neutrophil cell number. Monocyte depletion correlated fairly well with neutrophil reduction for each antitumor compound. The antitumor compounds did not affect the general white blood cell population, lymphocytes, and platelets. There was a correlation between antitumor activity and reduced neutrophil count. In general, toxicity (as determined by neutrophil depletion) and weight loss correlated with efficacy. Importantly, the toxicities observed with the very efficacious antitumor compounds were manageable and reversible; neutrophil count and body weight rebounded from their nadirs in just a few days. Table 15 summarized the weight reduction, neutrophil and monocyte measurements on Day 4, with tumor weight reduction calculated at Day 18:

**Table 15**

| Group | Antitumor Compound | Dose (mg/kg) | Weight (mg) | % Reduction | Neutrophils (K/µL) | % Reduction | Monocytes (K/µL) | % Reduction |
|---|---|---|---|---|---|---|---|---|
| 1 | Vehicle | n/a | 868.5 | - | 0.9115 | | 0.096 | - |
| 2 | 1771 | 107 | 0.1 | 100.0% | 0.044 | 95.2% | 0.045 | 53.1% |
| 3 | 1781 | 73 | 275.3 | 68.3% | 0.117 | 87.2% | 0.101 | - |

## Claims

1. A taxane having the formula: 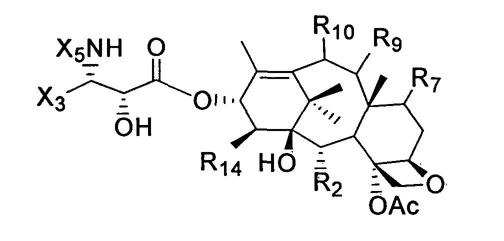 wherein
R₂ is acyloxy;
R₇ is hydroxy;
R₉ is keto, hydroxy, or acyloxy;
R₁₀ is carbonate;
R₁₄ is hydrido or hydroxy;
X₃ is substituted or unsubstituted alkyl, alkenyl, alkynyl, or heterocyclo, wherein alkyl comprises at least two carbon atoms;
X₅ is -COX₁₀, -COOX₁₀, or -CONHX₁₀;
X₁₀ is hydrocarbyl, substituted hydrocarbyl, or heterocyclo; and
Ac is acetyl.

2. The taxane of claim 1 wherein R₁₀ is R₁₀ₐOCOO- and R₁₀ₐ is substituted or unsubstituted C₁ - C₈ alkyl, C₂- C₈ alkenyl or C₂ - C₈ alkynyl.

3. The taxane of claim 2 wherein X₃ is furyl, thienyl, pyridyl, C₂ - C₈ alkyl, C₂ - C₈ alkenyl, or C₂ - C₈ alkynyl.

4. The taxane of claim 2 wherein X₅ is -COX₁₀ and X₁₀ is phenyl, or X₅ is -COOX₁₀ and X₁₀ is t-butyl.

5. The taxane of claim 2 wherein R₁₄ is hydrido.

6. The taxane of claim 5 wherein X₃ is furyl, thienyl, pyridyl, C₂ - C₈ alkyl, C₂ - C₈ alkenyl, or C₂ - C₈ alkynyl.

7. The taxane of claim 5 wherein X₅ is -COX₁₀ and X₁₀ is phenyl, or X₅ is -COOX₁₀ and X₁₀ is t-butyl.

8. The taxane of claim 2 wherein R₂ is benzoyloxy.

9. The taxane of claim 8 wherein X₃ is furyl, thienyl, pyridyl, C₂ - C₈ alkyl, C₂ - C₈ alkenyl, or C₂ - C₈ alkynyl.

10. The taxane of claim 8 wherein X₅ is -COX₁₀ and X₁₀ is phenyl, or X₅ is -COOX₁₀ and X₁₀ is t-butyl.

11. The taxane of claim 2 wherein R₁₄ is hydrido and R₉ is keto.

12. The taxane of claim 11 wherein X₃ is 2-furyl, 3-furyl, 2-thienyl, 3-thienyl, 2-pyridyl, 3-pyridyl, 4-pyridyl, C₂ - C₈ alkyl, C₂ - C₈ alkenyl, or C₂ - C₈ alkynyl.

13. The taxane of claim 11 wherein X₅ is -COX₁₀ and X₁₀ is phenyl, or X₅ is -COOX₁₀ and X₁₀ is t-butyl.

14. The taxane of claim 2 wherein R₂ is benzoyloxy and R₉ is keto.

15. The taxane of claim 14 wherein X₃ is 2-furyl, 3-furyl, 2-thienyl, 3-thienyl, 2-pyridyl, 3-pyridyl, 4-pyridyl, C₂ - C₈ alkyl, C₂ - C₈ alkenyl, or C₂ - C₈ alkynyl.

16. The taxane of claim 14 wherein X₅ is -COX₁₀ and X₁₀ is phenyl, or X₅ is -COOX₁₀ and X₁₀ is t-butyl.

17. The taxane of claim 2 wherein R₁₄ is hydrido and R₂ is benzoyloxy.

18. The taxane of claim 17 wherein X₃ is 2-furyl, 3-furyl, 2-thienyl, 3-thienyl, 2-pyridyl, 3-pyridyl, 4-pyridyl, C₂ - C₈ alkyl, C₂ - C₈ alkenyl, or C₂ - C₈ alkynyl.

19. The taxane of claim 17 wherein X₅ is -COX₁₀ and X₁₀ is phenyl, or X₅ is -COOX₁₀ and X₁₀ is t-butyl.

20. The taxane of claim 2 wherein R₁₄ is hydrido, R₉ is keto, and R₂ is benzoyloxy.

21. The taxane of claim 20 wherein X₃ is 2-furyl, 3-furyl, 2-thienyl, 3-thienyl, 2-pyridyl, 3-pyridyl, 4-pyridyl, C₂ - C₈ alkyl, C₂ - C₈ alkenyl, or C₂ - C₈ alkynyl.

22. The taxane of claim 20 wherein X₅ is -COX₁₀ and X₁₀ is phenyl, or X₅ is -COOX₁₀ and X₁₀ is t-butyl.

23. The taxane of claim 1 wherein R₁₀ is R₁₀ₐOCOO- and R₁₀ₐ is C₁ - C₈ alkyl.

24. The taxane of claim 23 wherein X₃ is 2-furyl, 3-furyl, 2-thienyl, 3-thienyl, 2-pyridyl, 3-pyridyl, 4-pyridyl, C₂ - C₈ alkyl, C₂ - C₈ alkenyl, or C₂ - C₈ alkynyl.

25. The taxane of claim 23 wherein X₅ is -COX₁₀ and X₁₀ is phenyl, or X₅ is -COOX₁₀ and X₁₀ is t-butyl.

26. The taxane of claim 23 wherein R₁₄ is hydrido.

27. The taxane of claim 26 wherein X₃ is 2-furyl, 3-furyl, 2-thienyl, 3-thienyl, 2-pyridyl, 3-pyridyl, 4-pyridyl, C₂ - C₈ alkyl, C₂ - C₈ alkenyl, or C₂ - C₈ alkynyl.

28. The taxane of claim 26 wherein X₅ is -COX₁₀ and X₁₀ is phenyl, or X₅ is -COOX₁₀ and X₁₀ is t-butyl.

29. The taxane of claim 23 wherein R₂ is benzoyloxy.

30. The taxane of claim 29 wherein X₃ is 2-furyl, 3-furyl, 2-thienyl, 3-thienyl, 2-pyridyl, 3-pyridyl, 4-pyridyl, C₂ - C₈ alkyl, C₂ - C₈ alkenyl, or C₂ - C₈ alkynyl.

31. The taxane of claim 29 wherein X₅ is -COX₁₀ and X₁₀ is phenyl, or X₅ is -COOX₁₀ and X₁₀ is t-butyl.

32. The taxane of claim 23 wherein R₁₄ is hydrido, R₉ is keto, and R₂ is benzoyloxy.

33. The taxane of claim 32 wherein X₃ is 2-furyl, 3-furyl, 2-thienyl, 3-thienyl, 2-pyridyl, 3-pyridyl, 4-pyridyl, C₂ - C₈ alkyl, C₂ - C₈ alkenyl, or C₂ - C₈ alkynyl.

34. The taxane of claim 32 wherein X₅ is -COX₁₀ and X₁₀ is phenyl, or X₅ is -COOX₁₀ and X₁₀ is t-butyl.

35. The taxane of claim 1 wherein R₁₀ is R₁₀ₐOCOO- and R₁₀ₐ is methyl or ethyl.

36. The taxane of claim 35 wherein X₃ is 2-furyl, 3-furyl, 2-thienyl, 3-thienyl, 2-pyridyl, 3-pyridyl, 4-pyridyl, C₂ - C₈ alkyl, C₂ - C₈ alkenyl, or C₂ - C₈ alkynyl.

37. The taxane of claim 35 wherein X₅ is -COX₁₀ and X₁₀ is phenyl, or X₅ is -COOX₁₀ and X₁₀ is t-butyl.

38. The taxane of claim 35 wherein R₁₄ is hydrido.

39. The taxane of claim 38 wherein X₃ is 2-furyl, 3-furyl, 2-thienyl, 3-thienyl, 2-pyridyl, 3-pyridyl, 4-pyridyl, C₂ - C₈ alkyl, C₂ - C₈ alkenyl, or C₂ - C₈ alkynyl.

40. The taxane of claim 38 wherein X₅ is -COX₁₀ and X₁₀ is phenyl, or X₅ is -COOX₁₀ and X₁₀ is t-butyl.

41. The taxane of claim 35 wherein R₂ is benzoyloxy.

42. The taxane of claim 41 wherein X₃ is 2-furyl, 3-furyl, 2-thienyl, 3-thienyl, 2-pyridyl, 3-pyridyl, 4-pyridyl, C₂ - C₈ alkyl, C₂ - C₈ alkenyl, or C₂ - C₈ alkynyl.

43. The taxane of claim 41 wherein X₅ is -COX₁₀ and X₁₀ is phenyl, or X₅ is -COOX₁₀ and X₁₀ is t-butyl.

44. The taxane of claim 35 wherein R₁₄ is hydrido and R₉ is keto.

45. The taxane of claim 44 wherein X₃ is 2-furyl, 3-furyl, 2-thienyl, 3-thienyl, 2-pyridyl, 3-pyridyl, 4-pyridyl, C₂ - C₈ alkyl, C₂ - C₈ alkenyl, or C₂ - C₈ alkynyl.

46. The taxane of claim 44 wherein X₅ is -COX₁₀ and X₁₀ is phenyl, or X₅ is -COOX₁₀ and X₁₀ is t-butyl.

47. The taxane of claim 35 wherein R₂ is benzoyloxy and R₉ is keto.

48. The taxane of claim 47 wherein X₃ is 2-furyl, 3-furyl, 2-thienyl, 3-thienyl, 2-pyridyl, 3-pyridyl, 4-pyridyl, C₂ - C₈ alkyl, C₂ - C₈ alkenyl, or C₂ - C₈ alkynyl.

49. The taxane of claim 47 wherein X₅ is -COX₁₀ and X₁₀ is phenyl, or X₅ is -COOX₁₀ and X₁₀ is t-butyl.

50. The taxane of claim 35 wherein R₁₄ is hydrido and R₂ is benzoyloxy.

51. The taxane of claim 50 wherein X₃ is 2-furyl, 3-furyl, 2-thienyl, 3-thienyl, 2-pyridyl, 3-pyridyl, 4-pyridyl, C₂ - C₈ alkyl, C₂ - C₈ alkenyl, or C₂ - C₈ alkynyl.

52. The taxane of claim 50 wherein X₅ is -COX₁₀ and X₁₀ is phenyl, or X₅ is -COOX₁₀ and X₁₀ is t-butyl.

53. The taxane of claim 35 wherein R₁₄ is hydrido, R₉ is keto, and R₂ is benzoyloxy.

54. The taxane of claim 53 wherein X₃ is 2-furyl, 3-furyl, 2-thienyl, 3-thienyl, 2-pyridyl, 3-pyridyl, 4-pyridyl, C₂ - C₈ alkyl, C₂ - C₈ alkenyl, or C₂ - C₈ alkynyl.

55. The taxane of claim 53 wherein X₅ is -COX₁₀ and X₁₀ is phenyl, or X₅ is -COOX₁₀ and X₁₀ is t-butyl.

56. The taxane of claim 53 wherein X₅ is -COOX₁₀ and X₁₀ is t-butyl.

57. The taxane of claim 56 wherein X₃ is 2-furyl, 3-furyl, 2-thienyl, 3-thienyl, 2-pyridyl, 3-pyridyl, 4-pyridyl, C₂ - C₈ alkyl, C₂- C₈ alkenyl, or C₂- C₈ alkynyl.

58. A taxane having the formula 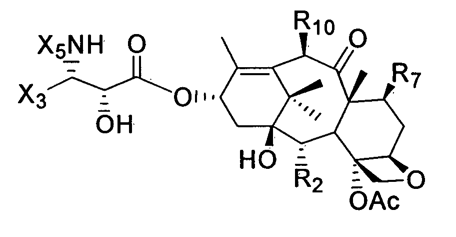
R₂ is benzoyloxy;
R₇ is hydroxy;
R₁₀ is R₁₀ₐOCOO-;
X₃ is substituted or unsubstituted alkyl, alkenyl, alkynyl, or heterocyclo, wherein alkyl comprises at least two carbon atoms;
X₅ is -COX₁₀, -COOX₁₀, or -CONHX₁₀;
X₁₀ is hydrocarbyl, substituted hydrocarbyl, or heterocyclo; and
R₁₀ₐ is hydrocarbyl, substituted hydrocarbyl, or heterocyclo; and
Ac is acetyl.

59. The taxane of claim 58 wherein X₃ is 2-furyl, 3-furyl, 2-thienyl, 3-thienyl, 2-pyridyl, 3-pyridyl, 4-pyridyl, C₂- C₈ alkyl, C₂ - C₈ alkenyl, or C₂ - C₈ alkynyl.

60. The taxane of claim 59 wherein X₅ is -COX₁₀ and X₁₀ is phenyl, or X₅ is -COOX₁₀ and X₁₀ is t-butyl.

61. The taxane of claim 58 wherein X₃ is furyl or thienyl.

62. The taxane of claim 61 wherein X₅ is -COX₁₀ and X₁₀ is substituted or unsubstituted phenyl, 2-furyl, 3-furyl, 2-thienyl, 3-thienyl, 2-pyridyl, 3-pyridyl, 4-pyridyl, C₁ - C₈ alkyl, C₂ - C₈ alkenyl, or C₂ - C₈ alkynyl, or X₅ is -COOX₁₀ and X₁₀ is substituted or unsubstituted C₁ - C₈ alkyl, C₂ - C₈ alkenyl, or C₂ - C₈ alkynyl.

63. The taxane of claim 61 wherein X₅ is -COX₁₀ and X₁₀ is phenyl, or X₅ is -COOX₁₀ and X₁₀ is t-butyl.

64. The taxane of claim 58 wherein R₁₀ₐ is methyl or ethyl.

65. The taxane of claim 64 wherein X₃ is 2-furyl, 3-furyl, 2-thienyl, 3-thienyl, 2-pyridyl, 3-pyridyl, 4-pyridyl, C₂ - C₈ alkyl, C₂- C₈ alkenyl, or C₂ - C₈ alkynyl.

66. The taxane of claim 65 wherein X₅ is -COX₁₀ and X₁₀ is substituted or unsubstituted phenyl, 2-furyl, 3-furyl, 2-thienyl, 3-thienyl, 2-pyridyl, 3-pyridyl, 4-pyridyl, C₁ - C₈ alkyl, C₂ - C₈ alkenyl, or C₂ - C₈ alkynyl, or X₅ is -COOX₁₀ and X₁₀ is substituted or unsubstituted C₁ - C₈ alkyl, C₂ - C₈ alkenyl, or C₂ - C₈ alkynyl.

67. The taxane of claim 65 wherein X₅ is -COX₁₀ and X₁₀ is phenyl, or X₅ is -COOX₁₀ and X₁₀ is t-butyl.

68. The taxane of claim 64 wherein X₃ is furyl or thienyl.

69. The taxane of claim 68 wherein X₅ is -COX₁₀ and X₁₀ is substituted or unsubstituted phenyl, 2-furyl, 3-furyl, 2-thienyl, 3-thienyl, 2-pyridyl, 3-pyridyl, 4-pyridyl, C₁ - C₈ alkyl, C₂ - C₈ alkenyl, or C₂ - C₈ alkynyl, or X₅ is -COOX₁₀ and X₁₀ is substituted or unsubstituted C₁ - C₈ alkyl, C₂ - C₈ alkenyl, or C₂ - C₈ alkynyl.

70. The taxane of claim 68 wherein X₅ is -COX₁₀ and X₁₀ is phenyl, or X₅ is -COOX₁₀ and X₁₀ is t-butyl.

71. The taxane of claim 64 wherein X₃ is cycloalkyl.

72. The taxane of claim 71 wherein X₅ is -COX₁₀ and X₁₀ is substituted or unsubstituted phenyl, 2-furyl, 3-furyl, 2-thienyl, 3-thienyl, 2-pyridyl, 3-pyridyl, 4-pyridyl, C₁ - C₈ alkyl, C₂ - C₈ alkenyl, or C₂ - C₈ alkynyl, or X₅ is -COOX₁₀ and X₁₀ is substituted or unsubstituted C₁ - C₈ alkyl, C₂ - C₈ alkenyl, or C₂ - C₈ alkynyl.

73. The taxane of claim 71 wherein X₅ is -COX₁₀ and X₁₀ is phenyl, or X₅ is -COOX₁₀ and X₁₀ is t-butyl.

74. The taxane of claim 64 wherein X₃ is isobutenyl.

75. The taxane of claim 74 wherein X₅ is -COX₁₀ and X₁₀ is substituted or unsubstituted phenyl, 2-furyl, 3-furyl, 2-thienyl, 3-thienyl, 2-pyridyl, 3-pyridyl, 4-pyridyl, C₁ - C₈ alkyl, C₂ - C₈ alkenyl, or C₂ - C₈ alkynyl, or X₅ is -COOX₁₀ and X₁₀ is substituted or unsubstituted C₁ - C₈ alkyl, C₂ - C₈ alkenyl, or C₂ - C₈ alkynyl.

76. The taxane of claim 74 wherein X₅ is -COX₁₀ and X₁₀ is phenyl, or X₅ is -COOX₁₀ and X₁₀ is t-butyl.

77. The taxane of claim 58 wherein X₃ is furyl or thienyl, R₁₀ₐ is ethyl, and X₅ is -COX₁₀ and X₁₀ is phenyl, or X₅ is -COOX₁₀ and X₁₀ is t-butyl.

78. The taxane of claim 58 wherein X₃ is 2-furyl or 2-thienyl, R₁₀ₐ is ethyl, X₅ is -COOX₁₀ and X₁₀ is t-butyl.

79. The taxane of claim 58 wherein X₃ is isobutenyl, X₅ is -COOX₁₀ and X₁₀ is t-butyl.

80. The taxane of claim 58 wherein X₃ is cycloalkyl, R₁₀ₐ is methyl or ethyl, X₅ is -COOX₁₀ and X₁₀ is t-butyl.

81. A taxane having the formula 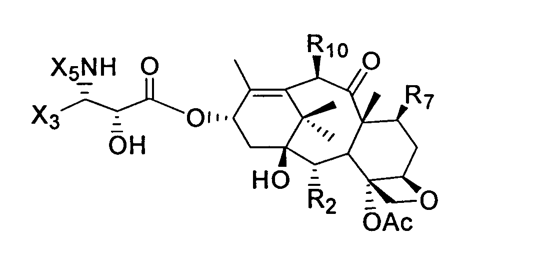
R₂ is benzoyloxy;
R₇ is hydroxy;
R₁₀ is R₁₀ₐOCOO-;
X₃ is cycloalkyl, alkenyl, alkynyl, phenyl or heterocyclo;
X₅ is -COX₁₀, -COOX₁₀, or -CONHX₁₀;
X₁₀ is hydrocarbyl, substituted hydrocarbyl, or heterocyclo; and
R₁₀ₐ is alkyl, alkenyl, alkynyl, phenyl, or heterocyclo wherein alkyl comprises at least two carbon atoms; and
Ac is acetyl.

82. The taxane of claim 81 wherein X₃ is 2-furyl, 3-furyl, 2-thienyl, 3-thienyl, 2-pyridyl, 3-pyridyl, 4-pyridyl, C₂- C₈ alkyl, C₂- C₈ alkenyl, or C₂- C₈ alkynyl.

83. The taxane of claim 82 wherein X₅ is -COX₁₀ and X₁₀ is substituted or unsubstituted phenyl, 2-furyl, 3-furyl, 2-thienyl, 3-thienyl, 2-pyridyl, 3-pyridyl, 4-pyridyl, C₁ - C₈ alkyl, C₂- C₈ alkenyl, or C₂- C₈ alkynyl, or X₅ is -COOX₁₀ and X₁₀ is substituted or unsubstituted C₁ - C₈ alkyl, C₂- C₈ alkenyl, or C₂ - C₈ alkynyl.

84. The taxane of claim 82 wherein X₅ is -COX₁₀ and X₁₀ is phenyl, or X₅ is -COOX₁₀ and X₁₀ is t-butyl.

85. The taxane of claim 81 wherein X₃ is furyl or thienyl.

86. The taxane of claim 85 wherein X₅ is -COX₁₀ and X₁₀ is substituted or unsubstituted phenyl, 2-furyl, 3-furyl, 2-thienyl, 3-thienyl, 2-pyridyl, 3-pyridyl, 4-pyridyl, C₁ - C₈ alkyl, C₂- C₈ alkenyl, or C₂- C₈ alkynyl, or X₅ is -COOX₁₀ and X₁₀ is substituted or unsubstituted C₁ - C₈ alkyl, C₂- C₈ alkenyl, or C₂- C₈ alkynyl.

87. The taxane of claim 85 wherein X₅ is -COX₁₀ and X₁₀ is phenyl, or X₅ is -COOX₁₀ and X₁₀ is t-butyl.

88. The taxane of claim 81 wherein X₃ is phenyl.

89. The taxane of claim 88 wherein X₅ is -COX₁₀ and X₁₀ is substituted or unsubstituted phenyl, 2-furyl, 3-furyl, 2-thienyl, 3-thienyl, 2-pyridyl, 3-pyridyl, 4-pyridyl, C₁ - C₈ alkyl, C₂ - C₈ alkenyl, or C₂ - C₈ alkynyl, or X₅ is -COOX₁₀ and X₁₀ is substituted or unsubstituted C₁ - C₈ alkyl, C₂- C₈ alkenyl, or C₂ - C₈ alkynyl.

90. The taxane of claim 88 wherein X₅ is -COX₁₀ and X₁₀ is phenyl, or X₅ is -COOX₁₀ and X₁₀ is t-butyl.

91. The taxane of claim 81 wherein X₃ is isobutenyl.

92. The taxane of claim 91 wherein X₅ is -COX₁₀ and X₁₀ is substituted or unsubstituted phenyl, 2-furyl, 3-furyl, 2-thienyl, 3-thienyl, 2-pyridyl, 3-pyridyl, 4-pyridyl, C₁ - C₈ alkyl, C₂- C₈ alkenyl, or C₂ - C₈ alkynyl, or X₅ is -COOX₁₀ and X₁₀ is substituted or unsubstituted C₁ - C₈ alkyl, C₂- C₈ alkenyl, or C₂- C₈ alkynyl.

93. The taxane of claim 91 wherein X₅ is -COX₁₀ and X₁₀ is phenyl, or X₅ is -COOX₁₀ and X₁₀ is t-butyl.

94. The taxane of claim 81 wherein R₁₀ₐ is ethyl.

95. The taxane of claim 94 wherein X₃ is 2-furyl, 3-furyl, 2-thienyl, 3-thienyl, 2-pyridyl, 3-pyridyl, 4-pyridyl, C₂- C₈ alkyl, C₂- C₈ alkenyl, or C₂ - C₈ alkynyl.

96. The taxane of claim 94 wherein X₅ is -COX₁₀ and X₁₀ is substituted or unsubstituted phenyl, 2-furyl, 3-furyl, 2-thienyl, 3-thienyl, 2-pyridyl, 3-pyridyl, 4-pyridyl, C₁ - C₈ alkyl, C₂- C₈ alkenyl, or C₂- C₈ alkynyl, or X₅ is -COOX₁₀ and X₁₀ is substituted or unsubstituted C₁ - C₈ alkyl, C₂- C₈ alkenyl, or C₂- C₈ alkynyl.

97. The taxane of claim 94 wherein X₅ is -COX₁₀ and X₁₀ is phenyl, or X₅ is -COOX₁₀ and X₁₀ is t-butyl.

98. The taxane of claim 94 wherein X₃ is furyl or thienyl.

99. The taxane of claim 98 wherein X₅ is -COX₁₀ and X₁₀ is substituted or unsubstituted phenyl, 2-furyl, 3-furyl, 2-thienyl, 3-thienyl, 2-pyridyl, 3-pyridyl, 4-pyridyl, C₁ - C₈ alkyl, C₂- C₈ alkenyl, or C₂- C₈ alkynyl, or X₅ is -COOX₁₀ and X₁₀ is substituted or unsubstituted C₁ - C₈ alkyl, C₂ - C₈ alkenyl, or C₂- C₈ alkynyl.

100. The taxane of claim 98 wherein X₅ is -COX₁₀ and X₁₀ is phenyl, or X₅ is -COOX₁₀ and X₁₀ is t-butyl.

101. The taxane of claim 98 wherein X₅ is -COOX₁₀ and X₁₀ is t-butyl.

102. A pharmaceutical composition comprising the taxane of claim 1 and one or more pharmacologically acceptable, inert or physiologically active diluents or adjuvants.

103. The pharmaceutical composition of claim 102 wherein X₃ is 2-furyl, 3-furyl, 2-thienyl, 3-thienyl, 2-pyridyl, 3-pyridyl, 4-pyridyl, C₂ - C₈ alkyl, C₂ - C₈ alkenyl, or C₂ - C₈ alkynyl.

104. The pharmaceutical composition of claim 103 wherein X₅ is -COX₁₀ and X₁₀ is substituted or unsubstituted phenyl, 2-furyl, 3-furyl, 2-thienyl, 3-thienyl, 2-pyridyl, 3-pyridyl, 4-pyridyl, C₁ - C₈ alkyl, C₂ - C₈ alkenyl, or C₂ - C₈ alkynyl, or X₅ is -COOX₁₀ and X₁₀ is substituted or unsubstituted C₁ - C₈ alkyl, C₂ - C₈ alkenyl, or C₂- C₈ alkynyl.

105. The pharmaceutical composition of claim 103 wherein X₅ is -COX₁₀ and X₁₀ is phenyl, or X₅ is -COOX₁₀ and X₁₀ is t-butyl.

106. The pharmaceutical composition of claim 102 wherein R₁₀ₐ is methyl, ethyl or propyl.

107. The pharmaceutical composition of claim 106 wherein X₃ is 2-furyl, 3-furyl, 2-thienyl, 3-thienyl, 2-pyridyl, 3-pyridyl, 4-pyridyl, C₂ - C₈ alkyl, C₂ - C₈ alkenyl, or C₂ - C₈ alkynyl.

108. The pharmaceutical composition of claim 107 wherein X₅ is -COX₁₀ and X₁₀ is substituted or unsubstituted phenyl, 2-furyl, 3-furyl, 2-thienyl, 3-thienyl, 2-pyridyl, 3-pyridyl, 4-pyridyl, C₁ - C₈ alkyl, C₂ - C₈ alkenyl, or C₂ - C₈ alkynyl, or X₅ is -COOX₁₀ and X₁₀ is substituted or unsubstituted C₁ - C₈ alkyl, C₂ - C₈ alkenyl, or C₂ - C₈ alkynyl.

109. The pharmaceutical composition of claim 107 wherein X₅ is -COX₁₀ and X₁₀ is phenyl, or X₅ is -COOX₁₀ and X₁₀ is t-butyl.

110. The pharmaceutical composition of claim 103 wherein X₃ is furyl or thienyl, R₁₀ₐ is methyl or ethyl, and X₅ is -COX₁₀ and X₁₀ is phenyl, or X₅ is - COOX₁₀ and X₁₀ is t-butyl.

111. The pharmaceutical composition of claim 103 wherein X₃ is substituted or unsubstituted furyl, R₁₀ₐ is methyl or ethyl, and X₅ is -COX₁₀ and X₁₀ is phenyl, or X₅ is -COOX₁₀ and X₁₀ is t-butyl.

112. The pharmaceutical composition of claim 103 wherein X₃ is substituted or unsubstituted thienyl, R₁₀ₐ is methyl or ethyl, and X₅ is -COX₁₀ and X₁₀ is phenyl, or X₅ is -COOX₁₀ and X₁₀ is t-butyl.

113. The pharmaceutical composition of claim 103 wherein X₃ is isobutenyl, R₁₀ₐ is methyl or ethyl, and X₅ is -COX₁₀ and X₁₀ is phenyl, or X₅ is - COOX₁₀ and X₁₀ is t-butyl.

114. The pharmaceutical composition of claim 103 wherein X₃ is alkyl, R₁₀ₐ is methyl, and X₅ is -COX₁₀ and X₁₀ is phenyl, or X₅ is -COOX₁₀ and X₁₀ is t-butyl.

115. The pharmaceutical composition of claim 103 wherein X₃ is 2-furyl or 2-thienyl, R₁₀ₐ is methyl, X₅ is -COOX₁₀ and X₁₀ is t-butyl.

116. The pharmaceutical composition of claim 103 wherein X₃ is 2-furyl, R₁₀ₐ is ethyl, X₅ is -COOX₁₀ and X₁₀ is t-butyl.

117. The pharmaceutical composition of claim 103 wherein X₃ is 2-thienyl, R₁₀ₐ is ethyl, X₅ is -COOX₁₀ and X₁₀ is t-butyl.

118. The pharmaceutical composition of claim 103 wherein X₃ is isobutenyl, X₅ is -COOX₁₀ and X₁₀ is t-butyl.

119. The pharmaceutical composition of claim 103 wherein X₃ is cycloalkyl, R₁₀ₐ is methyl, X₅ is -COOX₁₀ and X₁₀ is t-butyl.

120. A pharmaceutical composition comprising the taxane of claim 58 and one or more pharmacologically acceptable, inert or physiologically active diluents or adjuvants.

121. A pharmaceutical composition comprising the taxane of claim 61 and one or more pharmacologically acceptable, inert or physiologically active diluents or adjuvants.

122. A composition for oral administration comprising the taxane of claim 1 and at least one pharmaceutically acceptable carrier.

123. The composition of claim 122 wherein R₁₀ is R₁₀ₐOCOO- and R₁₀ₐ is substituted or unsubstituted C₁ - C₈ alkyl, C₂- C₈ alkenyl or C₂- C₈ alkynyl.

124. The composition of claim 123 wherein X₃ is phenyl, isobutenyl, 2-furyl, 3-furyl, 2-thienyl, 3-thienyl, 2-pyridyl, 3-pyridyl, 4-pyridyl, C₁ - C₈ alkyl, C₂ - C₈ alkenyl, or C₂ - C₈ alkynyl.

125. The composition of claim 124 herein X₅ is -COX₁₀ and X₁₀ is phenyl, or X₅ is -COOX₁₀ and X₁₀ is t-butyl.

126. The composition of claim 125 wherein R₁₀ₐ is methyl, ethyl or propyl.

127. The pharmaceutical composition of claim 126 wherein X₃ is isobutenyl, R₁₀ₐ is methyl or ethyl, and X₅ is -COX₁₀ and X₁₀ is phenyl, or X₅ is - COOX₁₀ and X₁₀ is t-butyl.

128. The pharmaceutical composition of claim 127 wherein X₃ is 2-furyl or 2-thienyl, R₁₀ₐ is ethyl, X₅ is -COOX₁₀ and X₁₀ is t-butyl.

129. The pharmaceutical composition of claim 127 wherein X₃ is isobutenyl, X₅ is -COOX₁₀, R₁₀ₐ is ethyl, and X₁₀ is t-butyl.

130. The pharmaceutical composition of claim 122 wherein X₃ is phenyl, R₁₀ₐ is ethyl, X₅ is -COOX₁₀ and X₁₀ is t-butyl.

131. A pharmaceutical composition comprising the taxane of claim 92 and at least one pharmaceutical acceptable carrier.

132. The use of a taxane as defined in any one of claims 1 - 101 for manufacturing a pharmaceutical composition effective for inhibiting tumor growth in a mammal when orally administering a therapeutically effective amount of said pharmaceutical composition, containing said taxane and at least one pharmaceutically acceptable carrier, to said mammal.

133. The use of claim 132 wherein R₁₀ is R₁₀ₐOCOO- and R₁₀ₐ is substituted or unsubstituted C₁ - C₈ alkyl, C₂- C₈ alkenyl or C₂- C₈ alkynyl.

134. The use of claim 133 wherein X₃ is phenyl, isobutenyl, 2-furyl, 3-furyl, 2-thienyl, 3-thienyl, 2-pyridyl, 3-pyridyl, 4-pyridyl, C₂- C₈ alkyl, C₂- C₈ alkenyl, or C₂- C₈ alkynyl.

135. The use of claim 134 herein X₅ is -COX₁₀ and X₁₀ is phenyl, or X₅ is -COOX₁₀ and X₁₀ is t-butyl.

136. The use of claim 135 wherein R₁₀ₐ is methyl, ethyl or propyl.

137. The use of claim 136 wherein X₃ is isobutenyl, R₁₀ₐ is methyl or ethyl, and X₅ is -COX₁₀ and X₁₀ is phenyl, or X₅ is -COOX₁₀ and X₁₀ is t-butyl.

138. The use of claim 137 wherein X₃ is isobutenyl, X₅ is -COOX₁₀, R₁₀ₐ is ethyl, and X₁₀ is t-butyl.

139. The use of claim 132 wherein X₃ is 2-furyl or 2-thienyl, R₁₀ₐ is ethyl, X₅ is -COOX₁₀ and X₁₀ is t-butyl.

140. The use of claim 132 wherein X₃ is phenyl, R₁₀ₐ is ethyl, X₅ is - COOX₁₀ and X₁₀ is t-butyl.

141. The use of any one of claims 132 - 140, said composition comprising a taxane as defined in claim 92.

142. A pharmaceutical composition as defined in any one of the preceeding claims, with an ID50 value, of at least 4 and preferably 5, 6, 7, 8, 9 or 10 times less than the ID50 value of paclitaxel or docetaxel, when measured as defined herein.

143. A pharmaceutical composition as defined in any one of the preceeding claims, in a single dose formulation comprising at least 20 mg of said taxane per m² of patient body surface area.

144. The pharmaceutical composition of claim 169, comprising less than 600 mg, preferably between 25 and 400 mg, more preferably between 40 and 300 mg and even more preferably between 50 and 200 mg, of said taxane per m² of patient body surface area, for oral administration, wherein the average body surface area for a human is 1.8 m².

145. The pharmaceutical composition of claim 169, comprising less than 500 mg, preferably 40 to 400 mg and more preferably 60 to 350 mg of said taxane per m² of patient body surface area, for parenteral administration, wherein the average body surface area for a human is 1.8 m².

146. A pharmaceutical composition as defined in any one of the preceeding claims, in the form of a liquid composition containing between 0.01 and 10 mg/ml of taxane, preferably between 0.1 and 7 mg/ml, more preferably between 0.5 and 5 mg/ml and most preferred between 1.5 and 4 mg/ml.

147. A pharmaceutical composition as defined in any one of the preceeding claims, in the form of a liquid composition containing between 5 wt% and 50 wt%, preferably between 8 wt% and 40 wt%, more preferred between 10 wt% and 30 wt% of said taxane.

148. A pharmaceutical composition as defined in any one of the preceeding claims, comprising a taxane antitumor agent in an amount effective for achieving significant tumor growth inhibition, preferably partial tumor shrinkage (Partial Response) and most preferably complete tumor shrinkage (Complete Response) as defined herein, in at least one of human lung carcinoma, human colon carcinoma, human breast carcinoma, human prostate carcinoma, human ovarian carcinoma, human melanoma and human pancreatic carcinoma.

149. The pharmaceutical composition of claim 148, said composition being more efficient than paclitaxel and/or docetaxel in comparable amount.

150. The pharmaceutical composition of any one of claims 142 - 149, said taxane selected from the compounds defined in claim 92 or preferably, claim 96.

151. The pharmaceutical composition of any one of claims 142 - 150, said taxane selected from compounds corresponding to formula 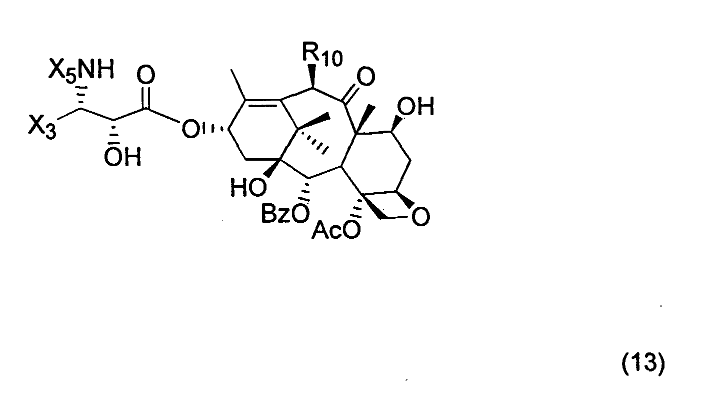
with X₅ = tBuOCO-, X₃ = 2-thienyl and R₁₀ = EtOCOO-,
or X₅ = tBuOCO-, X₃ = Phenyl and R₁₀ = EtOCOO-,
or X₅ = tBuOCO-, X₃ = Isobutenyl and R₁₀ = EtOCOO-,

152. The pharmaceutical composition of any one of the preceeding claims, the taxane having a solubility in ethanol of at least 100 mg/ml and preferably up to 800 mg/ml and higher.

## Amended claims

### Amended claims in accordance with Rule 86(2) EPC.

**1.** A taxane having the formula: 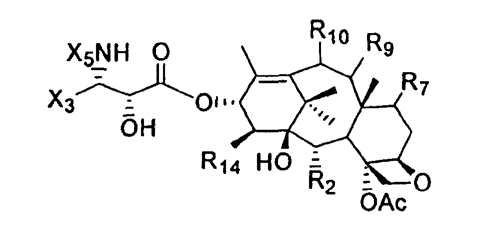 wherein
R₂ is acyloxy;
R₇ is hydroxy;
R₉ is keto, hydroxy, or acyloxy;
R₁₀ is carbonate;
R₁₄ is hydrido or hydroxy;
X₃ is substituted or unsubstituted alkyl, alkenyl, alkynyl, phenyl, or heterocyclo, wherein alkyl comprises at least two carbon atoms;
X₅ is -COX₁₀, -COOX₁₀, or -CONHX₁₀;
X₁₀ is hydrocarbyl, substituted hydrocarbyl, or heterocyclo; and
Ac is acetyl.

**2.** The taxane of claim 1 wherein R₁₀ is R₁₀ₐOCOO- and R₁₀ₐ is substituted or unsubstituted C₁ - C₈ alkyl, C₂ - C₈ alkenyl or C₂ - C₈ alkynyl.

**3.** The taxane of claim 2 wherein X₃ is furyl, thienyl, pyridyl, C₂ - C₈ alkyl, C₂ - C₈ alkenyl, or C₂ - C₈ alkynyl.

**4.** The taxane of claim 2 wherein X₅ is -COX₁₀ and X₁₀ is phenyl, or X₅ is -COOX₁₀ and X₁₀ is t-butyl.

**5.** The taxane of claim 2 wherein R₁₄ is hydrido.

**6.** The taxane of claim 5 wherein X₃ is furyl, thienyl, pyridyl, C₂ - C₈ alkyl, C₂ - C₈ alkenyl, or C₂ - C₈ alkynyl.

**7.** The taxane of claim 5 wherein X₅ is -COX₁₀ and X₁₀ is phenyl, or X₅ is -COOX₁₀ and X₁₀ is t-butyl.
